(19) Europäisches Patentamt
European Patent Office
Office européen des brevets

(11) EP 3 564 221 A1

(12) **EUROPEAN PATENT APPLICATION**
published in accordance with Art. 153(4) EPC

(43) Date of publication:
**06.11.2019 Bulletin 2019/45**

(21) Application number: **17888582.8**

(22) Date of filing: **14.12.2017**

(51) Int Cl.:
***C07D 277/82*** *(2006.01)* ***C07D 417/04*** *(2006.01)*
***C08F 20/38*** *(2006.01)* ***G02B 1/111*** *(2015.01)*
***G02B 5/30*** *(2006.01)*

(86) International application number:
**PCT/JP2017/044977**

(87) International publication number:
**WO 2018/123625 (05.07.2018 Gazette 2018/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **26.12.2016 JP 2016252108**

(71) Applicant: **Zeon Corporation**
**Tokyo 100-8246 (JP)**

(72) Inventors:
- **SAKAMOTO Kei**
**Tokyo 100-8246 (JP)**
- **OKUYAMA Kumi**
**Tokyo 100-8246 (JP)**

(74) Representative: **Beckmann, Claus**
**Kraus & Weisert**
**Patentanwälte PartGmbB**
**Thomas-Wimmer-Ring 15**
**80539 München (DE)**

# (54) POLYMERIZABLE COMPOUND, MIXTURE, POLYMER, OPTICAL FILM, OPTICALLY ANISOTROPIC BODY, POLARIZING SHEET, DISPLAY DEVICE, AND ANTIREFLECTIVE FILM

(57) Provided is a polymerizable compound usable in the preparation of an optical film, etc. having favorable reverse wavelength dispersibility. The polymerizable compound is represented by the following Formula (I) where $Ar^1$ represents a predetermined ring group, $A^1$ to $A^2$ and $B^1$ to $B^2$ represent a cyclic aliphatic group or an aromatic group that may have a substituent, $Z^1$ to $Z^2$, $L^1$ to $L^2$, and $L^{1a}$ to $L^{2a}$ represent a predetermined group such as -O-, -C(=O)-O-, or -O-C(=O)-, $G^1$ to $G^2$ represent an aliphatic hydrocarbon group with a carbon number of 3 to 20 that may have a substituent, $P^{1a}$ to $P^{2a}$ represent a polymerizable group, a and b represent 0 or 1, and a part represented by $-L^{1a}-G^1-P^{1a}$ and a part represented by $-L^{2a}-G^2-P^{2a}$ have different structures:

$$P^{1a}-G^1-L^{1a}\left[B^1-L^1\right]_a A^1-Z^1-Ar^1-Z^2-A^2\left[L^2-B^2\right]_b L^{2a}-G^2-P^{2a}$$

(I)

EP 3 564 221 A1

## Description

### TECHNICAL FIELD

The present disclosure relates to an optical film and an optically anisotropic product capable of uniform polarized light conversion over a wide wavelength range, and a polarizing plate, a display device, and an antireflection film using the optically anisotropic product.

The present disclosure also relates to a polymer usable in the preparation of the optical film and the optically anisotropic product, and a polymerizable compound and a mixture usable in the preparation of the polymer.

### BACKGROUND

Retardation plates used in various devices such as flat panel display devices include quarter wavelength plates for converting linearly polarized light into circularly polarized light and half wavelength plates for converting the plane of vibration of linearly polarized light by 90 degrees. These retardation plates are capable of accurately providing a phase difference of $\lambda/4$ or $\lambda/2$ of the light wavelength for specific monochromatic light.

However, conventional retardation plates have a problem in that polarized light output through a retardation plate is converted into colored polarized light. A material forming the retardation plate has wavelength dispersibility for phase differences, and a distribution occurs in the polarization state of each wavelength for white light which is a composite wave in which light rays in the visible light range are mixed. This makes it impossible to adjust input light to polarized light of an accurate $\lambda/4$ or $\lambda/2$ phase difference in all wavelength regions.

To solve this problem, various wide band retardation plates capable of providing a uniform phase difference to light over a wide wavelength range, that is, retardation plates having reverse wavelength dispersibility, have been studied.

Meanwhile, with the functionality enhancement and widespread use of mobile information terminals such as mobile PCs and mobile phones, the need to reduce the thicknesses of flat panel display devices as much as possible has been growing. This has led to the demand for thinner retardation plates as their constituent members.

The most effective method for reducing the thicknesses of retardation plates in recent years is a method of producing a retardation plate by applying a polymerizable composition containing a low-molecular polymerizable compound to a film substrate to form an optical film. Hence, many polymerizable compounds capable of forming optical films having excellent reverse wavelength dispersibility or polymerizable compositions using such polymerizable compounds have been developed.

For example, PTL 1 and PTL 2 each propose a polymerizable compound and a polymerizable composition that are capable of forming an optical film having excellent reverse wavelength dispersibility, have a low melting point suitable for processing and are easy to be applied to a substrate, have a wide temperature range in which liquid crystallinity is exhibited, and can be synthesized at low cost.

### CITATION LIST

#### Patent Literatures

PTL 1: WO 2014/010325 A1
PTL 2: JP 2015-200877 A

### SUMMARY

#### (Technical Problem)

In the production of an optical film or an optically anisotropic product (hereafter also collectively referred to as "optical film, etc.") using a polymerizable compound, it is desirable to not only obtain an optical film, etc. having excellent reverse wavelength dispersibility but also improve productivity.

Particularly when producing an optical film, etc. on an industrial scale by applying a polymerizable composition containing a polymerizable compound in a large area, temperature control in the coating step is important. It is therefore desirable to use a material that is usable in a temperature range easy to control industrially, to improve productivity and yield.

A polymerizable compound used in the production of an optical film, etc. having a phase difference of $\lambda/4$, which is employed in a display device such as an organic EL panel (OLED), is required to not only enable the preparation

of an optical film, etc. capable of uniform polarized light conversion over a wide wavelength range but also have low liquid crystallization temperature and excellent coatability.

The conventional polymerizable compounds described above have room for improvement in lowering the liquid crystallization temperature.

It could therefore be helpful to provide a polymerizable compound that can form an optical film, etc. having excellent reverse wavelength dispersibility and has lower liquid crystallization temperature.

(Solution to Problem)

As a result of extensive studies made to achieve the object stated above, the following fact has been discovered: A polymerizable compound represented by the following Formula (I) in which the structure of the part represented by $-L^{1a}-G^1-P^{1a}$ and the structure of the part represented by $-L^{2a}-G^2-P^{2a}$ are different has lower liquid crystallization temperature than a polymerizable compound in which the structure of the part represented by $-L^{1a}-G^1-P^{1a}$ and the structure of the part represented by $-L^{2a}-G^2-P^{2a}$ are the same, and, by using the polymerizable compound represented by the following Formula (I), an optical film, etc. having excellent reverse wavelength dispersibility can be formed at lower processing temperature. The present disclosure is based on these discoveries.

The below-described polymerizable compound, mixture, polymer, optical film, optically anisotropic product, polarizing plate, display device, and antireflection film are thus provided.

[1] A polymerizable compound represented by the following Formula (I):

$$P^{1a}-G^1-L^{1a}\left[B^1-L^1\right]_a A^1-Z^1-Ar^1-Z^2-A^2\left[L^2-B^2\right]_b L^{2a}-G^2-P^{2a}$$

(I)

where $Ar^1$ represents a divalent aromatic hydrocarbon ring group having at least $D^1$ as a substituent or a divalent aromatic heterocyclic group having at least $D^1$ as a substituent,
$D^1$ represents an organic group with a carbon number of 1 to 67 having at least one aromatic ring selected from the group consisting of an aromatic hydrocarbon ring and an aromatic heterocyclic ring,
$Z^1$ and $Z^2$ each independently represent a single bond, -O-, $-O-CH_2-$, $-CH_2-O-$, -C(=O)-O-, -O-C(=O)-, -C(=O)-S-, -S-C(=O)-, $-NR^{21}-C(=O)-$, $-C(=O)-NR^{21}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, and $R^{21}$ each independently represent a hydrogen atom or an alkyl group with a carbon number of 1 to 6,
$A^1$ and $A^2$ and $B^1$ and $B^2$ each independently represent a cyclic aliphatic group that may have a substituent or an aromatic group that may have a substituent,
$L^1$ and $L^2$ and $L^{1a}$ and $L^{2a}$ each independently represent a single bond, -O-, -C(=O)-, -C(=O)-O-, -O-C(=O)-, $-NR^{22}-C(=O)-$, $-C(=O)-NR^{22}-$, -O-C(=O)-O-, $-NR^{22}-C(=O)-O-$, $-O-C(=O)-NR^{22}-$, or $-NR^{22}-C(=O)-NR^{23}-$, and $R^{22}$ and $R^{23}$ each independently represent a hydrogen atom or an alkyl group with a carbon number of 1 to 6,
$G^1$ and $G^2$ each independently represent an aliphatic hydrocarbon group with a carbon number of 3 to 20 that may have a substituent, the aliphatic hydrocarbon group with a carbon number of 3 to 20 may be interrupted by at least one intervening group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, -N=, =N-, and -N=N-, in the case where two or more intervening groups are present, the two or more intervening groups may be same or different and are not adjacent to each other, and R each independently represent a hydrogen atom or an alkyl group with a carbon number of 1 to 6,
$P^{1a}$ and $P^{2a}$ each independently represent a polymerizable group,
a and b each independently represent 0 or 1, and
a part represented by $-L^{1a}-G^1-P^{1a}$ and a part represented by $-L^{2a}-G^2-P^{2a}$ have different structures.

[2] The polymerizable compound according to [1], wherein $L^{1a}$ and $L^{2a}$ have a same structure,
$G^1$ and $G^2$ have different structures, and
$P^{1a}$ and $P^{2a}$ have a same structure.
[3] The polymerizable compound according to [1] or [2], wherein $G^1$ and $G^2$ have different structures,
one of $G^1$ and $G^2$ is an organic group composed of a plurality of methylene groups that may be substituted and at least one group selected from the group consisting of -O-, -C(=O)-, -C(=O)-O-, -O-C(=O)-, -S-, -C(=O)-S-, -S-C(=O)-,

-NR-, -NR-C(=O)-, -C(=O)-NR-, -O-$(CH_2)_n$-O-, -CH=CH-C(=O)-O-, -O-C(=O)-CH=CH-, -CH=CH-, -N=CH-, -CH=N-, -N=C($CH_3$)-, -C($CH_3$)=N-, -N=N-, and -C=C- located between methylene groups that may be substituted,
an other one of $G^1$ and $G^2$ is an alkylene group with a carbon number of 3 to 20 that may have a substituent, or an organic group composed of a plurality of methylene groups that may be substituted and at least one group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, -O-$(CH_2)_n$-O-, -CH=CH-C(=O)-O-, -O-C(=O)-CH=CH-, -CH=CH-, -N=CH-, -CH=N-, -N=C($CH_3$)-, -C($CH_3$)=N-, -N=N-, and -C=C- located between methylene groups that may be substituted, and
R is as defined above, and n represents an integer of 1 to 18.
[4] The polymerizable compound according to any of [1] to [3], wherein $G^1$ and $G^2$ have different structures,
the aliphatic hydrocarbon group with a carbon number of 3 to 20 is an alkylene group with a carbon number of 3 to 20, at least one of $G^1$ and $G^2$ has at least one hydrogen atom substituted by a substituent formed by a halogen atom, a cyano group, an alkyl group with a carbon number of 1 to 6, an alkenyl group with a carbon number of 2 to 6, an alkynyl group with a carbon number of 2 to 6, an alkyl halide group with a carbon number of 1 to 6, an N,N-dialkylamino group with a carbon number of 2 to 6, an alkoxy group with a carbon number of 1 to 6, a nitro group, -C(=O)-$R^{1a}$, -C(=O)-O-$R^{1a}$, or -O-C(=O)-$R^{1a}$, and $R^{1a}$ represents an alkyl group with a carbon number of 1 to 6, an aromatic hydrocarbon ring group with a carbon number of 6 to 20, or an aliphatic hydrocarbon ring group with a carbon number of 6 to 20, and
in the case where a plurality of substituents are present, the plurality of substituents may be same or different.
[5] The polymerizable compound according to any of [1] to [4], wherein $Ar^1$ is a group represented by any of the following Formulas (III-1) to (III-3):

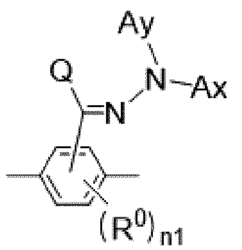
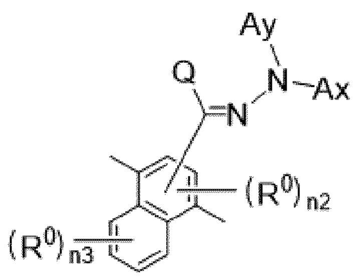
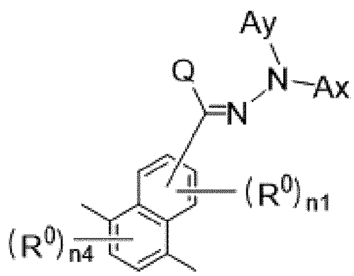

(III-1) (III-2) (III-3)

where Ax represents an organic group having at least one aromatic ring selected from the group consisting of an aromatic hydrocarbon ring with a carbon number of 6 to 30 and an aromatic heterocyclic ring with a carbon number of 2 to 30, and the aromatic ring of Ax may have a substituent,
Ay represents a hydrogen atom or an organic group with a carbon number of 1 to 30 that may have a substituent,
Q represents a hydrogen atom or an alkyl group with a carbon number of 1 to 6,
$R^0$ represents a halogen atom, a cyano group, an alkyl group with a carbon number of 1 to 6, an alkenyl group with a carbon number of 2 to 6, an alkyl halide group with a carbon number of 1 to 6, an N,N-dialkylamino group with a carbon number of 2 to 12, an alkoxy group with a carbon number of 1 to 6, a nitro group, -C(=O)-$R^a$, -C(=O)-O-$R^a$, or $SO_2R^a$, and $R^a$ represents an alkyl group with a carbon number of 1 to 6, or an aromatic hydrocarbon ring group with a carbon number of 6 to 20 that may have an alkyl group with a carbon number of 1 to 6 or an alkoxy group with a carbon number of 1 to 6 as a substituent,
n1 represents an integer of 0 to 3, n2 represents 0 or 1, n3 represents an integer of 0 to 4, and n4 represents an integer of 0 to 2, and
in the case where a plurality of $R^0$ are present, the plurality of $R^0$ may be same or different.

[6] The polymerizable compound according to [5], wherein $Ar^1$ is a group represented by any of the following Formulas (IV-1) to (IV-3):

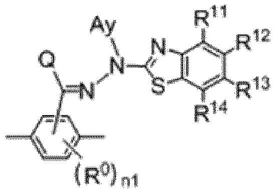 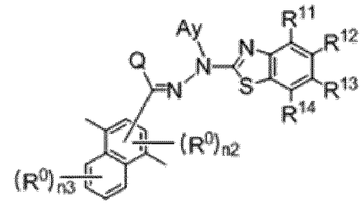 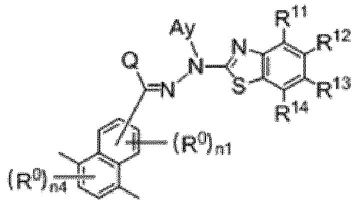

(IV-1) (IV-2) (IV-3)

where Ay, Q, $R^0$, n1, n2, n3, and n4 are as defined above, and
$R^{11}$ to $R^{14}$ each independently represent a hydrogen atom, a halogen atom, an alkyl group with a carbon number of 1 to 6, a cyano group, a nitro group, a fluoroalkyl group with a carbon number of 1 to 6, an alkoxy group with a carbon number of 1 to 6, or $-C(=O)-O-R^b$, $R^b$ represents an alkyl group with a carbon number of 1 to 20 that may have a substituent, an alkenyl group with a carbon number of 2 to 20 that may have a substituent, a cycloalkyl group with a carbon number of 3 to 12 that may have a substituent, or an aromatic hydrocarbon ring group with a carbon number of 5 to 12 that may have a substituent, and at least one of $C-R^{11}$ to $C-R^{14}$ forming a ring may be substituted by a nitrogen atom.

[7] The polymerizable compound according to any of [1] to [4], wherein $Ar^1$ is a group represented by any of the following Formulas (V-1) to (V-4):

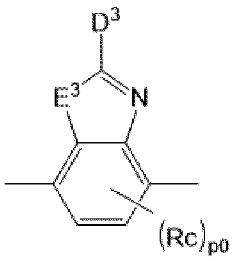 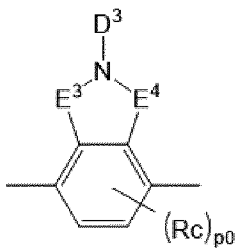 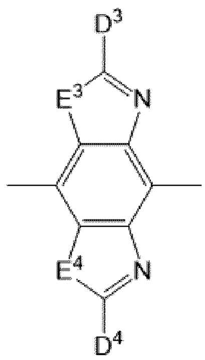 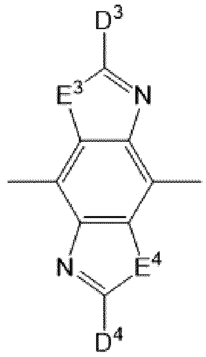

(V-1) (V-2) (V-3) (V-4)

where $E^3$ and $E^4$ each independently represent $-CR^{24}R^{25}-$, -S-, $-NR^{24}-$, -C(=O)-, or -O-, and $R^{24}$ and $R^{25}$ each independently represent a hydrogen atom or an alkyl group with a carbon number of 1 to 4,
Rc represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p0 represents an integer of 0 to 2,
$D^3$ and $D^4$ each independently represent an aromatic hydrocarbon ring group that may have a substituent or an aromatic heterocyclic group that may have a substituent, and
in the case where a plurality of Rc are present, the plurality of Rc may be same or different.

[8] The polymerizable compound according to [7], wherein $D^3$ and $D^4$ are each independently a group represented by any of the following Formulas (v-1) to (v-8):

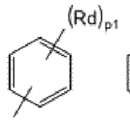
(v-1)
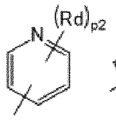
(v-2)
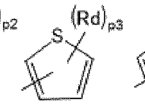
(v-3)
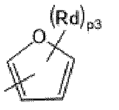
(v-4)
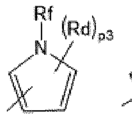
(v-5)
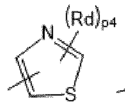
(v-6)
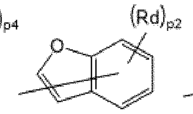
(v-7)
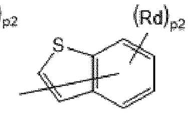
(v-8)

where Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p1 represents an integer of 0 to 5, p2 represents an integer of 0 to 4, p3 represents an integer of 0 to 3, and p4 represents an integer of 0 to 2,
Rf represents a hydrogen atom or a methyl group, and
in the case where a plurality of Rd are present, the plurality of Rd may be same or different.

[9] The polymerizable compound according to [7] or [8], wherein $Ar^1$ is a group represented by any of the following Formulas (VI-1) to (VI-5):

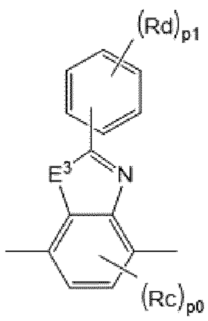
(VI-1)
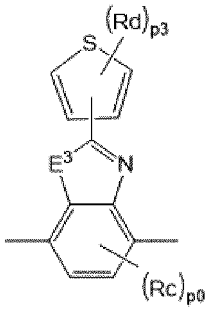
(VI-2)
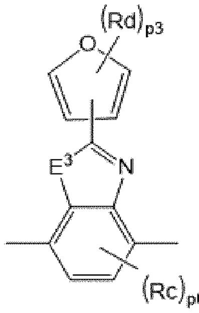
(VI-3)
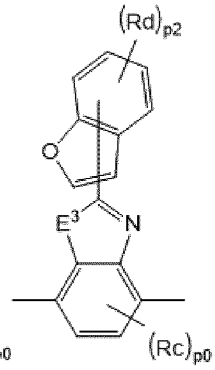
(VI-4)
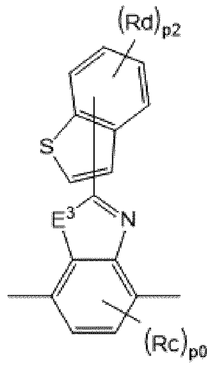
(VI-5)

where $E^3$, Rc, and p0 are as defined above,
Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p1 represents an integer of 0 to 5, p2 represents an integer of 0 to 4, and p3 represents an integer of 0 to 3, and in the case where a plurality of Rc and Rd are present, the plurality of Rc and Rd may be same or different.

[10] A mixture comprising:

the polymerizable compound according to any of [1] to [9]; and
a polymerizable compound represented by the following Formula (II):

$$P^{10a}-G^{10}-L^{10a}\left[B^{10}-L^{10}\right]_a A^{10}-Z^{10}-Ar^{10}-Z^{20}-A^{20}\left[L^{20}-B^{20}\right]_b L^{20a}-G^{20}-P^{20a}$$

(II)

where $Ar^{10}$ represents a divalent aromatic hydrocarbon ring group having at least $D^{10}$ as a substituent or a divalent aromatic heterocyclic group having at least $D^{10}$ as a substituent,

$D^{10}$ represents an organic group with a carbon number of 1 to 67 having at least one aromatic ring selected from the group consisting of an aromatic hydrocarbon ring and an aromatic heterocyclic ring,
$Z^{10}$ and $Z^{20}$ each independently represent a single bond, -O-, -O-$CH_2$-, -$CH_2$-O-, -C(=O)-O-, -O-C(=O)-, -C(=O)-S-, -S-C(=O)-, -$NR^{21}$-C(=O)-, -C(=O)-$NR^{21}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C≡C-, and $R^{21}$ each independently represent a hydrogen atom or an alkyl group with a carbon number of 1 to 6,
$A^{10}$ and $A^{20}$ and $B^{10}$ and $B^{20}$ each independently represent a cyclic aliphatic group that may have a substituent or an aromatic group that may have a substituent,
$L^{10}$ and $L^{20}$ and $L^{10a}$ and $L^{20a}$ each independently represent a single bond, -O-, -C(=O)-, -C(=O)-O-, -O-C(=O)-, -$NR^{22}$-C(=O)-, -C(=O)-$NR^{22}$-, -O-C(=O)-O-, -$NR^{22}$-C(=O)-O-, -O-C(=O)-$NR^{22}$-, or -$NR^{22}$-C(=O)-$NR^{23}$-, and $R^{22}$ and $R^{23}$ each independently represent a hydrogen atom or an alkyl group with a carbon number of 1 to 6,
$G^{10}$ and $G^{20}$ each independently represent an aliphatic hydrocarbon group with a carbon number of 3 to 20 that may have a substituent, the aliphatic hydrocarbon group with a carbon number of 3 to 20 may be interrupted by at least one intervening group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, -N=, =N-, and -N=N-, in the case where two or more intervening groups are present, the two or more intervening groups may be same or different and are not adjacent to each other, and R each independently represent a hydrogen atom or an alkyl group with a carbon number of 1 to 6,
$P^{10a}$ and $P^{20a}$ each independently represent a polymerizable group,
a and b each independently represent 0 or 1, and
a part represented by -$L^{10a}$-$G^{10}$-$P^{10a}$ and a part represented by -$L^{20a}$-$G^{20}$-$P^{20a}$ have a same structure.

[11] The mixture according to [10], wherein $Ar^{10}$ is a group represented by any of the following Formulas (III-1) to (III-3):

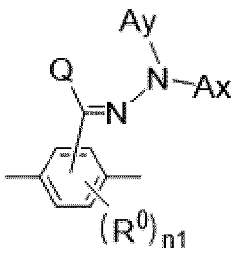

(III-1)

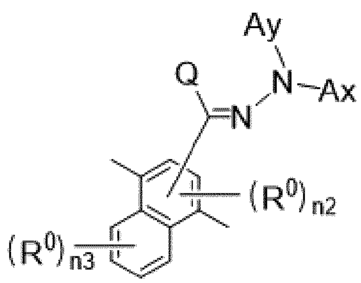

(III-2)

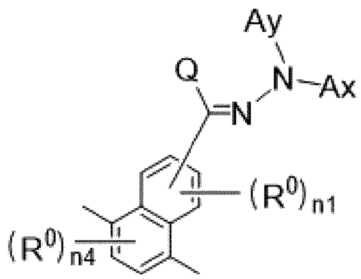

(III-3)

where Ax represents an organic group having at least one aromatic ring selected from the group consisting of an aromatic hydrocarbon ring with a carbon number of 6 to 30 and an aromatic heterocyclic ring with a carbon number of 2 to 30, and the aromatic ring of Ax may have a substituent,
Ay represents a hydrogen atom or an organic group with a carbon number of 1 to 30 that may have a substituent,
Q represents a hydrogen atom or an alkyl group with a carbon number of 1 to 6,
$R^0$ represents a halogen atom, a cyano group, an alkyl group with a carbon number of 1 to 6, an alkenyl group with a carbon number of 2 to 6, an alkyl halide group with a carbon number of 1 to 6, an N,N-dialkylamino group with a carbon number of 2 to 12, an alkoxy group with a carbon number of 1 to 6, a nitro group, -C(=O)-$R^a$, -C(=O)-O-$R^a$, or $SO_2R^a$, and $R^a$ represents an alkyl group with a carbon number of 1 to 6, or an aromatic hydrocarbon ring group with a carbon number of 6 to 20 that may have an alkyl group with a carbon number of 1 to 6 or an alkoxy group with a carbon number of 1 to 6 as a substituent,
n1 represents an integer of 0 to 3, n2 represents 0 or 1, n3 represents an integer of 0 to 4, and n4 represents an integer of 0 to 2, and
in the case where a plurality of $R^0$ are present, the plurality of $R^0$ may be same or different.

[12] The mixture according to [11], wherein $Ar^{10}$ is a group represented by any of the following Formulas (IV-1) to (IV-3):

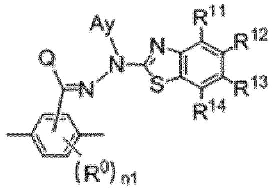
(IV-1)

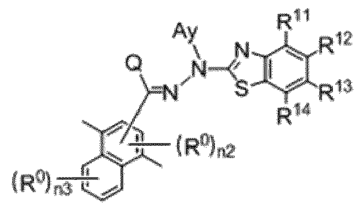
(IV-2)

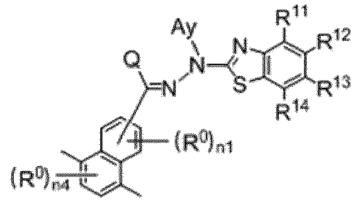
(IV-3)

where Ay, Q, $R^0$, n1, n2, n3, and n4 are as defined above, and
$R^{11}$ to $R^{14}$ each independently represent a hydrogen atom, a halogen atom, an alkyl group with a carbon number of 1 to 6, a cyano group, a nitro group, a fluoroalkyl group with a carbon number of 1 to 6, an alkoxy group with a carbon number of 1 to 6, or $-C(=O)-O-R^b$, $R^b$ represents an alkyl group with a carbon number of 1 to 20 that may have a substituent, an alkenyl group with a carbon number of 2 to 20 that may have a substituent, a cycloalkyl group with a carbon number of 3 to 12 that may have a substituent, or an aromatic hydrocarbon ring group with a carbon number of 5 to 12 that may have a substituent, and at least one of $C-R^{11}$ to $C-R^{14}$ forming a ring may be substituted by a nitrogen atom.

[13] The mixture according to [10], wherein $Ar^{10}$ is a group represented by any of the following Formulas (V-1) to (V-4):

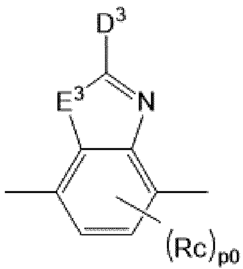
(V-1)

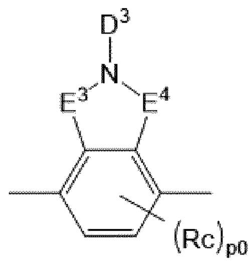
(V-2)

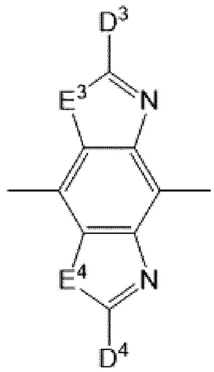
(V-3)

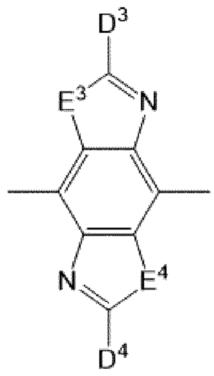
(V-4)

where $E^3$ and $E^4$ each independently represent $-CR^{24}R^{25}-$, -S-, $-NR^{24}-$, -C(=O)-, or -O-, and $R^{24}$ and $R^{25}$ each independently represent a hydrogen atom or an alkyl group with a carbon number of 1 to 4,
Rc represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p0 represents an integer of 0 to 2,
$D^3$ and $D^4$ each independently represent an aromatic hydrocarbon ring group that may have a substituent or an aromatic heterocyclic group that may have a substituent, and
in the case where a plurality of Rc are present, the plurality of Rc may be same or different.

[14] The mixture according to [13], wherein $D^3$ and $D^4$ are each independently a group represented by any of the following Formulas (v-1) to (v-8):

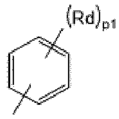 (v-1) 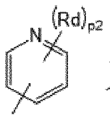 (v-2) 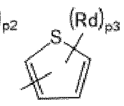 (v-3) 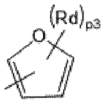 (v-4) 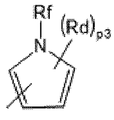 (v-5) 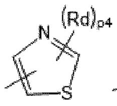 (v-6) 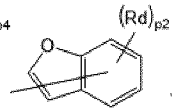 (v-7) 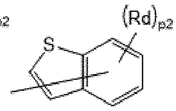 (v-8)

where Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,

p1 represents an integer of 0 to 5, p2 represents an integer of 0 to 4, p3 represents an integer of 0 to 3, and p4 represents an integer of 0 to 2,

Rf represents a hydrogen atom or a methyl group, and

in the case where a plurality of Rd are present, the plurality of Rd may be same or different.

[15] The mixture according to [13] or [14], wherein $Ar^{10}$ is a group represented by any of the following Formulas (VI-1) to (VI-5):

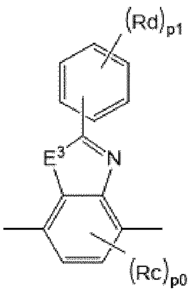 (VI-1) 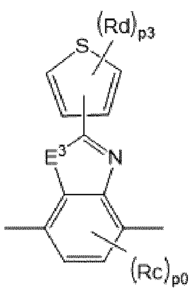 (VI-2) 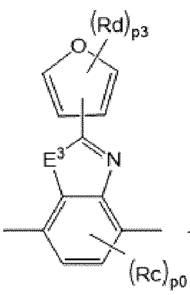 (VI-3) 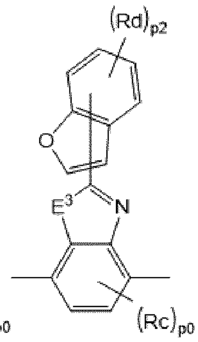 (VI-4) 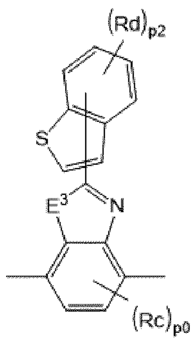 (VI-5)

where $E^3$, Rc, and p0 are as defined above,

Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,

p1 represents an integer of 0 to 5, p2 represents an integer of 0 to 4, and p3 represents an integer of 0 to 3, and in the case where a plurality of Rc and Rd are present, the plurality of Rc and Rd may be same or different.

[16] A polymer obtainable by polymerization of the polymerizable compound according to any of [1] to [9] or the mixture according to any of [10] to [15].

[17] An optical film comprising
the polymer according to [16] as a constituent material.

[18] An optically anisotropic product comprising
a layer having the polymer according to [16] as a constituent material.

[19] A polarizing plate comprising:

the optically anisotropic product according to [18]; and
a polarizing film.

[20] A display device comprising
the polarizing plate according to [19].

[21] An antireflection film comprising
the polarizing plate according to [19].

(Advantageous Effect)

It is therefore possible to provide a polymerizable compound that can form an optical film, etc. capable of uniform polarized light conversion over a wide wavelength range and can lower the temperature in the optical film production step, and a mixture and a polymer prepared using the polymerizable compound.

It is also possible to provide an optical film and an optically anisotropic product capable of uniform polarized light conversion over a wide wavelength range, and a polarizing plate, a display device, and an antireflection film using the same.

DETAILED DESCRIPTION

The presently disclosed techniques will be described in detail below. In the present disclosure, "may have a substituent" denotes "being unsubstituted or having a substituent". In the case where an organic group such as an alkyl group or an aromatic hydrocarbon ring group in a general formula has a substituent, the carbon number of the organic group having the substituent does not include the carbon number of the substituent. For example, in the case where an aromatic hydrocarbon ring group with a carbon number of 6 to 20 has a substituent, the carbon number of the aromatic hydrocarbon ring group with a carbon number of 6 to 20 does not include the carbon number of the substituent. In the present disclosure, the term "alkyl group" denotes a chain (linear or branched) saturated hydrocarbon group, and the term "alkyl group" does not include "cycloalkyl group" which is a cyclic saturated hydrocarbon group.

A presently disclosed polymerizable compound and a presently disclosed mixture containing the presently disclosed polymerizable compound can be, for example, mixed with a polymerization initiator to prepare a polymerizable liquid crystal composition, without being limited thereto.

The presently disclosed polymerizable compound, mixture, and polymerizable liquid crystal composition can be used, for example, in the preparation of a presently disclosed polymer, without being limited thereto.

The presently disclosed polymer can be used, for example, as a constituent material of a presently disclosed optical film and a constituent material of a layer of a presently disclosed optically anisotropic product, without being limited thereto. The presently disclosed optically anisotropic product can be used, for example, in a presently disclosed polarizing plate, without being limited thereto. The presently disclosed polarizing plate can be used, for example, in a display device such as a flat panel display device or an organic electroluminescent display device and an antireflection film, without being limited thereto.

(1) Polymerizable compound

The presently disclosed polymerizable compound is a compound represented by the following Formula (I) (hereafter also referred to as "polymerizable compound (I)"), and can be advantageously used in the preparation of the below-described mixture, polymer, optical film, and optically anisotropic product.

$$P^{1a}-G^{1}-L^{1a}\left[B^{1}-L^{1}\right]_{a}A^{1}-Z^{1}-Ar^{1}-Z^{2}-A^{2}\left[L^{2}-B^{2}\right]_{b}L^{2a}-G^{2}-P^{2a}$$

(I)

The polymerizable compound (I) has low liquid crystallization temperature and excellent coatability, and can form an optical film, etc. capable of uniform polarized light conversion over a wide wavelength range.

The reason for this is not clear, but is presumed to be as follows. As described below, in the polymerizable compound (I), the part represented by "$-L^{1a}-G^{1}-P^{1a}$" and the part represented by "$-L^{2a}-G^{2}-P^{2a}$" located at the respective ends have different structures (i.e. the part represented by "$-L^{1a}-G^{1}-P^{1a}$" and the part represented by "$-L^{2a}-G^{2}-P^{2a}$" are asymmetric in structure with respect to the $Ar^1$ side as the center of symmetry). Therefore, the polymerizable compound (I) easily changes to liquid crystalline phase at lower temperature (i.e. is easily supercooled at room temperature) while ensuring optical properties (in particular, reverse wavelength dispersibility), as compared with a polymerizable compound in which both ends have the same structure (i.e. the part represented by "$-L^{1a}-G^{1}-P^{1a}$" and the part represented by "$-L^{2a}-G^{2}-P^{2a}$" are symmetric in structure with respect to the $Ar^1$ side as the center of symmetry).

In Formula (I), a and b are each independently 0 or 1, and preferably 1.

$Ar^1$ is a divalent aromatic hydrocarbon ring group having at least $D^1$ as a substituent or a divalent aromatic

heterocyclic group having at least $D^1$ as a substituent. $D^1$ is an organic group with a carbon number of 1 to 67 having at least one aromatic ring selected from the group consisting of an aromatic hydrocarbon ring and an aromatic heterocyclic ring.

Herein, the divalent aromatic hydrocarbon ring group having at least $D^1$ as a substituent or the divalent aromatic heterocyclic group having at least $D^1$ as a substituent is a group obtained by removing, from a ring fragment of the aromatic hydrocarbon ring to which $D^1$ is bound or the aromatic heterocyclic ring to which $D^1$ is bound, two hydrogen atoms bound to carbon atoms other than the carbon atom to which $D^1$ is bound. The divalent aromatic hydrocarbon ring group and the divalent aromatic heterocyclic group for $Ar^1$ may have one or more substituents other than $D^1$. In the case where the divalent aromatic hydrocarbon ring group and the divalent aromatic heterocyclic group for $Ar^1$ have a plurality of substituents, the plurality of substituents may be the same or different.

Examples of the divalent aromatic hydrocarbon ring group for $Ar^1$ include, but are not limited to, 1,4-phenylene group, 1,3-phenylene group, 1,4-naphthylene group, 2,6-naphthylene group, 1,5-naphthylene group, anthracenyl-9,10-diyl group, anthracenyl-1,4-diyl group, and anthracenyl-2,6-diyl group.

Examples of the divalent aromatic heterocyclic group for $Ar^1$ include, but are not limited to, benzothiazole-4,7-diyl group, 1,2-benzisothiazole-4,7-diyl group, benzoxazole-4,7-diyl group, indole-4,7-diyl group, benzimidazole-4,7-diyl group, benzopyrazole-4,7-diyl group, 1-benzofuran-4,7-diyl group, 2-benzofuran-4,7-diyl group, benzo[1,2-d:4,5-d']dithiazolyl-4,8-diyl group, benzo[1,2-d:5,4-d']dithiazolyl-4,8-diyl group, benzothiophenyl-4,7-diyl group, 1H-isoindole-1,3(2H)-dione-4,7-diyl group, benzo[1,2-b:5,4-b']dithiophenyl-4,8-diyl group, benzo[1,2-b:4,5-b']dithiophenyl-4,8-diyl group, benzo[1,2-b:5,4-b'] difuranyl-4,8-diyl group, benzo[1,2-b:4,5-b'] difuranyl-4,8-diyl group, benzo[2,1-b:4,5-b']dipyrrole-4,8-diyl group, benzo[1,2-b:5,4-b']dipyrrole-4,8-diyl group, and benzo[1,2-d:4,5-d']diimidazole-4,8-diyl group.

Examples of the substituent(s) other than $D^1$ of the divalent aromatic hydrocarbon ring group and the divalent aromatic heterocyclic group for $Ar^1$ include, but are not limited to, a halogen atom, a cyano group, a nitro group, an alkyl group with a carbon number of 1 to 6, an alkenyl group with a carbon number of 2 to 6, an alkyl halide group with a carbon number of 1 to 6, an N-alkylamino group with a carbon number of 1 to 6, an N,N-dialkylamino group with a carbon number of 2 to 12, an alkoxy group with a carbon number of 1 to 6, an alkylsulfinyl group with a carbon number of 1 to 6, a carboxyl group, a thioalkyl group with a carbon number of 1 to 6, an N-alkylsulfamoyl group with a carbon number of 1 to 6, an N,N-dialkylsulfamoyl group with a carbon number of 2 to 12, $-C(=O)-R^a$, $-C(=O)-O-R^a$, and $SO_2R^a$. $R^a$ is an alkyl group with a carbon number of 1 to 6, or an aromatic hydrocarbon ring group with a carbon number of 6 to 20 that may have an alkyl group with a carbon number of 1 to 6 or an alkoxy group with a carbon number of 1 to 6 as a substituent. Examples of the substituent(s) other than $D^1$ include an organic group with a carbon number of 1 to 67 having at least one aromatic ring selected from the group consisting of an aromatic hydrocarbon ring and an aromatic heterocyclic ring.

Examples of the halogen atom for the substituent(s) other than $D^1$ include fluorine atom, chlorine atom, bromine atom, and iodine atom.

Examples of the alkyl group with a carbon number of 1 to 6 for the substituent(s) other than $D^1$ include methyl group, ethyl group, n-propyl group, isopropyl group, n-butyl group, isobutyl group, sec-butyl group, tert-butyl group, pentyl group, and hexyl group.

Examples of the alkenyl group with a carbon number of 2 to 6 for the substituent(s) other than $D^1$ include vinyl group, propenyl group, isopropenyl group, butenyl group, isobutenyl group, pentenyl group, and hexenyl group.

Examples of the alkyl halide group with a carbon number of 1 to 6 for the substituent(s) other than $D^1$ include fluoroalkyl group with a carbon number of 1 to 6 such as fluoromethyl group, trifluoromethyl group, fluoroethyl group, pentafluoroethyl group, heptafluoropropyl group, and nonafluorobutyl group.

Examples of the N-alkylamino group with a carbon number of 1 to 6 for the substituent(s) other than $D^1$ include N-methylamino group, N-ethylamino group, N-propylamino group, N-isopropylamino group, N-butylamino group, N-isobutylamino group, N-sec-butylamino group, N-tert-butylamino group, N-pentylamino group, and N-hexylamino group.

Examples of the N,N-dialkylamino group with a carbon number of 2 to 12 for the substituent(s) other than $D^1$ include N,N-dimethylamino group, N-methyl-N-ethylamino group, N,N-diethylamino group, N,N-dipropylamino group, N,N-diisopropylamino group, N,N-dibutylamino group, N,N-diisobutylamino group, N,N-dipentylamino group, and N,N-dihexylamino group.

Examples of the alkoxy group with a carbon number of 1 to 6 for the substituent(s) other than $D^1$ include methoxy group, ethoxy group, propoxy group, isopropoxy group, butoxy group, isobutoxy group, sec-butoxy group, tert-butoxy group, pentyloxy group, and hexyloxy group.

Examples of the alkylsulfinyl group with a carbon number of 1 to 6 for the substituent(s) other than $D^1$ include methylsulfinyl group, ethylsulfinyl group, propylsulfinyl group, isopropylsulfinyl group, butylsulfinyl group, isobutylsulfinyl group, sec-butylsulfinyl group, tert-butylsulfinyl group, pentylsulfinyl group, and hexylsulfinyl group.

Examples of the thioalkyl group with a carbon number of 1 to 6 for the substituent(s) other than $D^1$ include methylthio group, ethylthio group, propylthio group, isopropylthio group, butylthio group, isobutylthio group, sec-butylthio group, tert-butylthio group, pentylthio group, and hexylthio group.

Examples of the N-alkylsulfamoyl group with a carbon number of 1 to 6 for the substituent(s) other than $D^1$ include N-methylsulfamoyl group, N-ethylsulfamoyl group, N-propylsulfamoyl group, N-isopropylsulfamoyl group, N-butylsulfamoyl group, N-isobutylsulfamoyl group, N-sec-butylsulfamoyl group, N-tert-butylsulfamoyl group, N-pentylsulfamoyl group, and N-hexylsulfamoyl group.

Examples of the N,N-dialkylsulfamoyl group with a carbon number of 2 to 12 for the substituent(s) other than $D^1$ include N,N-dimethylsulfamoyl group, N-methyl-N-ethylsulfamoyl group, N,N-diethylsulfamoyl group, N,N-dipropylsulfamoyl group, N,N-diisopropylsulfamoyl group, N,N-dibutylsulfamoyl group, N,N-diisobutylsulfamoyl group, N,N-dipentylsulfamoyl group, and N,N-dihexylsulfamoyl group.

Examples of the alkyl group with a carbon number of 1 to 6 for $R^a$ include methyl group, ethyl group, n-propyl group, isopropyl group, n-butyl group, isobutyl group, sec-butyl group, tert-butyl group, pentyl group, and hexyl group.

Examples of the aromatic hydrocarbon ring group with a carbon number of 6 to 20 that may have alkyl group with a carbon number of 1 to 6 or alkoxy group with a carbon number of 1 to 6 as a substituent for $R^a$ include phenyl group and naphthyl group that may have alkyl group with a carbon number of 1 to 6 such as methyl group, ethyl group, n-propyl group, isopropyl group, n-butyl group, isobutyl group, sec-butyl group, tert-butyl group, pentyl group, and hexyl group or alkoxy group with a carbon number of 1 to 6 such as methoxy group and ethoxy group as a substituent.

Examples of the organic group with a carbon number of 1 to 67 having at least one aromatic ring selected from the group consisting of an aromatic hydrocarbon ring and an aromatic heterocyclic ring for the substituent(s) other than $D^1$ include, but are not limited to, the same organic groups as those described in detail below as $D^1$.

In the present disclosure, "aromatic ring" denotes a cyclic structure having aromaticity in a broad sense according to Huckel's rule, i.e. a cyclic conjugated structure having $(4n + 2)$ $\pi$ electrons, and a cyclic structure exhibiting aromaticity due to involvement of a lone electron pair of a heteroatom such as sulfur, oxygen, or nitrogen in $\pi$ electron system, such as thiophene, furan, or benzothiazole.

Examples of the aromatic hydrocarbon ring for $D^1$ include, but are not limited to, benzene ring, naphthalene ring, anthracene ring, phenanthrene ring, pyrene ring, and fluorene ring.

Examples of the aromatic heterocyclic ring for $D^1$ include, but are not limited to, 1H-isoindole-1,3(2H)-dione ring, 1-benzofuran ring, 2-benzofuran ring, acridine ring, isoquinoline ring, imidazole ring, indole ring, oxadiazole ring, oxazole ring, oxazolopyrazine ring, oxazolopyridine ring, oxazolopyridazine ring, oxazolopyrimidine ring, quinazoline ring, quinoxaline ring, quinoline ring, cinnoline ring, thiadiazole ring, thiazole ring, thiazolopyrazine ring, thiazolopyridine ring, thiazolopyridazine ring, thiazolopyrimidine ring, thiophene ring, triazine ring, triazole ring, naphthyridine ring, pyrazine ring, pyrazole ring, pyranone ring, pyran ring, pyridine ring, pyridazine ring, pyrimidine ring, pyrrole ring, phenanthridine ring, phthalazine ring, furan ring, benzo[c]thiophene ring, benzoisooxazole ring, benzoisothiazole ring, benzimidazole ring, benzooxadiazole ring, benzoxazole ring, benzothiadiazole ring, benzothiazole ring, benzothiophene ring, benzotriazine ring, benzotriazole ring, benzopyrazole ring, benzopyranone ring, dihydropyran ring, tetrahydropyran ring, dihydrofuran ring, and tetrahydrofuran ring.

The aromatic hydrocarbon ring and the aromatic heterocyclic ring for $D^1$ may be substituted by a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, an N,N-dialkylsulfamoyl group with a carbon number of 2 to 12, or -C(=O)-O-$R^b$. Here, $R^b$ is an alkyl group with a carbon number of 1 to 20 that may have a substituent, an alkenyl group with a carbon number of 2 to 20 that may have a substituent, a cycloalkyl group with a carbon number of 3 to 12 that may have a substituent, or an aromatic hydrocarbon ring group with a carbon number of 5 to 12 that may have a substituent.

The aromatic hydrocarbon ring and the aromatic heterocyclic ring may have one or more substituents selected from the foregoing substituents. In the case where the aromatic hydrocarbon ring and the aromatic heterocyclic ring have a plurality of substituents, the plurality of substituents may be the same or different.

Examples of the halogen atom, the alkyl group with a carbon number of 1 to 6, the alkylsulfinyl group with a carbon number of 1 to 6, the alkoxy group with a carbon number of 1 to 6, the thioalkyl group with a carbon number of 1 to 6, the N-alkylamino group with a carbon number of 1 to 6, the N,N-dialkylamino group with a carbon number of 2 to 12, the N-alkylsulfamoyl group with a carbon number of 1 to 6, and the N,N-dialkylsulfamoyl group with a carbon number of 2 to 12 which the aromatic hydrocarbon ring and the aromatic heterocyclic ring for $D^1$ may have are the same as those listed above as the substituent(s) other than $D^1$.

Examples of the alkylsulfonyl group with a carbon number of 1 to 6 which the aromatic hydrocarbon ring and the aromatic heterocyclic ring for $D^1$ may have include methylsulfonyl group, ethylsulfonyl group, propylsulfonyl group, isopropylsulfonyl group, butylsulfonyl group, isobutylsulfonyl group, sec-butylsulfonyl group, tert-butylsulfonyl group, pentylsulfonyl group, and hexylsulfonyl group.

Examples of the fluoroalkyl group with a carbon number of 1 to 6 which the aromatic hydrocarbon ring and the aromatic heterocyclic ring for $D^1$ may have include fluoromethyl group, trifluoromethyl group, fluoroethyl group, pentafluoroethyl group, heptafluoropropyl group, and nonafluorobutyl group.

Examples of the alkyl group with a carbon number of 1 to 20 for $R^b$ include methyl group, ethyl group, n-propyl group, isopropyl group, n-butyl group, isobutyl group, 1-methylpentyl group, 1-ethylpentyl group, sec-butyl group, t-butyl group, n-pentyl group, isopentyl group, neopentyl group, n-hexyl group, isohexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, n-nonadecyl group, and n-icosyl group.

Examples of the alkenyl group with a carbon number of 2 to 20 for $R^b$ include vinyl group, propenyl group, isopropenyl group, butenyl group, isobutenyl group, pentenyl group, hexenyl group, heptenyl group, octenyl group, decenyl group, undecenyl group, dodecenyl group, tridecenyl group, tetradecenyl group, pentadecenyl group, hexadecenyl group, heptadecenyl group, octadecenyl group, nonadecenyl group, and icosenyl group.

Examples of the cycloalkyl group with a carbon number of 3 to 12 for $R^b$ include cyclopropyl group, cyclobutyl group, cyclopentyl group, cyclohexyl group, and cyclooctyl group.

Examples of the aromatic hydrocarbon ring group with a carbon number of 5 to 12 for $R^b$ include phenyl group, 1-naphthyl group, and 2-naphthyl group.

Examples of the substituent of the alkyl group with a carbon number of 1 to 20 that may have a substituent, the alkenyl group with a carbon number of 2 to 20 that may have a substituent, and the aromatic hydrocarbon ring group with a carbon number of 5 to 12 that may have a substituent for $R^b$ include a halogen atom such as fluorine atom and chlorine atom; a cyano group; an alkoxy group with a carbon number of 1 to 20 such as methoxy group, ethoxy group, isopropoxy group, and butoxy group; a nitro group; an aromatic hydrocarbon ring group with a carbon number of 6 to 20 such as phenyl group and naphthyl group; an aromatic heterocyclic group with a carbon number of 2 to 20 such as furanyl group and thiophenyl group; a cycloalkyl group with a carbon number of 3 to 8 such as cyclopropyl group, cyclopentyl group, and cyclohexyl group; and a fluoroalkyl group with a carbon number of 1 to 12 at least one hydrogen atom of which is substituted by a fluorine atom, such as trifluoromethyl group, pentafluoroethyl group, and $-CH_2CF_3$. The alkyl group with a carbon number of 1 to 20, the alkenyl group with a carbon number of 2 to 20, and the aromatic hydrocarbon ring group with a carbon number of 5 to 12 for $R^b$ may have one or more substituents selected from the foregoing substituents. In the case where the alkyl group with a carbon number of 1 to 20, the alkenyl group with a carbon number of 2 to 20, and the aromatic hydrocarbon ring group with a carbon number of 5 to 12 have a plurality of substituents, the plurality of substituents may be the same or different.

Examples of the substituent of the cycloalkyl group with a carbon number of 3 to 12 for $R^b$ include a halogen atom such as fluorine atom and chlorine atom; a cyano group; an alkyl group with a carbon number of 1 to 6 such as methyl group, ethyl group, and propyl group; an alkoxy group with a carbon number of 1 to 6 such as methoxy group, ethoxy group, and isopropoxy group; a nitro group; and an aromatic hydrocarbon group with a carbon number of 6 to 20 such as phenyl group and naphthyl group. The cycloalkyl group with a carbon number of 3 to 12 for $R^b$ may have one or more substituents selected from the foregoing substituents. In the case where the cycloalkyl group with a carbon number of 3 to 12 has a plurality of substituents, the plurality of substituents may be the same or different.

Examples of $Ar^1$ (divalent aromatic hydrocarbon ring group having at least $D^1$ as a substituent or divalent aromatic heterocyclic group having at least $D^1$ as a substituent) include phenylene group substituted by a group represented by formula: $-C(R^f)=N-N(R^g)R^h$ or formula: $-C(R^f)=N-N=C(R^{g1})R^h$, benzothiazole-4,7-diyl group substituted by 1-benzofuran-2-yl group, benzothiazole-4,7-diyl group substituted by 5-(2-butyl)-1-benzofuran-2-yl group, benzothiazole-4,7-diyl group substituted by 4,6-dimethyl-1-benzofuran-2-yl group, benzothiazole-4,7-diyl group substituted by 6-methyl-1-benzofuran-2-yl group, benzothiazole-4,7-diyl group substituted by 4,6,7-trimethyl-1-benzofuran-2-yl group, benzothiazole-4,7-diyl group substituted by 4,5,6-trimethyl-1-benzofuran-2-yl group, benzothiazole-4,7-diyl group substituted by 5-methyl-1-benzofuran-2-yl group, benzothiazole-4,7-diyl group substituted by 5-propyl-1-benzofuran-2-yl group, benzothiazole-4,7-diyl group substituted by 7-propyl-1-benzofuran-2-yl group, benzothiazole-4,7-diyl group substituted by 5-fluorol-benzofuran-2-yl group, benzothiazole-4,7-diyl group substituted by phenyl group, benzothiazole-4,7-diyl group substituted by 4-fluorophenyl group, benzothiazole-4,7-diyl group substituted by 4-nitrophenyl group, benzothiazole-4,7-diyl group substituted by 4-trifluoromethylphenyl group, benzothiazole-4,7-diyl group substituted by 4-cyanophenyl group, benzothiazole-4,7-diyl group substituted by 4-methanesulfonylphenyl group, benzothiazole-4,7-diyl group substituted by thiophene-2-yl group, benzothiazole-4,7-diyl group substituted by thiophene-3-yl group, benzothiazole-4,7-diyl group substituted by 5-methylthiophene-2-yl group, benzothiazole-4,7-diyl group substituted by 5-chlorothiophene-2-yl group, benzothiazole-4,7-diyl group substituted by thieno[3,2-b]thiophene-2-yl group, benzothiazole-4,7-diyl group substituted by 2-benzothiazolyl group, benzothiazole-4,7-diyl group substituted by 4-biphenyl group, benzothiazole-4,7-diyl group substituted by 4-propylbiphenyl group, benzothiazole-4,7-diyl group substituted by 4-thiazolyl group, benzothiazole-4,7-diyl group substituted by 1-phenylethylene-2-yl group, benzothiazole-4,7-diyl group substituted by 4-pyridiyl group, benzothiazole-4,7-diyl group substituted by 2-furyl group, benzothiazole-4,7-diyl group substituted by naphth[1,2-b]furan-2-yl group, 1H-isoindole-1,3(2H)-dione-4,7-diyl group substituted by 5-methoxy-2-benzothiazolyl group, 1H-isoindole-1,3(2H)-dione-4,7-diyl group substituted by phenyl group, 1H-isoindole-1,3(2H)-dione-4,7-diyl group substituted by 4-nitrophenyl group, and 1H-isoindole-1,3(2H)-dione-4,7-diyl group substituted by 2-thiazolyl group. In the foregoing formulas, Reach independently represent a hydrogen atom, or an alkyl group with a carbon number of 1 to 6 such as methyl group, ethyl group, propyl group, and isopropyl group. In the foregoing formulas, $R^g$ and $R^{g1}$ represent a hydrogen atom, or an organic group with a carbon number of 1 to 30 that may have a substituent. Examples of the organic group with a carbon number of 1 to 30 and its substituent are the same as those listed as specific examples of the organic group with a carbon number of 1 to 30 and its substituent for Ay described later. In the foregoing formulas, $R^h$ represents an organic group having at least one aromatic ring selected from the group consisting of an aromatic hydrocarbon ring with a carbon number of 6 to 30 and an aromatic heterocyclic ring with a carbon number of 2 to 30. Specific examples of the organic group having at least one aromatic ring selected from the group consisting of an aromatic hydrocarbon ring with a carbon number of 6 to 30 and an aromatic heterocyclic ring with a carbon number of 2 to 30 are the same as those listed as specific examples of the organic group having at least one aromatic ring selected from the group consisting of an aromatic hydrocarbon ring with a carbon number of 6 to 30 and an aromatic heterocyclic ring with a carbon number of 2 to 30 for Ax described later.

$Ar^1$ (divalent aromatic hydrocarbon ring having at least $D^1$ as a substituent or divalent aromatic heterocyclic group having at least $D^1$ as a substituent) is preferably a divalent aromatic hydrocarbon ring group or a divalent aromatic heterocyclic group that may have a substituent and that has a group represented by formula: -C(Q)=N-N(Ax)Ay as $D^1$, more preferably a divalent aromatic hydrocarbon ring group that may have a substituent and that has a group represented by formula: -C(Q)=N-N(Ax)Ay as $D^1$, and further preferably a group represented by any of the following Formulas (III-1) to (III-3).

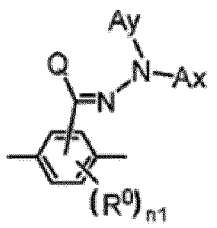
(III-1)

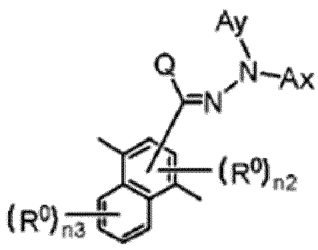
(III-2)

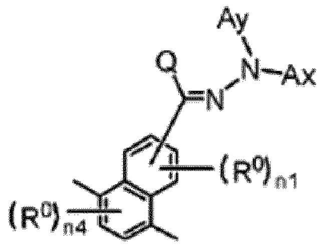
(III-3)

Here, Ax is an organic group having at least one aromatic ring selected from the group consisting of an aromatic hydrocarbon ring with a carbon number of 6 to 30 and an aromatic heterocyclic ring with a carbon number of 2 to 30. The aromatic ring of Ax may have a substituent. Ax may have a plurality of aromatic rings.

Examples of the aromatic hydrocarbon ring with a carbon number of 6 to 30 for Ax are the same as those listed as the aromatic hydrocarbon ring for $D^1$. Of these, the aromatic hydrocarbon ring with a carbon number of 6 to 30 for Ax is preferably benzene ring, naphthalene ring, or anthracene ring.

Examples of the aromatic heterocyclic ring with a carbon number of 2 to 30 for Ax are the same as those listed as the aromatic heterocyclic ring for $D^1$. Of these, the aromatic heterocyclic ring with a carbon number of 2 to 30 for Ax is preferably: a monocyclic aromatic heterocyclic ring such as furan ring, thiophene ring, oxazole ring, and thiazole ring; or a condensed cyclic aromatic heterocyclic ring such as benzothiazole ring, benzoxazole ring, quinoline ring, 1-benzofuran ring, 2-benzofuran ring, benzothiophene ring, thiazolopyridine ring, and thiazolopyrazine ring.

The aromatic ring of Ax may have a substituent. Examples of the substituent include a halogen atom, an alkyl group with a carbon number of 1 to 6, a cyano group, a nitro group, a fluoroalkyl group with a carbon number of 1 to 6, an alkoxy group with a carbon number of 1 to 6, and -C(=O)-O-$R^b$. $R^b$ is an alkyl group with a carbon number of 1 to 20 that may have a substituent, an alkenyl group with a carbon number of 2 to 20 that may have a substituent, a cycloalkyl group with a carbon number of 3 to 12 that may have a substituent, or an aromatic hydrocarbon ring group with a carbon number of 5 to 12 that may have a substituent.

Ax may have a plurality of substituents selected from the foregoing substituents. In the case where Ax has a plurality of substituents, the substituents may be the same or different.

Examples of the halogen atom, the alkyl group with a carbon number of 1 to 6, the fluoroalkyl group with a carbon number of 1 to 6, the alkoxy group with a carbon number of 1 to 6, and -C(=O)-O-$R^b$ of the substituent of the aromatic ring of Ax are the same as those listed as the substituent which the aromatic hydrocarbon ring and the aromatic heterocyclic ring for $D^1$ may have.

Of these, the substituent of the aromatic ring of Ax is preferably a halogen atom, a cyano group, an alkyl group with a carbon number of 1 to 6, or an alkoxy group with a carbon number of 1 to 6.

The carbon number of the alkyl group with a carbon number of 1 to 20 that may have a substituent for $R^b$ is

preferably 1 to 12, and further preferably 4 to 10.

The carbon number of the alkenyl group with a carbon number of 2 to 20 that may have a substituent for $R^b$ is preferably 2 to 12.

The cycloalkyl group of the cycloalkyl group with a carbon number of 3 to 12 that may have a substituent for $R^b$ is preferably cyclopentyl group or cyclohexyl group.

The aromatic hydrocarbon ring group of the aromatic hydrocarbon ring group with a carbon number of 5 to 12 that may have a substituent for $R^b$ is preferably phenyl group.

The substituent of the alkyl group with a carbon number of 1 to 20, the substituent of the alkenyl group with a carbon number of 2 to 20, and the substituent of the aromatic hydrocarbon ring group with a carbon number of 5 to 12 for $R^b$ are preferably: a halogen atom such as fluorine atom and chlorine atom; a cyano group; an alkoxy group with a carbon number of 1 to 20 such as methoxy group, ethoxy group, isopropoxy group, and butoxy group; a nitro group; an aromatic hydrocarbon ring group with a carbon number of 6 to 20 such as phenyl group and naphthyl group; an aromatic heterocyclic group with a carbon number of 2 to 20 such as furanyl group and thiophenyl group; a cycloalkyl group with a carbon number of 3 to 8 such as cyclopropyl group, cyclopentyl group, and cyclohexyl group; and a fluoroalkyl group with a carbon number of 1 to 12 at least one hydrogen atom of which is substituted by a fluorine atom, such as trifluoromethyl group, pentafluoroethyl group, and $-CH_2CF_3$.

The alkyl group with a carbon number of 1 to 20, the alkenyl group with a carbon number of 2 to 20, and the aromatic hydrocarbon ring group with a carbon number of 5 to 12 for $R^b$ may have a plurality of substituents selected from the foregoing substituents. In the case where the alkyl group with a carbon number of 1 to 20, the alkenyl group with a carbon number of 2 to 20, and the aromatic hydrocarbon ring group with a carbon number of 5 to 12 for $R^b$ have a plurality of substituents, the plurality of substituents may be the same or different.

The substituent of the cycloalkyl group with a carbon number of 3 to 12 for $R^b$ is preferably: a halogen atom such as fluorine atom and chlorine atom; a cyano group; an alkyl group with a carbon number of 1 to 6 such as methyl group, ethyl group, and propyl group; an alkoxy group with a carbon number of 1 to 6 such as methoxy group, ethoxy group, and isopropoxy group; a nitro group; and an aromatic hydrocarbon group with a carbon number of 6 to 20 such as phenyl group and naphthyl group.

The cycloalkyl group with a carbon number of 3 to 12 for $R^b$ may have a plurality of substituents selected from the foregoing substituents. In the case where the cycloalkyl group with a carbon number of 3 to 12 for $R^b$ has a plurality of substituents, the plurality of substituents may be the same or different.

The aromatic ring of Ax may have a plurality of substituents that may be the same or different, and two adjacent substituents may be joined together to form a ring which may be a monocyclic, condensed polycyclic, unsaturated, or saturated ring.

The "carbon number" of the organic group with a carbon number of 2 to 20 for Ax denotes the total carbon number of the whole organic group excluding the carbon atom of the substituent.

Examples of the organic group having at least one aromatic ring selected from the group consisting of an aromatic hydrocarbon ring with a carbon number of 6 to 30 and an aromatic heterocyclic ring with a carbon number of 2 to 30 for Ax include the following 1) to 5):

1) hydrocarbon ring group with a carbon number of 6 to 40 having at least one aromatic hydrocarbon ring with a carbon number of 6 to 30;
2) heterocyclic group with a carbon number of 2 to 40 having at least one aromatic ring selected from the group consisting of an aromatic hydrocarbon ring with a carbon number of 6 to 30 and an aromatic heterocyclic ring with a carbon number of 2 to 30;
3) alkyl group with a carbon number of 1 to 12 substituted by at least one of an aromatic hydrocarbon ring group with a carbon number of 6 to 30 and an aromatic heterocyclic group with a carbon number of 2 to 30;
4) alkenyl group with a carbon number of 2 to 12 substituted by at least one of an aromatic hydrocarbon ring group with a carbon number of 6 to 30 and an aromatic heterocyclic group with a carbon number of 2 to 30; and
5) alkynyl group with a carbon number of 2 to 12 substituted by at least one of an aromatic hydrocarbon ring group with a carbon number of 6 to 30 and an aromatic heterocyclic group with a carbon number of 2 to 30.

Specific examples of the aromatic hydrocarbon ring in 1) "hydrocarbon ring group with a carbon number of 6 to 40 having at least one aromatic hydrocarbon ring with a carbon number of 6 to 30" are the same as those listed as specific examples of the aromatic hydrocarbon ring of Ax. Examples of the hydrocarbon ring group in 1) include aromatic hydrocarbon ring group with a carbon number of 6 to 30 (e.g. phenyl group, naphthyl group, anthracenyl group, phenanthrenyl group, pyrenyl group, and fluorenyl group), indanyl group, 1,2,3,4-tetrahydronaphthyl group, and 1,4-dihydronaphthyl group.

Specific examples of the aromatic hydrocarbon ring and the aromatic heterocyclic ring in 2) "heterocyclic group with a carbon number of 2 to 40 having at least one aromatic ring selected from the group consisting of an aromatic

hydrocarbon ring with a carbon number of 6 to 30 and an aromatic heterocyclic ring with a carbon number of 2 to 30" are the same as those listed as specific examples of the aromatic hydrocarbon ring and the aromatic heterocyclic ring of Ax. Examples of the heterocyclic group in 2) include aromatic heterocyclic group with a carbon number of 2 to 30 (e.g. phthalimide group, 1-benzofuranyl group, 2-benzofuranyl group, acrydinyl group, isoquinolinyl group, imidazolyl group, indolinyl group, furazanyl group, oxazolyl group, oxazolopyrazinyl group, oxazolopyridinyl group, oxazolopyridazinyl group, oxazolopyrimidinyl group, quinazolinyl group, quinoxalinyl group, quinolyl group, cinnolinyl group, thiadiazolyl group, thiazolyl group, thiazolopyrazinyl group, thiazolopyridinyl group, thiazolopyridazinyl group, thiazolopyrimidinyl group, 2-thienyl group, 3-thienyl group, triazinyl group, triazolyl group, naphthyridinyl group, pyrazinyl group, pyrazolyl group, pyranonyl group, pyranyl group, pyridyl group, pyridazinyl group, pyrimidinyl group, pyrrolyl group, phenanthridinyl group, phthalazinyl group, furanyl group, benzo[c]thienyl group, benzoisoxazolyl group, benzoisothiazolyl group, benzoimidazolyl group, benzoxazolyl group, benzothiadiazolyl group, benzothiazolyl group, benzothiophenyl group, benzotriadinyl group, benzotriazolyl group, benzopyrazolyl group, benzopyranonyl group, dihydropyranyl group, tetrahydropyranyl group, dihydrofuranyl group, and tetrahydrofuranyl group), 2,3-dihydroindolyl group, 9,10-dihydroacridinyl group, and 1,2,3,4-tetrahydroquinolyl group.

Specific examples of the alkyl group with a carbon number of 1 to 12 in 3) "alkyl group with a carbon number of 1 to 12 substituted by at least one of an aromatic hydrocarbon ring group with a carbon number of 6 to 30 and an aromatic heterocyclic group with a carbon number of 2 to 30" include methyl group, ethyl group, propyl group, and isopropyl group. Specific examples of the aromatic hydrocarbon ring group with a carbon number of 6 to 30 and the aromatic heterocyclic group with a carbon number of 2 to 30 in 3) are the same as those listed as specific examples of the aromatic hydrocarbon ring group with a carbon number of 6 to 30 and the aromatic heterocyclic group with a carbon number of 2 to 30 in 1) and 2).

Specific examples of the alkenyl group with a carbon number of 2 to 12 in 4) "alkenyl group with a carbon number of 2 to 12 substituted by at least one of an aromatic hydrocarbon ring group with a carbon number of 6 to 30 and an aromatic heterocyclic group with a carbon number of 2 to 30" include vinyl group and allyl group. Specific examples of the aromatic hydrocarbon ring group with a carbon number of 6 to 30 and the aromatic heterocyclic group with a carbon number of 2 to 30 in 4) are the same as those listed as specific examples of the aromatic hydrocarbon ring group with a carbon number of 6 to 30 and the aromatic heterocyclic group with a carbon number of 2 to 30 in 1) and 2).

Specific examples of the alkynyl group with a carbon number of 2 to 12 in 5) "alkynyl group with a carbon number of 2 to 12 substituted by at least one of an aromatic hydrocarbon ring group with a carbon number of 6 to 30 and an aromatic heterocyclic group with a carbon number of 2 to 30" include ethynyl group and propynyl group. Specific examples of the aromatic hydrocarbon ring group with a carbon number of 6 to 30 and the aromatic heterocyclic group with a carbon number of 2 to 30 in 5) are the same as those listed as specific examples of the aromatic hydrocarbon ring group with a carbon number of 6 to 30 and the aromatic heterocyclic group with a carbon number of 2 to 30 in 1) and 2).

Each of the organic groups listed in 1) to 5) may have one or more substituents. In the case where the organic group has a plurality of substituents, the plurality of substituents may be the same or different.

Examples of the substituent(s) include: a halogen atom such as fluorine atom and chlorine atom; a cyano group; an alkyl group with a carbon number of 1 to 6 such as methyl group, ethyl group, and propyl group; an alkenyl group with a carbon number of 2 to 6 such as vinyl group and allyl group; an alkyl halide group with a carbon number of 1 to 6 such as trifluoromethyl group; an N,N-dialkylamino group with a carbon number of 2 to 12 such as dimethylamino group; an alkoxy group with a carbon number of 1 to 6 such as methoxy group, ethoxy group, and isopropoxy group; a nitro group; an aromatic hydrocarbon ring group with a carbon number of 6 to 20 such as phenyl group and naphthyl group; $-OCF_3$; $-C(=O)-R^b$; $-O-C(=O)-R^b$; $-C(=O)-O-R^b$; and $-SO_2R^a$. Here, $R^b$ and $R^a$ are as defined above.

Of these, the substituent of each of the organic groups listed in 1) to 5) is preferably at least one substituent selected from a halogen atom, a cyano group, an alkyl group with a carbon number of 1 to 6, and an alkoxy group with a carbon number of 1 to 6.

Preferable specific examples of the organic group having at least one aromatic ring selected from the group consisting of an aromatic hydrocarbon ring with a carbon number of 6 to 30 and an aromatic heterocyclic ring with a carbon number of 2 to 30 for Ax are given below. The present disclosure is, however, not limited to such. In the following formulas, "-" represents atomic bonding with an N atom (i.e. N atom that bonds with Ax) extending from any position in the ring.

1) Specific examples of the hydrocarbon ring group with a carbon number of 6 to 40 having at least one aromatic hydrocarbon ring with a carbon number of 6 to 30 include the structures represented by the following Formulas (1-1) to (1-21). An aromatic hydrocarbon ring group with a carbon number of 6 to 30 represented by, for example, any of Formulas (1-9) to (1-21) is preferable.

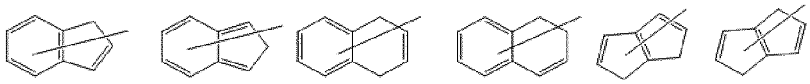

(1-1) (1-2) (1-3) (1-4) (1-5) (1-6)

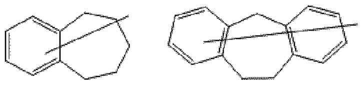

(1-7) (1-8)

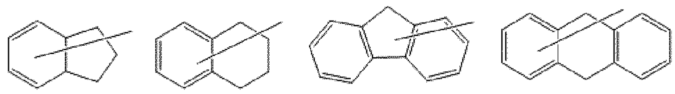

(1-9) (1-10) (1-11) (1-12)

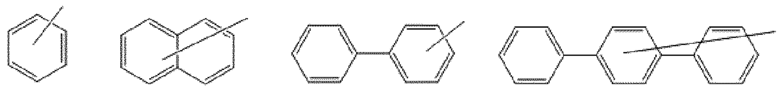

(1-13) (1-14) (1-15) (1-16)

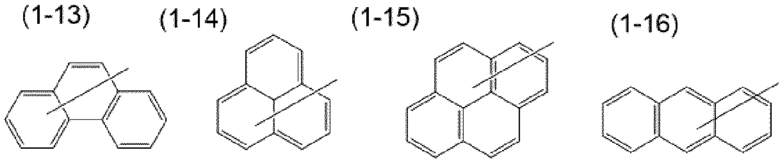

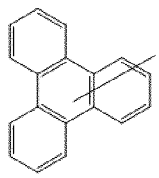

(1-17) (1-18) (1-19) (1-20) (1-21)

2) Specific examples of the heterocyclic group with a carbon number of 2 to 40 having at least one aromatic ring selected from the group consisting of an aromatic hydrocarbon ring with a carbon number of 6 to 30 and an aromatic heterocyclic ring with a carbon number of 2 to 30 include the structures represented by the following Formulas (2-1) to (2-51). An aromatic heterocyclic group with a carbon number of 2 to 30 represented by, for example, any of Formulas (2-12) to (2-51) is preferable.

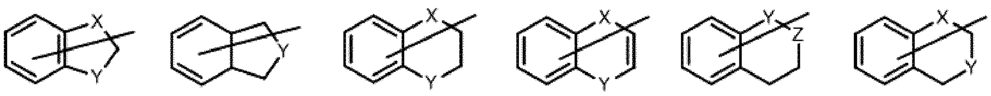

(2-1) (2-2) (2-3) (2-4) (2-5) (2-6)

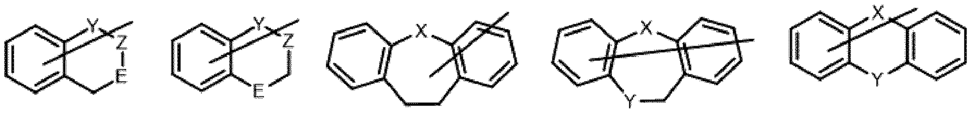

(2-7) (2-8) (2-9) (2-10) (2-11)

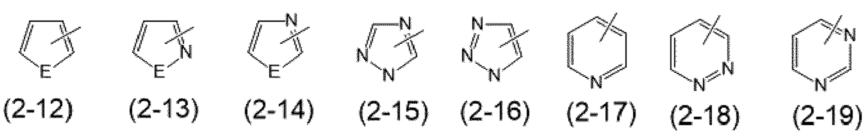

(2-12) (2-13) (2-14) (2-15) (2-16) (2-17) (2-18) (2-19)

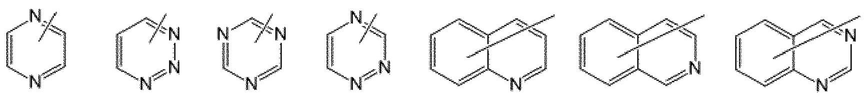

(2-20) (2-21) (2-22) (2-23) (2-24) (2-25) (2-26)

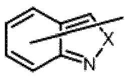 (2-27) 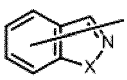 (2-28) 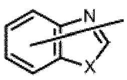 (2-29) 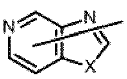 (2-30) 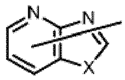 (2-31) 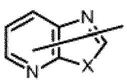 (2-32) 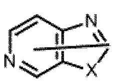 (2-33)

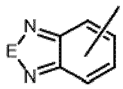 (2-34) 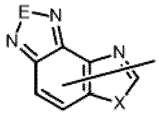 (2-35) 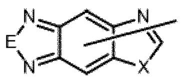 (2-36) 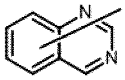 (2-37) 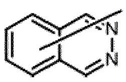 (2-38) 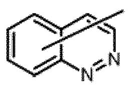 (2-39)

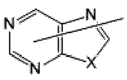 (2-40) 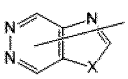 (2-41) 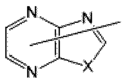 (2-42) 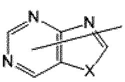 (2-43) 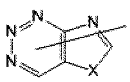 (2-44) 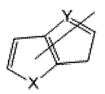 (2-45) 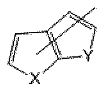 (2-46)

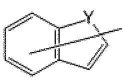 (2-47) 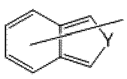 (2-48) 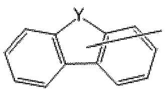 (2-49) 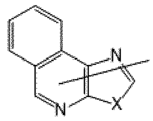 (2-50) 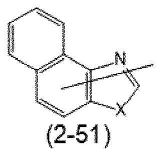 (2-51)

[where X represents $-CH_2-$, $-NR^c-$, an oxygen atom, a sulfur atom, -SO-, or $-SO_2-$, Y and Z each independently represent $-NR^c-$, an oxygen atom, a sulfur atom, -SO-, or $-SO_2-$, and E represents $-NR^c-$, an oxygen atom, or a sulfur atom. $R^c$ represents a hydrogen atom, or an alkyl group with a carbon number of 1 to 6 such as methyl group, ethyl group, and propyl group (in each formula, oxygen atom, sulfur atom, -SO-, and $-SO_2-$ are not adjacent to each other).]

3) Specific examples of the alkyl group with a carbon number of 1 to 12 substituted by at least one of an aromatic hydrocarbon ring group with a carbon number of 6 to 30 and an aromatic heterocyclic group with a carbon number of 2 to 30 include the structures represented by the following Formulas (3-1) to (3-8).

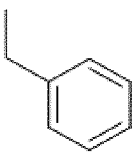 (3-1) 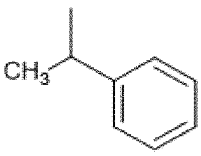 (3-2) 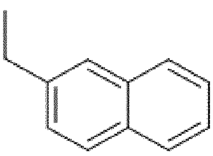 (3-3) 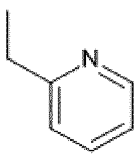 (3-4) 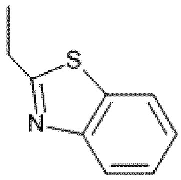 (3-5)

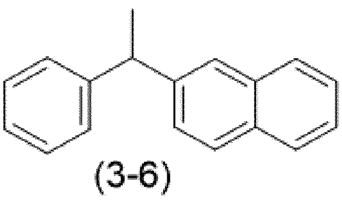 (3-6) 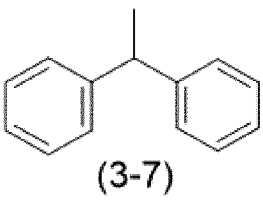 (3-7) 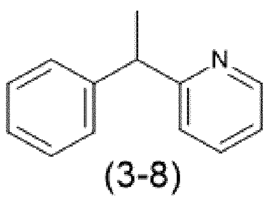 (3-8)

4) Specific examples of the alkenyl group with a carbon number of 2 to 12 substituted by at least one of an aromatic hydrocarbon ring group with a carbon number of 6 to 30 and an aromatic heterocyclic group with a carbon number of 2 to 30 include the structures represented by the following Formulas (4-1) to (4-5).

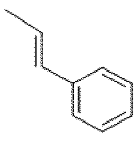 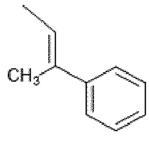 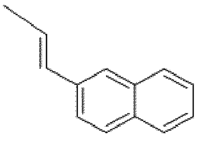 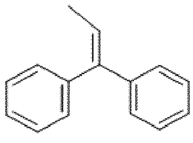 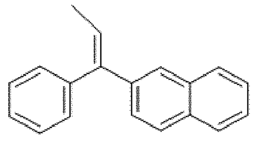

(4-1) (4-2) (4-3) (4-4) (4-5)

5) Specific examples of the alkynyl group with a carbon number of 2 to 12 substituted by at least one selected from the group consisting of an aromatic hydrocarbon ring and an aromatic heterocyclic ring include the structures represented by the following Formulas (5-1) to (5-2).

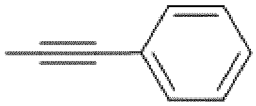 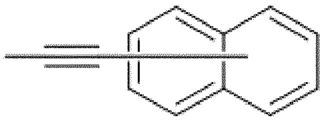

(5-1) (5-2)

The ring of each preferable specific example of Ax described above may have one or more substituents. In the case where the ring has a plurality of substituents, the plurality of substituents may be the same or different. Examples of the substituent(s) include: a halogen atom such as fluorine atom and chlorine atom; a cyano group; an alkyl group with a carbon number of 1 to 6 such as methyl group, ethyl group, and propyl group; an alkenyl group with a carbon number of 2 to 6 such as vinyl group and allyl group; an alkyl halide group with a carbon number of 1 to 6 such as trifluoromethyl group; an N,N-dialkylamino group with a carbon number of 1 to 12 such as dimethylamino group; an alkoxy group with a carbon number of 1 to 6 such as methoxy group, ethoxy group, and isopropoxy group; a nitro group; an aromatic hydrocarbon ring group with a carbon number of 6 to 20 such as phenyl group and naphthyl group; $-OCF_3$; $-C(=O)-R^b$; $-O-C(=O)-R^b$; $-C(=O)-O-R^b$; and $-SO_2R^a$.

Here, $R^b$ and $R^a$ are as defined above. Of these, the substituent of the foregoing ring of Ax is preferably a halogen atom, a cyano group, an alkyl group with a carbon number of 1 to 6, or an alkoxy group with a carbon number of 1 to 6.

Of these, Ax is preferably an aromatic hydrocarbon ring group with a carbon number of 6 to 30, an aromatic heterocyclic group with a carbon number of 2 to 30, or a group represented by the foregoing Formula (1-9).

Ax is more preferably an aromatic hydrocarbon ring group with a carbon number of 6 to 20 or an aromatic heterocyclic group with a carbon number of 4 to 20, and further preferably any of the groups represented by the foregoing Formulas (1-14), (1-20), (2-27) to (2-33), (2-35) to (2-43), and (2-51).

Each of the foregoing rings may have one or more substituents, as mentioned above. In the case where the ring has a plurality of substituents, the plurality of substituents may be the same or different. Examples of the substituent(s) include: a halogen atom such as fluorine atom and chlorine atom; a cyano group; an alkyl group with a carbon number of 1 to 6 such as methyl group, ethyl group, and propyl group; an alkenyl group with a carbon number of 2 to 6 such as vinyl group and allyl group; an alkyl halide group with a carbon number of 1 to 6 such as trifluoromethyl group and pentafluoroethyl group; an N,N-dialkylamino group with a carbon number of 1 to 12 such as dimethylamino group; an alkoxy group with a carbon number of 1 to 6 such as methoxy group, ethoxy group, and isopropoxy group; a nitro group; an aromatic hydrocarbon ring group with a carbon number of 6 to 20 such as phenyl group and naphthyl group; $-C(=O)-R^b$; $-O-C(=O)-R^b$; $-C(=O)-O-R^b$; and $-SO_2R^a$.

Here, $R^b$ and $R^a$ are as defined above.

Of these, the substituent of the foregoing ring is preferably a halogen atom, a cyano group, an alkyl group with a carbon number of 1 to 6, or an alkoxy group with a carbon number of 1 to 6.

As Ax, a group represented by the following Formula (iv) is further preferable.

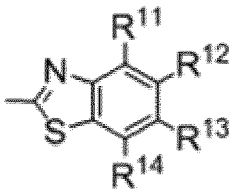

(iv)

That is, $Ar^1$ is preferably a group represented by any of the following Formulas (IV-1) to (IV-3). In Formulas (IV-1) to (IV-3), Ay, Q, $R^0$, n1, n2, n3, and n4 are as defined in the foregoing Formulas (III-1) to (III-3).

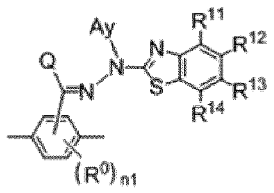
(IV-1)

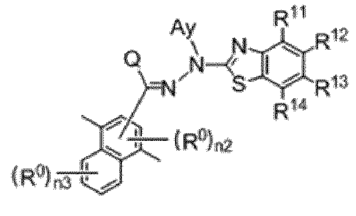
(IV-2)

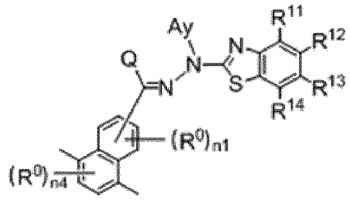
(IV-3)

In Formulas (iv) and (IV-1) to (IV-3), $R^{11}$ to $R^{14}$ are each independently a hydrogen atom, a halogen atom, an alkyl group with a carbon number of 1 to 6, a cyano group, a nitro group, a fluoroalkyl group with a carbon number of 1 to 6, an alkoxy group with a carbon number of 1 to 6, or -C(=O)-O-$R^b$, where $R^b$ is as defined above.

It is preferable that $R^{11}$ to $R^{14}$ are all a hydrogen atom, or at least one of $R^{11}$ to $R^{14}$ is an alkoxy group with a carbon number of 1 to 6 that may have a substituent and the rest is a hydrogen atom.

C-$R^{11}$ to C-$R^{14}$ may all be the same or different, and at least one of C-$R^{11}$ to C-$R^{14}$ forming the ring may be substituted by a nitrogen atom.

Specific examples of the group obtained by substituting at least one of C-$R^{11}$ to C-$R^{14}$ of the group represented by the foregoing Formula (iv) by a nitrogen atom are given below. The group obtained by substituting at least one of C-$R^{11}$ to C-$R^{14}$ by a nitrogen atom is, however, not limited to such.

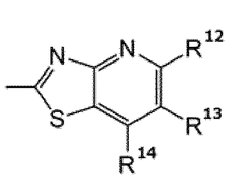
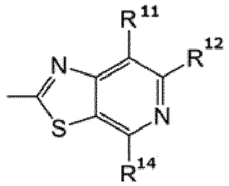
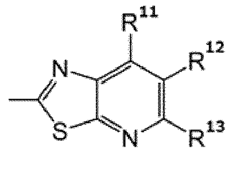
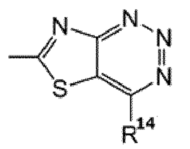

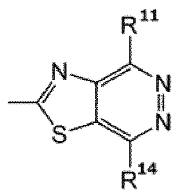
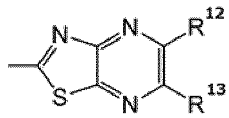
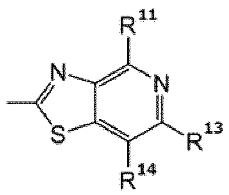
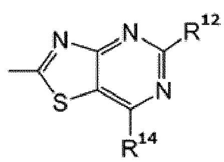

[where $R^{11}$ to $R^{14}$ are as defined above.]

Ay is a hydrogen atom or an organic group with a carbon number of 1 to 30 that may have a substituent.

Examples of the organic group with a carbon number of 1 to 30 that may have a substituent for Ay include, but are not limited to, an alkyl group with a carbon number of 1 to 20 that may have a substituent, an alkenyl group with a carbon number of 2 to 20 that may have a substituent, an alkynyl group with a carbon number of 2 to 20 that may have a substituent, a cycloalkyl group with a carbon number of 3 to 12 that may have a substituent, -$SO_2R^a$, -C(=O)-$R^b$, -CS-NH-$R^b$, an aromatic hydrocarbon ring group with a carbon number of 6 to 30 that may have a substituent, and an aromatic heterocyclic group with a carbon number of 2 to 30 that may have a substituent.

Here, $R^a$ and $R^b$ are as defined above.

Examples of the alkyl group with a carbon number of 1 to 20 of the alkyl group with a carbon number of 1 to 20 that may have a substituent, the alkenyl group with a carbon number of 2 to 20 of the alkenyl group with a carbon number of 2 to 20 that may have a substituent, and the cycloalkyl group with a carbon number of 3 to 12 of the cycloalkyl group with a carbon number of 3 to 12 that may have a substituent for Ay are the same as those listed above as specific examples of the alkyl group with a carbon number of 1 to 20 of the alkyl group with a carbon number of 1 to 20 that may have a substituent, the alkenyl group with a carbon number of 2 to 20 of the alkenyl group with a carbon number of 2 to 20 that may have a substituent, and the cycloalkyl group with a carbon number of 3 to 12 of the cycloalkyl group with a carbon number of 3 to 12 that may have a substituent for $R^b$. The carbon number of the alkyl group with a carbon number of 1 to 20 that may have a substituent is preferably 1 to 10. The carbon number of the alkenyl group with a carbon

number of 2 to 20 that may have a substituent is preferably 2 to 10. The carbon number of the cycloalkyl group with a carbon number of 3 to 12 that may have a substituent is preferably 3 to 10.

Examples of the alkynyl group with a carbon number of 2 to 20 of the alkynyl group with a carbon number of 2 to 20 that may have a substituent for Ay include ethynyl group, propynyl group, 2-propynyl group (propargyl group), butynyl group, 2-butynyl group, 3-butynyl group, pentynyl group, 2-pentynyl group, hexynyl group, 5-hexynyl group, heptynyl group, octynyl group, 2-octynyl group, nonanyl group, decanyl group, and 7-decanyl group.

Examples of the substituent of the alkyl group with a carbon number of 1 to 20 that may have a substituent, the alkenyl group with a carbon number of 2 to 20 that may have a substituent, the cycloalkyl group with a carbon number of 3 to 12 that may have a substituent, and the alkynyl group with a carbon number of 2 to 20 that may have a substituent for Ay include: a halogen atom such as fluorine atom and chlorine atom; a cyano group; an N,N-dialkylamino group with a carbon number of 2 to 12 such as dimethylamino group; an alkoxy group with a carbon number of 1 to 20 such as methoxy group, ethoxy group, isopropoxy group, and butoxy group; an alkoxy group with a carbon number of 1 to 12 substituted by an alkoxy group with a carbon number of 1 to 12, such as methoxymethoxy group and methoxyethoxy group; a nitro group; an aromatic hydrocarbon ring group with a carbon number of 6 to 20 such as phenyl group and naphthyl group; an aromatic heterocyclic group with a carbon number of 2 to 20 such as triazolyl group, pyrrolyl group, furanyl group, and thiophenyl group; a cycloalkyl group with a carbon number of 3 to 8 such as cyclopropyl group, cyclopentyl group, and cyclohexyl group; a cycloalkyloxy group with a carbon number of 3 to 8 such as cyclopentyloxy group and cyclohexyloxy group; a cyclic ether group with a carbon number of 2 to 12 such as tetrahydrofuranyl group, tetrahydropyranyl group, dioxolanyl group, and dioxanyl group; an aryloxy group with a carbon number of 6 to 14 such as phenoxy group and naphthoxy group; a fluoroalkyl group with a carbon number of 1 to 12 at least one hydrogen atom of which is substituted by a fluorine atom, such as trifluoromethyl group, pentafluoroethyl group, and $-CH_2CF_3$; a benzofuryl group; a benzopyranyl group; a benzodioxolyl group; a benzodioxanyl group; $-O-C(=O)-R^b$; $-C(=O)-R^b$; $-C(=O)-O-R^b$; $-SO_2R^a$; $-SR^b$; an alkoxy group with a carbon number of 1 to 12 substituted by $-SR^b$; and a hydroxy group. Here, $R^a$ and $R^b$ are as defined above.

The alkyl group with a carbon number of 1 to 20, the alkenyl group with a carbon number of 2 to 20, the cycloalkyl group with a carbon number of 3 to 12, and the alkynyl group with a carbon number of 2 to 20 for Ay may have a plurality of substituents described above. In the case where the group has a plurality of substituents, the plurality of substituents may be the same or different.

Examples of the aromatic hydrocarbon ring group with a carbon number of 6 to 30 and the aromatic heterocyclic group with a carbon number of 2 to 30 and their substituents for Ay are the same as those listed above as the aromatic hydrocarbon ring group and the aromatic heterocyclic group and their substituents for Ax. The aromatic hydrocarbon ring group with a carbon number of 6 to 30 and the aromatic heterocyclic group with a carbon number of 2 to 30 for Ay may each have a plurality of substituents selected from the foregoing substituents. In the case where the aromatic hydrocarbon ring group and the aromatic heterocyclic group for Ay each have a plurality of substituents, the plurality of substituents may be the same or different. The carbon number of the aromatic hydrocarbon ring group for Ay is preferably 6 to 20, more preferably 6 to 18, and further preferably 6 to 12. The carbon number of the aromatic heterocyclic group for Ay is preferably 2 to 20, and more preferably 2 to 18.

Of these, Ay is preferably a hydrogen atom, an alkyl group with a carbon number of 1 to 20 that may have a substituent, an alkenyl group with a carbon number of 2 to 20 that may have a substituent, an alkynyl group with a carbon number of 2 to 20 that may have a substituent, a cycloalkyl group with a carbon number of 3 to 12 that may have a substituent, an aromatic hydrocarbon ring group with a carbon number of 6 to 18 that may have a substituent, or an aromatic heterocyclic group with a carbon number of 2 to 18 that may have a substituent. Ay is more preferably a hydrogen atom, an alkyl group with a carbon number of 1 to 18 that may have a substituent, an alkenyl group with a carbon number of 2 to 18 that may have a substituent, an alkynyl group with a carbon number of 2 to 18 that may have a substituent, a cycloalkyl group with a carbon number of 3 to 10 that may have a substituent, an aromatic hydrocarbon ring group with a carbon number of 6 to 12 that may have a substituent, or an aromatic heterocyclic group with a carbon number of 2 to 18 that may have a substituent. Of these, Ay is particularly preferably an alkyl group with a carbon number of 1 to 18 that may have a substituent, and further preferably an alkyl group with a carbon number of 2 to 12 that may have a substituent.

Moreover, Q is a hydrogen atom or an alkyl group with a carbon number of 1 to 6. Examples of the alkyl group with a carbon number of 1 to 6 for Q include methyl group, ethyl group, n-propyl group, and isopropyl.

$R^0$ in the foregoing Formulas (III-1) to (III-3) is: a halogen atom; a cyano group; an alkyl group with a carbon number of 1 to 6 such as methyl group, ethyl group, propyl group, isopropyl group, butyl group, sec-butyl group, and tertiary butyl group; an alkenyl group with a carbon number of 2 to 6; an alkyl halide group with a carbon number of 1 to 6; an N,N-dialkylamino group with a carbon number of 2 to 12; an alkoxy group with a carbon number of 1 to 6; a nitro group; $-C(=O)-R^a$; $-C(=O)-O-R^a$; or $SO_2R^a$. $R^a$ is an alkyl group with a carbon number of 1 to 6 such as methyl group and ethyl group, or an aromatic hydrocarbon ring group with a carbon number of 6 to 20 that may have an alkyl group with a carbon number of 1 to 6 or an alkoxy group with a carbon number of 1 to 6 as a substituent such as phenyl group,

4-methylphenyl group, and 4-methoxyphenyl group. In the case where $R^a$ has a plurality of substituents, the plurality of substituents may be the same or different.

$R^0$ is preferably a halogen atom, a cyano group, an alkyl group with a carbon number of 1 to 6, an alkyl halide group with a carbon number of 1 to 6, an alkoxy group with a carbon number of 1 to 6, or a nitro group.

In each of Formulas (III-1) to (III-3), in the case where there are a plurality of $R^0$, they may be the same or different.

In Formula (III-1) to (III-3), n1 is an integer of 0 to 3, n2 is 0 or 1, n3 is an integer of 0 to 4, and n4 is an integer of 0 to 2. n1 to n4 are preferably 0.

$Ar^1$ (divalent aromatic hydrocarbon ring group having at least $D^1$ as a substituent or divalent aromatic heterocyclic group having at least $D^1$ as a substituent) is also preferably a group represented by any of the following Formulas (V-1) to (V-4). In $Ar^1$ represented by any of the following Formulas (V-1) to (V-4), $D^3$ is a substituent corresponding to $D^1$. In Formulas (V-3) and (V-4), $D^4$ corresponds to a substituent other than $D^1$.

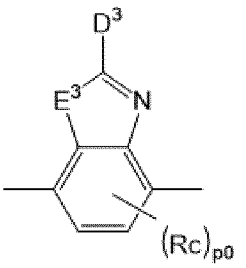
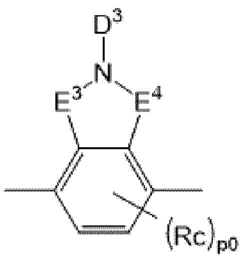
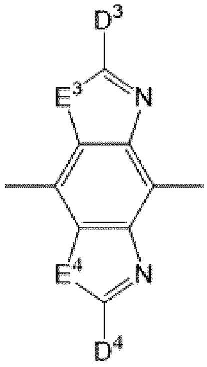
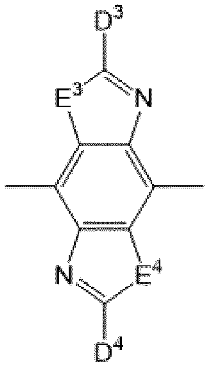

(V-1) (V-2) (V-3) (V-4)

In Formulas (V-1) to (V-4), p0 is an integer of 0 to 2, and is preferably 0 or 1.

Rc is a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12.

In each of Formulas (V-1) to (V-2), in the case where there are a plurality of Rc, the plurality of Rc may be the same or different.

Examples of the halogen atom, the alkyl group with a carbon number of 1 to 6, the alkylsulfinyl group with a carbon number of 1 to 6, the fluoroalkyl group with a carbon number of 1 to 6, the alkoxy group with a carbon number of 1 to 6, the thioalkyl group with a carbon number of 1 to 6, the N-alkylamino group with a carbon number of 1 to 6, the N,N-dialkylamino group with a carbon number of 2 to 12, the N-alkylsulfamoyl group with a carbon number of 1 to 6, and the N,N-dialkylsulfamoyl group with a carbon number of 2 to 12 for Rc are the same as those listed as specific examples of the substituent other than $D^1$.

Examples of the alkylsulfonyl group with a carbon number of 1 to 6 for Rc include methylsulfonyl group, ethylsulfonyl group, propylsulfonyl group, isopropylsulfonyl group, butylsulfonyl group, isobutylsulfonyl group, sec-butylsulfonyl group, tert-butylsulfonyl group, pentylsulfonyl group, and hexylsulfonyl group.

The halogen atom for Rc is preferably fluorine atom, chlorine atom, or bromine atom.

The alkyl group with a carbon number of 1 to 6 for Rc is preferably alkyl group with a carbon number of 1 to 4, and particularly preferably tert-butyl group or methyl group.

The alkylsulfinyl group with a carbon number of 1 to 6 for Rc is preferably alkylsulfinyl group with a carbon number of 1 to 4, more preferably alkylsulfinyl group with a carbon number of 1 to 2, and particularly preferably methylsulfinyl group.

The alkylsulfonyl group with a carbon number of 1 to 6 for Rc is preferably alkylsulfonyl group with a carbon number of 1 to 4, more preferably alkylsulfonyl group with a carbon number of 1 to 2, and particularly preferably methylsulfonyl group.

The fluoroalkyl group with a carbon number of 1 to 6 for Rc is preferably fluoroalkyl group with a carbon number of 1 to 4, more preferably fluoroalkyl group with a carbon number of 1 to 2, and particularly preferably trifluoromethyl group.

The alkoxy group with a carbon number of 1 to 6 for Rc is preferably alkoxy group with a carbon number of 1 to 4, more preferably alkoxy group with a carbon number of 1 to 2, and particularly preferably methoxy group.

The thioalkyl group with a carbon number of 1 to 6 for Rc is preferably thioalkyl group with a carbon number of 1 to 4, more preferably thioalkyl group with a carbon number of 1 to 2, and particularly preferably methylthio group.

The N-alkylamino group with a carbon number of 1 to 6 for Rc is preferably N-alkylamino group with a carbon number of 1 to 4, more preferably N-alkylamino group with a carbon number of 1 to 2, and particularly preferably N-methylamino group.

The N,N-dialkylamino group with a carbon number of 2 to 12 for Rc is preferably N,N-dialkylamino group with a carbon number of 2 to 8, N,N-dialkylamino group with a carbon number of 2 to 4, and particularly preferably N,N-dimethylamino group.

The N-alkylsulfamoyl group with a carbon number of 1 to 6 for Rc is preferably N-alkylsulfamoyl group with a carbon number of 1 to 4, more preferably N-alkylsulfamoyl group with a carbon number of 1 to 2, and particularly preferably N-methylsulfamoyl group.

The N,N-dialkylsulfamoyl group with a carbon number of 2 to 12 for Rc is preferably N,N-dialkylsulfamoyl group with a carbon number of 2 to 8, more preferably N,N-dialkylsulfamoyl group with a carbon number of 2 to 4, and particularly preferably N,N-dimethylsulfamoyl group.

Of these, Rc is preferably halogen atom, tert-butyl group, methyl group, cyano group, nitro group, carboxyl group, methylsulfonyl group, trifluoromethyl group, methoxy group, methylthio group, N-methylamino group, N,N-dimethylamino group, N-methylsulfamoyl group, N,N-dimethylsulfamoyl group, or methylsulfinyl group.

In Formulas (V-1) to (V-4), $E^3$ and $E^4$ each independently represent $-CR^{24}R^{25}-$, -S-, $-NR^{24}-$, -C(=O)-, or -O-, and $R^{24}$ and $R^{25}$ each independently represent a hydrogen atom or an alkyl group with a carbon number of 1 to 4. Examples of the alkyl group with a carbon number of 1 to 4 in $R^{24}$ and $R^{25}$ include methyl group, ethyl group, n-propyl group, isopropyl group, butyl group, isobutyl group, sec-butyl group, and tert-butyl group. Alkyl group with a carbon number of 1 to 2 is preferable, and methyl group is more preferable.

$E^3$ and $E^4$ are preferably each independently -S-, -C(=O)-, -NH-, or $-N(CH_3)-$.

In Formulas (V-1) to (V-4), $D^3$ and $D^4$ each independently represent an aromatic hydrocarbon ring group that may have a substituent or an aromatic heterocyclic group that may have a substituent.

Specifically, examples of the aromatic hydrocarbon ring group for $D^3$ and $D^4$ include phenyl group, naphthyl group, anthracenyl group, phenanthrenyl group, pyrenyl group, and fluorenyl group.

Of these, the aromatic hydrocarbon ring group is preferably phenyl group or naphthyl group.

Examples of the aromatic heterocyclic group for $D^3$ and $D^4$ include phthalimide group, 1-benzofuranyl group, 2-benzofuranyl group, acrydinyl group, isoquinolinyl group, imidazolyl group, indolinyl group, furazanyl group, oxazolyl group, oxazolopyrazinyl group, oxazolopyridinyl group, oxazolopyridazinyl group, oxazolopyrimidinyl group, quinazolinyl group, quinoxalinyl group, quinolyl group, cinnolinyl group, thiadiazolyl group, thiazolyl group, thiazolopyrazinyl group, thiazolopyridyl group, thiazolopyridazinyl group, thiazolopyrimidinyl group, 2-thienyl group, 3-thienyl group, triazinyl group, triazolyl group, naphthyridinyl group, pyrazinyl group, pyrazolyl group, pyranonyl group, pyranyl group, pyridyl group, pyridazinyl group, pyrimidinyl group, pyrrolyl group, phenanthridinyl group, phthalazinyl group, furanyl group, benzo[c]thienyl group, benzoisoxazolyl group, benzoisothiazolyl group, benzoimidazolyl group, benzoxazolyl group, benzothiadiazolyl group, benzothiazolyl group, benzothienyl group, benzotriadinyl group, benzotriazolyl group, benzopyrazolyl group, benzopyranonyl group, dihydropyranyl group, tetrahydropyranyl group, dihydrofuranyl group, and tetrahydrofuranyl group.

Of these, the aromatic heterocyclic group is preferably furanyl group, 2-thienyl group, 3-thienyl group, oxazolyl group, thiazolyl group, benzothiazolyl group, benzoxazolyl group, 1-benzofuranyl group, 2-benzofuranyl group, benzothienyl group, or thiazolopyridiyl group.

The aromatic hydrocarbon ring group and the aromatic heterocyclic group for $D^3$ and $D^4$ may be substituted by a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12.

The aromatic hydrocarbon ring group and the aromatic heterocyclic group may have one or more substituents selected from the foregoing substituents. In the case where the group has a plurality of substituents, the plurality of substituents may be the same or different.

Specific examples and preferred examples of the halogen atom, the alkyl group with a carbon number of 1 to 6, the alkylsulfinyl group with a carbon number of 1 to 6, the alkylsulfonyl group with a carbon number of 1 to 6, the fluoroalkyl group with a carbon number of 1 to 6, the alkoxy group with a carbon number of 1 to 6, the thioalkyl group with a carbon number of 1 to 6, the N-alkylamino group with a carbon number of 1 to 6, the N,N-dialkylamino group with a carbon number of 2 to 12, the N-alkylsulfamoyl group with a carbon number of 1 to 6, and the N,N-dialkylsulfamoyl group with a carbon number of 2 to 12 of the substituent of $D^3$ and $D^4$ are the same as those listed as specific examples

and preferred examples of the halogen atom, the alkyl group with a carbon number of 1 to 6, the alkylsulfinyl group with a carbon number of 1 to 6, the alkylsulfonyl group with a carbon number of 1 to 6, the fluoroalkyl group with a carbon number of 1 to 6, the alkoxy group with a carbon number of 1 to 6, the thioalkyl group with a carbon number of 1 to 6, the N-alkylamino group with a carbon number of 1 to 6, the N,N-dialkylamino group with a carbon number of 2 to 12, the N-alkylsulfamoyl group with a carbon number of 1 to 6, and the N,N-dialkylsulfamoyl group with a carbon number of 2 to 12 for Rc.

$D^3$ and $D^4$ are preferably each independently a group represented by any of the following Formulas (v-1) to (v-8).

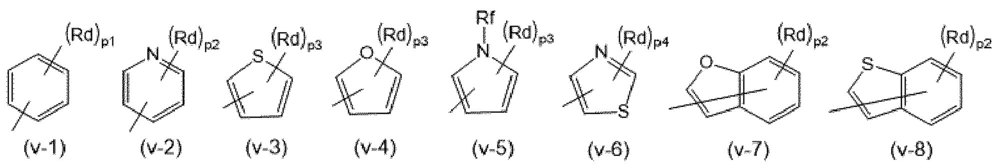

(v-1) (v-2) (v-3) (v-4) (v-5) (v-6) (v-7) (v-8)

In Formulas (v-1) to (v-8), Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12. p1 represents an integer of 0 to 5, p2 represents an integer of 0 to 4, p3 represents an integer of 0 to 3, and p4 represents an integer of 0 to 2. p1, p3, and p4 are preferably 0 or 1, and p2 is preferably an integer of 0 to 3. Rf represents a hydrogen atom or a methyl group.

In each of Formulas (v-1) to (v-8), in the case where there are a plurality of Rd, the plurality of Rd may be the same or different.

Specific examples and preferred examples of the halogen atom, the alkyl group with a carbon number of 1 to 6, the alkylsulfinyl group with a carbon number of 1 to 6, the alkylsulfonyl group with a carbon number of 1 to 6, the fluoroalkyl group with a carbon number of 1 to 6, the alkoxy group with a carbon number of 1 to 6, the thioalkyl group with a carbon number of 1 to 6, the N-alkylamino group with a carbon number of 1 to 6, the N,N-dialkylamino group with a carbon number of 2 to 12, the N-alkylsulfamoyl group with a carbon number of 1 to 6, and the N,N-dialkylsulfamoyl group with a carbon number of 2 to 12 for Rd are the same as those listed as specific examples and preferred examples of the halogen atom, the alkyl group with a carbon number of 1 to 6, the alkylsulfinyl group with a carbon number of 1 to 6, the alkylsulfonyl group with a carbon number of 1 to 6, the fluoroalkyl group with a carbon number of 1 to 6, the alkoxy group with a carbon number of 1 to 6, the thioalkyl group with a carbon number of 1 to 6, the N-alkylamino group with a carbon number of 1 to 6, the N,N-dialkylamino group with a carbon number of 2 to 12, the N-alkylsulfamoyl group with a carbon number of 1 to 6, and the N,N-dialkylsulfamoyl group with a carbon number of 2 to 12 for Rc.

Rd is preferably a halogen atom, a methyl group, a cyano group, a nitro group, a carboxyl group, a trifluoromethyl group, a methoxy group, a methylthio group, an N,N-dimethylamino group, or an N-methylamino group.

$D^3$ and $D^4$ are particularly preferably each independently a group represented by Formula (v-1), (v-3), or (v-7), in terms of the optical properties of the polymerizable compound (I) and the costs.

$Ar^1$ formed by a group represented by any of the foregoing Formulas (V-1) to (V-4) is more preferably a group represented by any of the following Formulas (VI-1) to (VI-5).

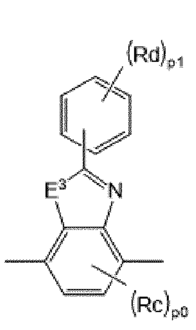 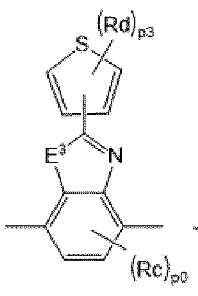 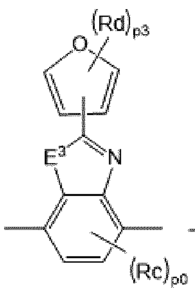 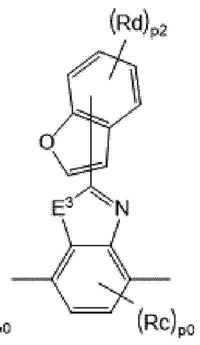 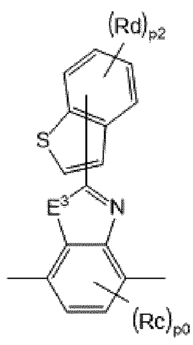

(VI-1) (VI-2) (VI-3) (VI-4) (VI-5)

In Formulas (VI-1) to (VI-5), $E^3$, Rc, Rd, and p0 to p3 are as defined above, and their preferred examples are

the same as above.

The following Formulas (ar-1) to (ar-94) represent specific examples of $Ar^2$ formed by a group represented by any of the foregoing Formulas (V-1) to (V-4).

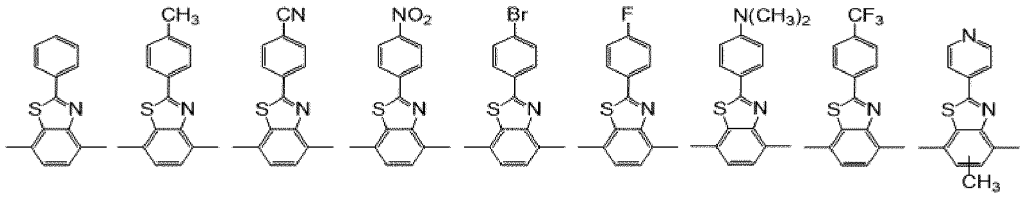

(ar-1) (ar-2) (ar-3) (ar-4) (ar-5) (ar-6) (ar-7) (ar-8) (ar-9)

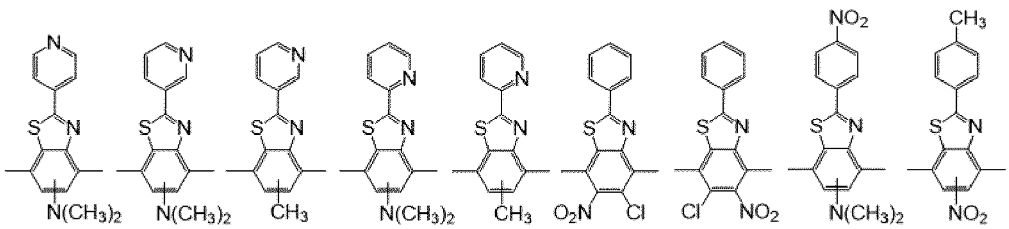

(ar-10) (ar-11) (ar-12) (ar-13) (ar-14) (ar-15) (ar-16) (ar-17) (ar-18)

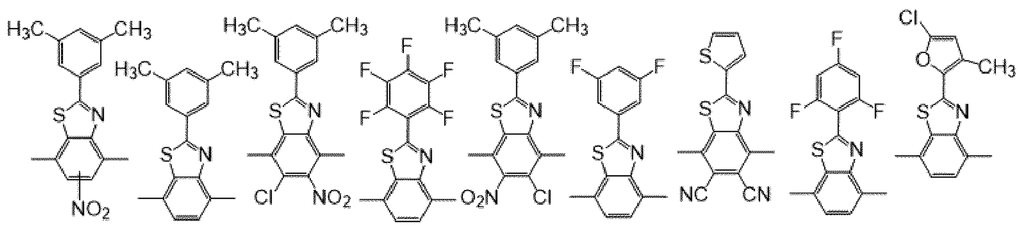

(ar-19) (ar-20) (ar-21) (ar-22) (ar-23) (ar-24) (ar-25) (ar-26) (ar-27)

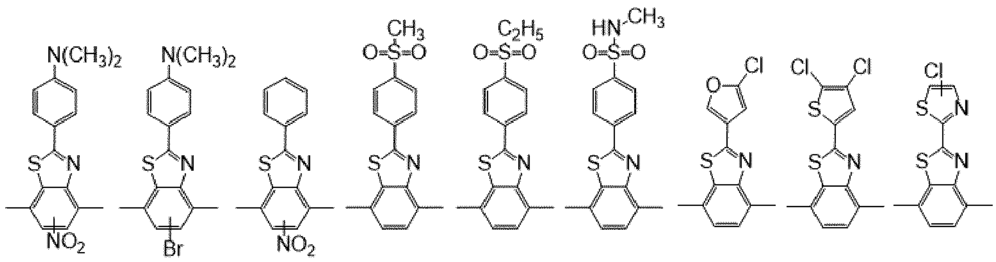

(ar-28) (ar-29) (ar-30) (ar-31) (ar-32) (ar-33) (ar-34) (ar-35) (ar-36)

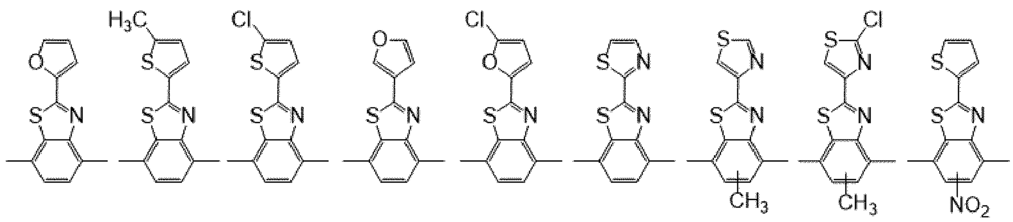

(ar-37) (ar-38) (ar-39) (ar-40) (ar-41) (ar-42) (ar-43) (ar-44) (ar-45)

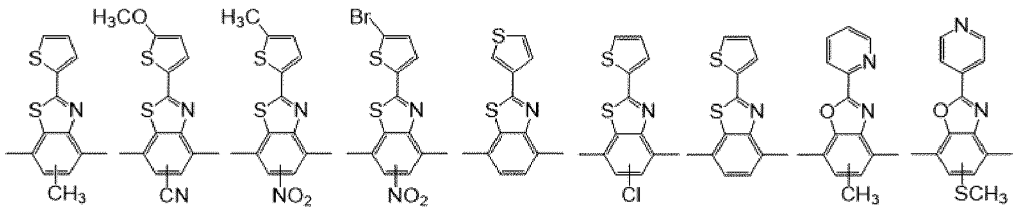

(ar-46) (ar-47) (ar-48) (ar-49) (ar-50) (ar-51) (ar-52) (ar-53) (ar-54)

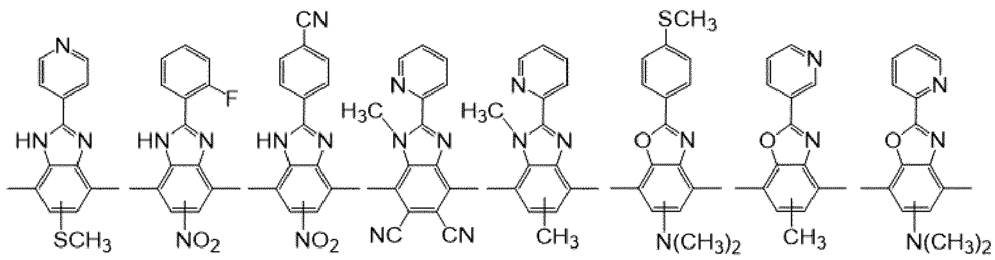

(ar-55) (ar-56) (ar-57) (ar-58) (ar-59) (ar-60) (ar-61) (ar-62)

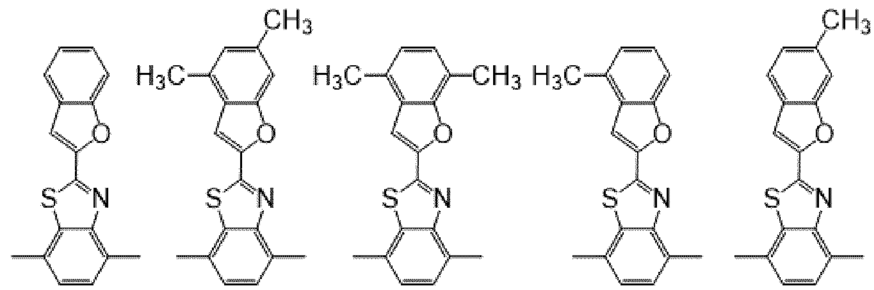

(ar-63) (ar-64) (ar-65) (ar-66) (ar-67)

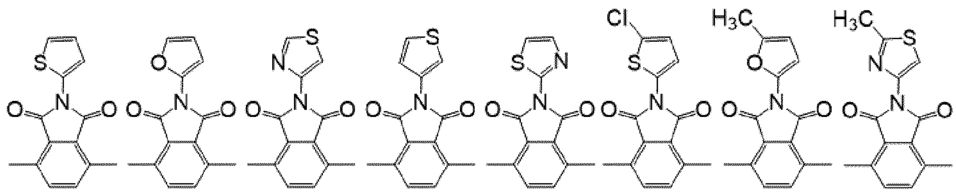

(ar-68) (ar-69) (ar-70) (ar-71) (ar-72) (ar-73) (ar-74) (ar-75)

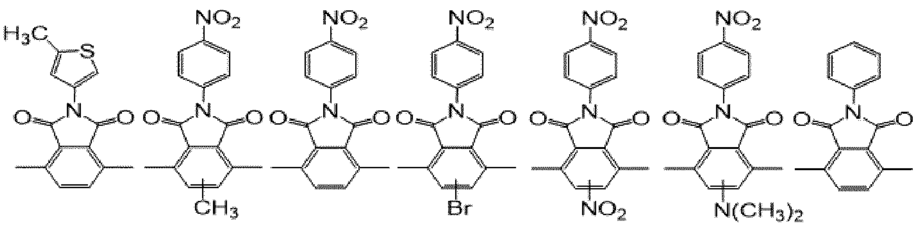

(ar-76) (ar-77) (ar-78) (ar-79) (ar-80) (ar-81) (ar-82)

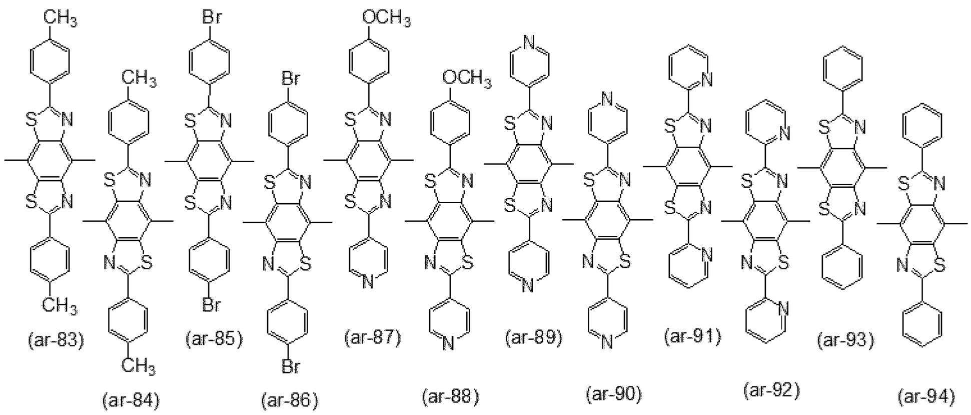

(ar-83) (ar-84) (ar-85) (ar-86) (ar-87) (ar-88) (ar-89) (ar-90) (ar-91) (ar-92) (ar-93) (ar-94)

In the foregoing Formula (I), $Z^1$ and $Z^2$ are each independently a single bond, -O-, -O-$CH_2$-, -$CH_2$-O-, -C(=O)-O-, -O-C(=O)-, -C(=O)-S-, -S-C(=O)-, -$NR^{21}$-C(=O)-, -C(=O)-$NR^{21}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C=C-. $R^{21}$ is a hydrogen atom or an alkyl group with a carbon number of 1 to 6. Examples of the alkyl group with a carbon number of 1 to 6 for $R^{21}$ include methyl group, ethyl group, propyl group, and isopropyl group.

Of these, $Z^1$ is preferably -C(=O)-O-, and $Z^2$ is preferably -O-C(=O)-.

$A^1$ and $A^2$ are each independently a cyclic aliphatic group that may have a substituent or an aromatic group that may have a substituent. Of these, $A^1$ and $A^2$ are each preferably a cyclic aliphatic group that may have a substituent.

The cyclic aliphatic group that may have a substituent is an unsubstituted divalent cyclic aliphatic group or a divalent cyclic aliphatic group having a substituent. The divalent cyclic aliphatic group is a divalent aliphatic group having a cyclic structure and typically with a carbon number of 5 to 20.

Specific examples of the divalent cyclic aliphatic group for $A^1$ and $A^2$ include: a cycloalkanediyl group with a carbon number of 5 to 20 such as cyclopentane-1,3-diyl, cyclohexane-1,4-diyl, cycloheptane-1,4-diyl, and cyclooctane-1,5-diyl; and a bicycloalkanediyl group with a carbon number of 5 to 20 such as decahydronaphthalene-1,5-diyl and decahydronaphthalene-2,6-diyl.

The aromatic group that may have a substituent is an unsubstituted divalent aromatic group or a divalent aromatic group having a substituent. The divalent aromatic group is a divalent aromatic group having an aromatic ring structure and typically with a carbon number of 2 to 20.

Specific examples of the divalent aromatic group for $A^1$ and $A^2$ include: a divalent aromatic hydrocarbon ring group with a carbon number of 6 to 20 such as 1,4-phenylene group, 1,4-naphthylene group, 1,5-naphthylene group, 2,6-naphthylene group, and 4,4'-biphenylene group; and a divalent aromatic heterocyclic group with a carbon number of 2 to 20 such as furan-2,5-diyl, thiophene-2,5-diyl, pyridine-2,5-diyl, and pyrazine-2,5-diyl.

Examples of the substituent of the divalent cyclic aliphatic group and the divalent aromatic group for $A^1$ and $A^2$ include: a halogen atom such as fluorine atom, chlorine atom, and bromine atom; an alkyl group with a carbon number of 1 to 6 such as methyl group and ethyl group; an alkoxy group with a carbon number of 1 to 5 such as methoxy group and isopropoxy group; a nitro group; and a cyano group. The cyclic aliphatic group and the aromatic group may each have at least one substituent selected from the foregoing substituents. In the case where the group has a plurality of substituents, the substituents may be the same or different.

In the case where a and/or b is 1, $L^1$ and $L^2$ are each independently a single bond, -O-, -C(=O)-, -C(=O)-O-, -O-C(=O)-, -$NR^{22}$-C(=O)-, -C(=O)-$NR^{22}$-, -O-C(=O)-O-, -$NR^{22}$-C(=O)-O-, -O-C(=O)-$NR^{22}$-, or -$NR^{22}$-C(=O)-$NR^{21}$-. $R^{22}$ and $R^{23}$ are each independently a hydrogen atom or an alkyl group with a carbon number of 1 to 6. Of these, $L^1$ and $L^2$ are preferably each independently -O-, -C(=O)-O-, or -O-C(=O)-.

Examples of the alkyl group with a carbon number of 1 to 6 for $R^{22}$ and $R^{23}$ include methyl group, ethyl group, propyl group, and isopropyl group.

In the case where a and/or b is 1, $B^1$ and $B^2$ are each independently a cyclic aliphatic group that may have a substituent or an aromatic group that may have a substituent. Of these, $B^1$ and $B^2$ are preferably an aromatic group that may have a substituent.

The cyclic aliphatic group that may have a substituent is an unsubstituted divalent cyclic aliphatic group or a divalent cyclic aliphatic group having a substituent. The divalent cyclic aliphatic group is a divalent aliphatic group having a cyclic structure and typically with a carbon number of 5 to 20.

Specific examples of the divalent cyclic aliphatic group for $B^1$ and $B^2$ are the same as those listed above as the divalent cyclic aliphatic group for $A^1$.

The aromatic group that may have a substituent is an unsubstituted divalent aromatic group or a divalent aromatic group having a substituent. The divalent aromatic group is a divalent aromatic group having an aromatic ring structure and typically with a carbon number of 2 to 20.

Specific examples of the divalent aromatic group for $B^1$ and $B^2$ are the same as those listed above as the divalent aromatic group for $A^1$.

Specific examples of the substituent of the divalent cyclic aliphatic group and the divalent aromatic group for $B^1$ and $B^2$ are the same as those listed above as the substituent of the divalent cyclic aliphatic group and the divalent aromatic group for $A^1$.

In the foregoing Formula (I), the structure of the part represented by "-$L^{1a}$-$G^1$-$P^{1a}$" and the structure of the part represented by "-$L^{2a}$-$G^2$-$P^{2a}$" are different from each other. That is, $L^{1a}$ and $L^{2a}$, $G^1$ and $G^2$, and $P^{1a}$ and $P^{2a}$ satisfy any of the following relationships:

(1) $L^{1a}$ and $L^{2a}$ have different structures, $G^1$ and $G^2$ have different structures, and $P^{1a}$ and $P^{2a}$ have different structures.
(2) $L^{1a}$ and $L^{2a}$ have the same structure, $G^1$ and $G^2$ have different structures, and $P^{1a}$ and $P^{2a}$ have different structures.
(3) $L^{1a}$ and $L^{2a}$ have different structures, $G^1$ and $G^2$ have the same structure, and $P^{1a}$ and $P^{2a}$ have different structures.
(4) $L^{1a}$ and $L^{2a}$ have different structures, $G^1$ and $G^2$ have different structures, and $P^{1a}$ and $P^{2a}$ have the same structure.
(5) $L^{1a}$ and $L^{2a}$ have the same structure, $G^1$ and $G^2$ have the same structure, and $P^{1a}$ and $P^{2a}$ have different structures.
(6) $L^{1a}$ and $L^{2a}$ have the same structure, $G^1$ and $G^2$ have different structures, and $P^{1a}$ and $P^{2a}$ have the same structure.
(7) $L^{1a}$ and $L^{2a}$ have different structures, $G^1$ and $G^2$ have the same structure, and $P^{1a}$ and $P^{2a}$ have the same structure.

In the present disclosure, "two groups have the same structure" denotes that the types and sequence of the atomic groups constituting each group are the same in the direction from the center ($Ar^1$ side) to the end ($P^{1a}$, $P^{2a}$ side) of the polymerizable compound. For example, "$P^{1a}$-$G^1$-O-C(=O)-" and "-C(=O)-O-$G^2$-$P^{2a}$" correspond to the case where $L^{1a}$ and $L^{2a}$ have the same structure. In the present disclosure, "two groups have different structures" denotes that the types and/or sequence of the atomic groups constituting each group are different in the direction from the center ($Ar^1$ side) to the end ($P^{1a}$, $P^{2a}$ side) of the polymerizable compound. For example, "$P^{1a}$-$G^1$-O-C(=O)-" and "-O-C(=O)-$G^2$-$P^{2a}$" correspond to the case where $L^{1a}$ and $L^{2a}$ have different structures.

Of these, $L^{1a}$ and $L^{2a}$, $G^1$ and $G^2$, and $P^{1a}$ and $P^{2a}$ preferably satisfy the relationship (1), (2), (4), or (6), and further preferably satisfy the relationship (4) or (6).

$L^{1a}$ and $L^{2a}$ are each independently a single bond, -O-, -C(=O)-, -C(=O)-O-, -O-C(=O)-, -$NR^{22}$-C(=O)-, -C(=O)-$NR^{22}$-, -O-C(=O)-O-, -$NR^{22}$-C(=O)-O-, -O-C(=O)-$NR^{22}$-, or -$NR^{22}$-C(=O)-$NR^{23}$-. $R^{22}$ and $R^{23}$ are each independently a hydrogen atom or an alkyl group with a carbon number of 1 to 6. Of these, $L^{1a}$ and $L^{2a}$ are preferably each independently -O-, -C(=O)-O-, or -O-C(=O)-.

Examples of the alkyl group with a carbon number of 1 to 6 for $R^{22}$ and $R^{23}$ include methyl group, ethyl group, propyl group, and isopropyl group.

$G^1$ and $G^2$ are each independently an aliphatic hydrocarbon group with a carbon number of 3 to 20 that may have a substituent. The aliphatic hydrocarbon group with a carbon number of 3 to 20 for $G^1$ and $G^2$ may be interrupted by at least one intervening group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, -N=, =N-, and -N=N-. In the case where there are two or more intervening groups in the aliphatic hydrocarbon group with a carbon number of 3 to 20, they may be the same or different, and are not adjacent to each other.

R each independently represent a hydrogen atom or an alkyl group with a carbon number of 1 to 6. Examples of the alkyl group with a carbon number of 1 to 6 include methyl group, ethyl group, propyl group, and isopropyl group.

Examples of the aliphatic hydrocarbon group with a carbon number of 3 to 20 for $G^1$ and $G^2$ include, but are not limited to, a chain aliphatic hydrocarbon group such as alkylene group with a carbon number of 3 to 20, alkenylene group with a carbon number of 3 to 20, and alkynylene group with a carbon number of 3 to 20. The carbon number of the aliphatic hydrocarbon group is preferably 3 to 12, and more preferably 4 to 10.

It is preferable that two or more carbon atoms are present between the intervening group in $G^1$ and/or $G^2$ and $P^{1a}$ and/or $P^{2a}$.

In the case where $L^{1a}$ and $L^{2a}$, $G^1$ and $G^2$, and $P^{1a}$ and $P^{2a}$ satisfy the relationship (6) and $G^1$ and/or $G^2$ has only one -C(=O)-O- or -O-C(=O)- as an intervening group, it is more preferable that three or more carbon atoms are present between the intervening group in $G^1$ and/or $G^2$ and $P^{1a}$ and/or $P^{2a}$.

The intervening group is preferably -O-, -C(=O)-, -C(=O)-O-, -O-C(=O)-, -S-, -C(=O)-S-, -S-C(=O)-, -NR-, -NR-C(=O)-, or -C(=O)-NR-.

In the case where $G^1$ and $G^2$ have different structures, one of $G^1$ and $G^2$ is preferably an organic group composed of a plurality of methylene groups ($-CH_2-$) that may be substituted and at least one group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-,

$-O-(CH_2)_n-O-$, $-CH=CH-C(=O)-O-$, $-O-C(=O)-CH=CH-$, $-CH=CH-$, $-N=CH-$, $-CH=N-$, $-N=C(CH_3)-$, $-C(CH_3)=N-$, $-N=N-$, and $-C≡C-$ located between methylene groups that may be substituted. The other one of $G^1$ and $G^2$ is preferably an alkylene group with a carbon number of 3 to 20 that may have a substituent, or an organic group composed of a plurality of methylene groups that may be substituted and at least one group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, $-O-(CH_2)_n-O-$, $-CH=CH-C(=O)-O-$, $-O-C(=O)-CH=CH-$, $-CH=CH-$, $-N=CH-$, $-CH=N-$, $-N=C(CH_3)-$, $-C(CH_3)=N-$, $-N=N-$, and $-C≡C-$ located between methylene groups that may be substituted. Of these, $G^1$ and $G^2$ are preferably a combination of an organic group and an unsubstituted alkylene group with a carbon number of 3 to 20. The organic group is preferably a group in which at least one selected from the group consisting of -O-, -C(=O)-O-, -O-C(=O)-, -S-, -C(=O)-, -C(=O)-S-, -S-C(=O)-, -NR-, -NR-C(=O)-, and -C(=O)-NR- is located between methylene groups that may be substituted.

R is as defined above. n is an integer of 1 to 18.

In the case where $G^1$ and $G^2$ have different structures, $G^1$ and $G^2$ are each preferably an alkylene group with a carbon number of 3 to 20 that may have a substituent and that may be interrupted by the foregoing intervening group, and more preferably an alkylene group with a carbon number of 3 to 20 that may have a substituent. Further preferably, at least one of $G^1$ and $G^2$ is an alkylene group with a carbon number of 3 to 20 having a substituent. Particularly preferably, one of $G^1$ and $G^2$ is an alkylene group with a carbon number of 3 to 20 having a substituent, and the other one of $G^1$ and $G^2$ is an unsubstituted alkylene group with a carbon number of 3 to 20.

The alkylene group with a carbon number of 3 to 20 having a substituent is preferably a group at least one hydrogen atom of which is substituted by a substituent formed by a halogen atom, a cyano group, an alkyl group with a carbon number of 1 to 6, an alkenyl group with a carbon number of 2 to 6, an alkynyl group with a carbon number of 2 to 6, an alkyl halide group with a carbon number of 1 to 6, an N,N-dialkylamino group with a carbon number of 2 to 6, an alkoxy group with a carbon number of 1 to 6, a nitro group, $-C(=O)-R^{1a}$, $-C(=O)-O-R^{1a}$, or $-O-C(=O)-R^{1a}$, and more preferably a group at least one hydrogen atom of which is substituted by a substituent formed by a halogen atom, a cyano group, an alkyl group with a carbon number of 1 to 6, an alkenyl group with a carbon number of 2 to 6, an alkynyl group with a carbon number of 2 to 6, an alkyl halide group with a carbon number of 1 to 6, an N,N-dialkylamino group with a carbon number of 2 to 6, an alkoxy group with a carbon number of 1 to 6, or a nitro group. $R^{1a}$ represents an alkyl group with a carbon number of 1 to 6 such as methyl group, ethyl group, n-propyl group, isopropyl group, n-butyl group, isobutyl group, sec-butyl group, tert-butyl group, pentyl group, and hexyl group, an aromatic hydrocarbon ring group with a carbon number of 6 to 20 such as phenyl group and naphthyl group, or an aliphatic hydrocarbon ring group with a carbon number of 6 to 20 such as cyclohexyl group and norbornyl group. In the case where there are a plurality of substituents, they may be the same or different.

Examples of the halogen atom for the substituent include fluorine atom, chlorine atom, bromine atom, and iodine atom.

Examples of the alkyl group with a carbon number of 1 to 6 include methyl group, ethyl group, n-propyl group, isopropyl group, n-butyl group, isobutyl group, sec-butyl group, tert-butyl group, pentyl group, and hexyl group.

Examples of the alkenyl group with a carbon number of 2 to 6 include vinyl group, propenyl group, isopropenyl group, butenyl group, isobutenyl group, pentenyl group, and hexenyl group.

Examples of the alkynyl group with a carbon number of 2 to 6 include ethynyl group, propynyl group, butynyl group, pentynyl group, and hexynyl group.

Examples of the alkyl halide group with a carbon number of 1 to 6 include fluoroalkyl group with a carbon number of 1 to 6 such as fluoromethyl group, trifluoromethyl group, fluoroethyl group, pentafluoroethyl group, heptafluoropropyl group, and nonafluorobutyl group.

Examples of the N,N-dialkylamino group with a carbon number of 2 to 6 include N,N-dimethylamino group, N-methyl-N-ethylamino group, N,N-diethylamino group, N,N-dipropylamino group, and N,N-diisopropylamino group.

Examples of the alkoxy group with a carbon number of 1 to 6 include methoxy group, ethoxy group, propoxy group, isopropoxy group, butoxy group, isobutoxy group, sec-butoxy group, tert-butoxy group, pentyloxy group, and hexyloxy group.

In the case where $G^1$ and $G^2$ have different structures, it is preferable that the aliphatic hydrocarbon group with a carbon number of 3 to 20 that may have a substituent and that may be interrupted by the foregoing intervening group for $G^1$ and $G^2$ differs in carbon number. In the case where $G^1$ and $G^2$ have different structures, $G^1$ and $G^2$ are each more preferably an alkylene group with a different carbon number that may have a substituent and that may be interrupted by the foregoing intervening group, and further preferably an unsubstituted alkylene group with a different carbon number.

In the foregoing Formula (I), $P^{1a}$ and $P^{2a}$ each independently represent a polymerizable group.

Examples of the polymerizable group for $P^{1a}$ and $P^{2a}$ include a group represented by $CH_2=CR^1-C(=O)-O-$ such as acryloyloxy group and methacryloyloxy group ($R^1$ represents hydrogen atom, methyl group, or chlorine atom), vinyl group, vinyl ether group, p-stilbene group, acryloyl group, methacryloyl group, carboxyl group, methylcarbonyl group, hydroxy group, amide group, alkylamino group with a carbon number of 1 to 4, amino group, epoxy group, oxetanyl group, aldehyde group, isocyanate group, and thioisocyanate group. Of these, a group represented by

$CH_2=CR^1-C(=O)-O-$is preferable, and $CH_2=CH-C(=O)-O-$ (acryloyloxy group) and $CH_2=C(CH_3)-C(=O)-O-$ (methacryloyloxy group) are more preferable. In the case where $P^{1a}$ and $P^{2a}$ have the same structure, $P^{1a}$ and $P^{2a}$ are each particularly preferably an acryloyloxy group. In the case where two $R^1$ are present in the polymerizable compound represented by Formula (I), they may be the same or different.

In terms of obtaining an optical film, etc. having excellent reverse wavelength dispersibility, the polymerizable compound (I) preferably has a structure that is approximately bilaterally symmetric with respect to $Ar^1$. Specifically, it is preferable that, in the polymerizable compound (I), a and b are the same and $-[B^1-L^1]_a-A^1-Z^1-(*)$ and $(*)-Z^2-A^2-[L^2-B^2]_b-$ have a symmetric structure with respect to the side (*) on which $-[B^1-L^1]_a-A^1-Z^1-(*)$ and $(*)-Z^2-A^2-[L^2-B^2]_b-$ bond with $Ar^1$.

The expression "having a symmetric structure with respect to (*)" means, for example, to have a structure of -C(=O)-O-(*) and (*)-O-C(=O)-, a structure of -O-(*) and (*)-O-, or a structure of -O-C(=O)-(*) and (*)-C(=O)-O-.

The polymerizable compound (I) can be synthesized by combining synthesis reactions known in the art. Specifically, the polymerizable compound (I) can be synthesized with reference to methods described in various literatures (e.g. March's Advanced Organic Chemistry (Wiley), S.R. Sandler and W. Karo "Organic Functional Group Preparations", jointly translated by Naoki Inamoto (Hirokawa Shoten)) and JP 2010-031223 A.

(2) Mixture containing polymerizable compounds

The presently disclosed mixture is a mixture containing the polymerizable compound (I) and a polymerizable compound represented by the following Formula (II) (polymerizable compound (II)), and is usable, for example, in the production of the below-described polymerizable liquid crystal composition and polymer.

The mass ratio of the polymerizable compound (I) and the polymerizable compound (II) in the mixture may be freely adjusted. In terms of enabling the formation of an optical film, etc. having excellent reverse wavelength dispersibility at lower processing temperature, the proportion of the polymerizable compound (I) to the total of the polymerizable compounds (I) and (II) is preferably 20 mass% or more, and further preferably 50 mass% or more.

$$P^{10a}-G^{10}-L^{10a}\left[B^{10}-L^{10}\right]_a A^{10}-Z^{10}-Ar^{10}-Z^{20}-A^{20}\left[L^{20}-B^{20}\right]_b L^{20a}-G^{20}-P^{20a}$$

(II)

In Formula (II), a and b are each independently 0 or 1, and preferably 1.

Specific examples and preferred examples of $Ar^{10}$ in Formula (II) are the same as those listed as specific examples and preferred examples of $Ar^1$.

Specific examples and preferred examples of $Z^{10}$ and $Z^{20}$ are the same as those listed as specific examples and preferred examples of $Z^1$ and $Z^2$.

Specific examples and preferred examples of $A^{10}$ and $A^{20}$ are the same as those listed as specific examples and preferred examples of $A^1$ and $A^2$.

In the case where a and/or b is 1, specific examples and preferred examples of $L^{10}$ and $L^{20}$ are the same as those listed as specific examples and preferred examples of $L^1$ and $L^2$.

In the case where a and/or b is 1, specific examples and preferred examples of $B^{10}$ and $B^{20}$ are the same as those listed as specific examples and preferred examples of $B^1$ and $B^2$.

In Formula (II), the structure of the part represented by "$-L^{10a}-G^{10}-P^{10a}$" and the structure of the part represented by "$-L^{20a}-G^{20}-P^{20a}$" are the same. That is, $L^{1a}$ and $L^{2a}$ have the same structure, $G^1$ and $G^2$ have the same structure, and $P^{1a}$ and $P^{2a}$ have the same structure.

Specific examples and preferred examples of $L^{10a}$ and $L^{20a}$ are the same as those listed as specific examples and preferred examples of $L^{1a}$ and $L^{2a}$.

Specific examples and preferred examples of $G^{10}$ and $G^{20}$ are the same as those listed as specific examples and preferred examples of $G^1$ and $G^2$. Of these, $G^1$ and $G^2$ are preferably an alkylene group with a carbon number of 3 to 20, more preferably an alkylene group with a carbon number of 3 to 12, and further preferably an alkylene group with a carbon number of 4 to 10.

Specific examples and preferred examples of $P^{10a}$ and $P^{20a}$ are the same as those listed as specific examples and preferred examples of $P^{1a}$ and $p^{2a}$.

In terms of obtaining an optical film, etc. having excellent reverse wavelength dispersibility, the polymerizable compound (II) preferably has a structure that is bilaterally symmetric with respect to $Ar^{10}$. Specifically, it is preferable that, in the polymerizable compound (II), a and b are the same and $-[B^{10}-L^{10}]_a-A^{10}-Z^{10}-(*)$ and $(*)-Z^{20}-A^{20}-[L^{20}-B^{20}]_b-$ have a symmetric structure with respect to the side (*) on which $-[B^{10}-L^{10}]_a-A^{10}-Z^{10}-(*)$ and $(*)-Z^{20}-A^{20}-[L^{20}-B^{20}]_b-$ bond with $Ar^{10}$.

The expression "having a symmetric structure with respect to (*)" means, for example, to have a structure of

-C(=O)-O-(*) and (*)-O-C(=O)-, a structure of -O-(*) and (*)-O-, or a structure of -O-C(=O)-(*) and (*)-C(=O)-O-.

The polymerizable compound (II) can be produced by combining synthesis reactions known in the art. Specifically, the polymerizable compound (I) can be synthesized with reference to methods described in various literatures (e.g. March's Advanced Organic Chemistry (Wiley), S.R. Sandler and W. Karo "Organic Functional Group Preparations", jointly translated by Naoki Inamoto (Hirokawa Shoten)) and JP 2010-031223 A.

In terms of enhancing the reverse wavelength dispersibility of an optical film, etc., it is preferable that $Ar^1$, $Z^1$, $Z^2$, $A^1$, $A^2$, $B^1$, $B^2$, $L^1$, $L^2$, and a to b of the polymerizable compound (I) are respectively the same as $Ar^{10}$, $Z^{10}$, $Z^{20}$, $A^{10}$, $A^{20}$, $B^{10}$, $B^{20}$, $L^{10}$, $L^{20}$, and a to b of the polymerizable compound (II) in the presently disclosed mixture, without being limited thereto.

That is, the polymerizable compound (II) has the same structure as the polymerizable compound (I), except that "$-L^{10a}-G^{10}-P^{10a}$" and "$-L^{20a}-G^{20}-P^{20a}$" located at the ends have the same structure.

The mixture containing the polymerizable compounds can be, for example, prepared by mixing, at a desired proportion, at least one polymerizable compound (I) and at least one polymerizable compound (II) prepared separately, without being limited thereto.

(3) Polymerizable liquid crystal composition

A polymerizable liquid crystal composition using the presently disclosed polymerizable compound or mixture contains the foregoing polymerizable compound or mixture (mixture containing the polymerizable compounds (I) and (II)) and a polymerization initiator.

As described later, the polymerizable liquid crystal composition using the presently disclosed polymerizable compound or mixture is useful as the raw material for the manufacture of the presently disclosed polymer, optical film, and optically anisotropic product. The use of the polymerizable liquid crystal composition using the presently disclosed polymerizable compound or mixture allows for efficient production of an optical film, etc. capable of uniform polarized light conversion over a wide wavelength range.

The polymerization initiator is blended for more efficient polymerization reaction of the polymerizable compounds contained in the polymerizable liquid crystal composition.

Examples of polymerization initiators used include radical polymerization initiators, anion polymerization initiators, and cation polymerization initiators.

For radical polymerization initiators, both of thermal radical generators (compounds that on heating generate active species that may initiate polymerization of polymerizable compounds) and photo-radical generators (compounds that on exposure to exposure light such as visible ray, ultraviolet ray (e.g. i line), far-ultraviolet ray, electron ray, or X ray generate active species that may initiate polymerization of polymerizable compounds) can be used, with photo-radical generators being suitable.

Examples of the photo-radical generators include acetophenone compounds, biimidazole compounds, triazine compounds, O-acyloxime compounds, onium salt compounds, benzoin compounds, benzophenone compounds, $\alpha$-diketone compounds, polynuclear quinone compounds, xanthone compounds, diazo compounds, and imidesulfonate compounds. These compounds are components that on exposure to light generate one or both of active radicals and active acid. These photo-radical generators may be used alone or in combination.

Specific examples of the acetophenone compounds include 2-hydroxy-2-methyl-1-phenylpropane-1-one, 2-methyl-1-[4-(methylthio)phenyl]-2-morpholinopropane-1-one, 2-benzyl-2-dimethylamino-1-(4-morpholinophenyl)butane-1-one, 1-hydroxycyclohexyl phenyl ketone, 2,2-dimethoxy-1,2-diphenylethane-1-one, 1,2-octanedione, and 2-benzyl-2-dimethylamino-4'-morpholinobutyrophenone.

Specific examples of the biimidazole compounds include 2,2'-bis(2-chlorophenyl)-4,4',5,5'-tetrakis(4-ethoxycarbonylphenyl)-1,2'-bii midazole,
2,2'-bis(2-bromophenyl)-4,4',5,5'-tetrakis(4-ethoxycarbonylphenyl)-1,2'-bii midazole, 2,2'-bis(2-chlorophenyl)-4,4',5,5'-tetraphenyl-1,2'-biimidazole, 2,2'-bis(2,4-dichlorophenyl)-4,4',5,5'-tetraphenyl-1,2'-biimidazole, 2,2'-bis(2,4,6-trichlorophenyl)-4,4',5,5'-tetraphenyl-1,2'-biimidazole, 2,2'-bis(2-bromophenyl)-4,4',5,5'-tetraphenyl-1,2'-biimidazole, 2,2'-bis(2,4-dibromophenyl)-4,4',5,5'-tetraphenyl-1,2'-biimidazole, and 2,2'-bis(2,4,6-tribromophenyl)-4,4',5,5'-tetraphenyl-1,2'-biimidazole.

In the present disclosure, in the case where biimidazole compounds are used as photopolymerization initiators (photo-radical generators), it is preferable to use hydrogen donors in combination for further improvement in sensitivity.

By "hydrogen donor" is meant a compound that can donate hydrogen atom to a radical generated on exposure to light from a biimidazole compound. Preferred hydrogen donors are mercaptan compounds and amine compounds defined below.

Examples of the mercaptan compounds include 2-mercaptobenzothiazole, 2-mercaptobenzoxazole, 2-mercaptobenzimidazole, 2,5-dimercapto-1,3,4-thiadiazole, and 2-mercapto-2,5-dimethylaminopyridine. Examples of the amine compounds include 4,4'-bis(dimethylamino)benzophenone, 4,4'-bis(diethylamino)benzophenone, 4-diethylaminoacetophenone, 4-dimethylaminopropiophenone, ethyl-4-dimethylaminobenzoate, 4-dimethylamino benzoic acid, and 4-dimethylaminobenzonitrile.

Examples of the triazine compounds include triazine compounds having a halomethyl group, such as 2,4,6-tris(trichloromethyl)-s-triazine, 2-methyl-4,6-bis(trichloromethyl)-s-triazine, 2-[2-(5-methylfuran-2-yl)ethenyl]-4,6-bis(trichloromethyl)-s-triazine, 2-[2-(furan-2-yl)ethenyl]-4,6-bis(trichloromethyl)-s-triazine, 2-[2-(4-diethylamino-2-methylphenyl)ethenyl]-4,6-bis(trichloromethyl)-s-tria zine, 2-[2-(3,4-dimethoxyphenyl)ethenyl]-4,6-bis(trichloromethyl)-s-triazine, 2-(4-methoxyphenyl)-4,6-bis(trichloromethyl)-s-triazine, 2-(4-ethoxystyryl)-4,6-bis(trichloromethyl)-s-triazine, and 2-(4-n-butoxyphenyl)-4,6-bis(trichloromethyl)-s-triazine.

Specific examples of the O-acyloxime compounds include 1-[4-(phenylthio)phenyl]-heptane-1,2-dione2-(O-benzoyloxime), 1-[4-(phenylthio)phenyl]-octane-1,2-dione2-(O-benzoyloxime), 1-[4-(benzoyl)phenyl]-octane-1,2-dione2-(O-benzoyloxime), 1-[9-ethyl-6-(2-methylbenzoyl)-9H-carbozole-3-yl]-ethanone1-(O-acetyloxim e), 1-[9-ethyl-6-(3-methylbenzoyl)-9H-carbozole-3-yl]-ethanone1-(O-acetyloxim e), 1-(9-ethyl-6-benzoyl-9H-carbozole-3-yl)-ethanone1-(O-acetyloxime), ethanone-1-[9-ethyl-6-(2-methyl-4-tetrahydrofuranylbenzoyl)-9H-carbozole-3 -yl]-1-(O-acetyloxime), ethanone-1-[9-ethyl-6-(2-methyl-4-tetrahydropyranylbenzoyl)-9H-carbozole-3-yl]-1-(O-acetyloxime), ethanone-1-[9-ethyl-6-(2-methyl-5-tetrahydrofuranylbenzoyl)-9H-carbozole-3 -yl]-1-(O-acetyloxime), ethanone-1-[9-ethyl-6-(2-methyl-5-tetrahydropyranylbenzoyl)-9H-carbozole-3-yl]-1-(O-acetyloxime), ethanone-1-[9-ethyl-6-{2-methyl-4-(2,2-dimethyl-1,3-dioxolanyl)benzoyl}-9 H-carbozole-3-yl]-1-(O-acetyloxime), ethanone-1-[9-ethyl-6-(2-methyl-4-tetrahydrofuranylmethoxybenzoyl)-9H-car bozole-3-yl]-1-(O-acetyloxime), ethanone-1-[9-ethyl-6-(2-methyl-4-tetrahydropyranylmethoxybenzoyl)-9H-ca rbozole-3-yl]-1-(O-acetyloxime), ethanone-1-[9-ethyl-6-(2-methyl-5-tetrahydrofuranylmethoxybenzoyl)-9H-car bozole-3-yl]-1-(O-acetyloxime), ethanone-1-[9-ethyl-6-(2-methylbenzoyl)-9H-carbozole-3-yl]-1-(O-acetyloxi me), ethanone-1-[9-ethyl-6-(2-methyl-5-tetrahydropyranylmethoxybenzoyl)-9H-ca rbozole-3-yl]-1-(O-acetyloxime), and ethanone-1-[9-ethyl-6-{2-methyl-4-(2,2-dimethyl-1,3-dioxolanyl)methoxyben zoyl}-9H-carbozole-3-yl]-1-(O-acetyloxime).

Commercially available photo-radical generators can be used directly. Specific examples include Irgacure 907, Irgacure 184, Irgacure 369, Irgacure 651, Irgacure 819, Irgacure 907, and Irgacure OXE02 (produced by BASF), and ADEKAARKLS N1919T (produced by ADEKA Corporation).

Examples of the anion polymerization initiators include: alkyllithium compounds; monolithium or monosodium salts of biphenyl, naphthalene, pyrene, and the like; and polyfunctional initiators such as dilithium salts and trilithium salts.

Examples of the cation polymerization initiators include: protonic acids such as sulfuric acid, phosphoric acid, perchloric acid, and trifluoromethanesulfonic acid; Lewis acids like boron trifluoride, aluminum chloride, titanium tetrachloride, and tin tetrachloride; and aromatic onium salts or combinations of aromatic onium salts with reductants.

These polymerization initiators may be used alone or in combination.

In the polymerizable liquid crystal composition using the presently disclosed polymerizable compound or mixture, the polymerization initiator is blended at an amount of typically 0.1 to 30 parts by mass and preferably 0.5 to 10 parts by mass, per 100 parts by mass of the mixture containing the polymerizable compounds.

The polymerizable liquid crystal composition using the presently disclosed polymerizable compound or mixture is preferably blended with surfactants for adjustment of surface tension. Although the surfactants are not limited, nonionic surfactants are generally preferred. Commercially available nonionic surfactants will suffice, e.g. nonionic surfactants made of oligomers with a molecular weight on the order of several thousands, such as Ftergent 208G (produced by NEOS).

In the polymerizable liquid crystal composition using the presently disclosed polymerizable compound or mixture, the surfactant is blended at an amount of typically 0.01 to 10 parts by mass and preferably 0.1 to 2 parts by mass, per 100 parts by mass of the total of the polymerizable compounds.

In addition to the mixture containing the polymerizable compounds, the polymerization initiator, and the surfactant, the polymerizable liquid crystal composition using the presently disclosed polymerizable compound or mixture may further contain other components at amounts that do not compromise the effects of the present disclosure. Examples of the other components include metals, metal complexes, dyes, pigments, fluorescent materials, phosphorescent materials, leveling agents, thixotropic agents, gelling agents, polysaccharides, ultraviolet absorbers, infrared absorbers, antioxidants, ion-exchange resins, and metal oxides such as titanium oxide.

Examples of the other components also include other copolymerizable monomers. Specific examples include, but are not limited to, 4'-methoxyphenyl 4-(2-methacryloyloxyethyloxy)benzoate, biphenyl 4-(6-methacryloyloxyhexyloxy)benzoate, 4'-cyanobiphenyl 4-(2-acryloyloxyethyloxy)benzoate, 4'-cyanobiphenyl 4-(2-methacryloyloxyethyloxy)benzoate, 3',4' -difluorophenyl 4-(2-methacryloyloxyethyloxy)benzoate, naphthyl 4-(2-methacryloyloxyethyloxy)benzoate, 4-acryloyloxy-4'-decylbiphenyl, 4-acryloyloxy-4'-cyanobiphenyl, 4-(2-acryloyloxyethyloxy)-4'-cyanobiphenyl, 4-(2-methacryloyloxyethyloxy)-4'-methoxybiphenyl, 4-(2-methacryloyloxyethyloxy)-4'-(4"-fluorobenzyloxy)-biphenyl, 4-acryloyloxy-4'-propylcyclohexylphenyl, 4-methacryloyl-4'-butylbicyclohexyl, 4-acryloyl-4'-amyltolane, 4-acryloyl-4'-(3,4-difluorophenyl)bicyclohexyl, (4-amylphenyl) 4-(2-acryloyloxyethyl)benzoate, (4-(4'-propylcyclohexyl)phenyl) 4-(2-acryloyloxyethyl)benzoate, a commercially available product "LC-242" (produced by BASF), trans-1,4-bis[4-[6-(acryloyloxy)hexyloxy]phenyl]cyclohexanedicarboxylate, and compounds disclosed in JP 2007-002208 A, JP 2009-173893 A, JP 2009-274984 A, JP 2010-030979 A, JP 2010-031223 A, JP 2011-006360 A, and JP 2010-24438 A.

These other components are blended at amounts of typically 0.1 to 20 parts by mass per 100 parts by mass of the total of the polymerizable compounds.

The polymerizable liquid crystal composition using the presently disclosed polymerizable compound or mixture can be typically prepared by mixing and dissolving the polymerizable compound or mixture, the polymerization initiator, and optional other components in predetermined proportions in a suitable organic solvent.

In this case, the polymerizable compounds (I) and (II) as a mixture may be added in the form of pre-mix or may be added separately.

Examples of organic solvents that can be used include: ketones such as cyclopentanone, cyclohexanone, and methyl ethyl ketone; acetic esters such as butyl acetate and amyl acetate; halogenated hydrocarbons such as chloroform, dichloromethane, and dichloroethane; and ethers such as 1,4-dioxane, cyclopentyl methyl ether, tetrahydrofuran, tetrahydropyran, and 1,3-dioxolane.

(4) Polymer

The presently disclosed polymer can be obtained by polymerizing the foregoing polymerizable compound, mixture (mixture containing the polymerizable compounds (I) and (II)), or polymerizable liquid crystal composition.

By the term "polymerization" herein is meant a chemical reaction in a broad sense including a crosslinking reaction as well as a normal polymerization reaction.

The presently disclosed polymer typically includes a monomer unit derived from the polymerizable compound (I) (repeat unit (I)'), and optionally further includes a monomer unit derived from the polymerizable compound (II) (repeat unit (II)').

Because the presently disclosed polymer is prepared using the polymerizable compound (I), it can be advantageously used as the constituent material for an optical film, etc.

The presently disclosed polymer can be used in any shape or form according to its intended use, including film, powder, or layer made of an aggregation of powder.

Specifically, films made of the polymer can be suitably used as the constituent material for the below-described optical film and optically anisotropic product; powders made of the polymer can be utilized for paints, anti-forgery items, security items and the like; and layers made of the polymer powder can be suitably used as the constituent material for the optically anisotropic product.

The presently disclosed polymer can be suitably produced for example by ($\alpha$) polymerizing the mixture containing the polymerizable compounds or the polymerizable liquid crystal composition in a suitable organic solvent, thereafter isolating the target polymer, dissolving the polymer in a suitable organic solvent to prepare a solution, applying the solution on a suitable substrate to form thereon a coating film, and drying the coating film followed by optional heating, or ($\beta$) dissolving the mixture containing the polymerizable compounds or the polymerizable liquid crystal composition in an organic solvent, applying the resulting solution on a substrate by a coating method known in the art and then removing the solvent, and thereafter effecting polymerization by heating or actinic radiation.

Any organic solvent can be used for the polymerization by the method ($\alpha$) as long as it is inert. Examples of the organic solvent include: aromatic hydrocarbons such as toluene, xylene, and mesitylene; ketones such as cyclohexanone, cyclopentanone, and methyl ethyl ketone; acetates such as butyl acetate and amyl acetate; halogenated hydrocarbons such as chloroform, dichloromethane, and dichloroethane; and ethers such as cyclopentyl methyl ether, tetrahydrofuran, and tetrahydropyran.

Of these organic solvents, preferred are those having a boiling point of 60 °C to 250 °C and more preferably those having a boiling point of 60 °C to 150 °C, from the viewpoint of handling capability.

Examples of organic solvents used to dissolve the isolated polymer in the method ($\alpha$) and organic solvents used in the method ($\beta$) include: ketone-based solvents such as acetone, methyl ethyl ketone, methyl isobutyl ketone, cyclopentanone, and cyclohexanone; ester-based solvents such as butyl acetate and amyl acetate; halogenated hydrocarbon-based solvents such as dichloromethane, chloroform, and dichloroethane; ether-based solvents such as tetrahydrofuran, tetrahydropyran, 1,2-dimethoxyethane, 1,4-dioxane, cyclopentyl methyl ether, and 1,3-dioxolane; and aprotic polar solvents such as N,N-dimethylformamide, N,N-dimethylacetamide, dimethyl sulfoxide, $\gamma$-butyrolactone, and N-methylpyrrolidone. Of these organic solvents, preferred are those having a boiling point of 60 °C to 200 °C from the viewpoint of handling capability. These solvents may be used alone or in combination of two or more.

Substrates made of any of organic or inorganic materials known in the art can be used in the methods ($\alpha$) and ($\beta$). Examples of the organic material include polycycloolefins (such as Zeonex® and Zeonor® (Zeonex and Zeonor are registered trademarks in Japan, other countries, or both) produced by Zeon Corporation), Arton® (Arton is a registered trademark in Japan, other countries, or both) produced by JSR Corporation, and Apel® (Apel is a registered trademark in Japan, other countries, or both) produced by Mitsui Chemicals Inc.), polyethylene terephthalates, polycarbonates,

polyimides, polyamides, polymethyl methacrylates, polystyrenes, polyvinyl chlorides, polytetrafluoroethylene, celluloses, cellulose triacetate, and polyethersulfones. Examples of the inorganic material include silicon, glass, and calcite.

The substrate used may be single-layer or laminate.

The substrate is preferably made of organic material, and further preferably a resin film formed of organic material.

Additional examples of the substrate include those used for the production of the below-described optically anisotropic product.

Coating methods known in the art can be used for applying the polymer solution on the substrate in the method (α) and for applying the solution for polymerization reaction on the substrate in the method (β). Specific examples of usable coating methods include curtain coating, extrusion coating, roll coating, spin coating, dip coating, bar coating, spray coating, slide coating, print coating, gravure coating, die coating, and cap coating.

Drying or solvent removal in the methods (α) and (β) can be effected by natural drying, drying by heating, drying under reduced pressure, drying by heating under reduced pressure, or the like.

Polymerization of the mixture and the polymerizable liquid crystal composition can be effected for example by irradiation with actinic radiation or by thermal polymerization, with irradiation with actinic radiation being preferred as heating is unnecessary so that the reaction proceeds at room temperature. Irradiation with UV or other like light is particularly preferred because the operation is simple.

The temperature during irradiation is preferably set to 30 °C or less. The irradiation intensity is typically 1 $W/m^2$ to 10 $kW/m^2$, and preferably 5 $W/m^2$ to 2 $kW/m^2$.

The polymer obtained as described above can be transferred from the substrate for use, removed from the substrate for single use, or used as it is as the constituent material for an optical film etc. without being removed from the substrate.

The polymer removed from the substrate can also be made into powder form by a grinding method known in the art before use.

The number-average molecular weight of the presently disclosed polymer obtained as described above is preferably 500 to 500,000, and more preferably 5,000 to 300,000. If the number-average molecular weight is in such ranges, the resulting film advantageously exhibits high hardness as well as high handling capability. The number-average molecular weight of the polymer can be determined by gel permeation chromatography (GPC) using monodisperse polystyrene as a standard and tetrahydrofuran as an eluant.

The presently disclosed polymer allows for manufacture of a high performance optical film etc. capable of uniform polarized light conversion over a wide wavelength range.

(5) Optical film

The presently disclosed optical film is formed using the presently disclosed polymer, and includes a layer having an optical function. By "optical function" as used herein is meant simple transmittance, reflection, refraction, birefringence, or the like.

The presently disclosed optical film may be used in any of the following arrangements: "alignment substrate/(alignment film)/optical film" where the optical film remains formed on an alignment substrate that may have an alignment film; "transparent substrate film/optical film" where the optical film has been transferred to a transparent substrate film or the like different from the alignment substrate; and single optical film form in the case where the optical film is self-supportive.

Usable alignment films and alignment substrates are the same as those exemplified for the below-described optically anisotropic product.

The presently disclosed optical film can be produced by (A) applying on an alignment substrate a solution of the polymerizable compound, the mixture containing the polymerizable compounds, or the polymerizable liquid crystal composition, drying the resulting coating film, subjecting the film to heat treatment (for alignment of liquid crystals), and irradiation and/or heating treatment (for polymerization); or (B) applying on an alignment substrate a solution of a liquid crystal polymer obtained by polymerization of the polymerizable compound, the mixture containing the polymerizable compounds, or the liquid crystal composition, and optionally drying the resulting coating film.

The presently disclosed optical film can be used for optically anisotropic products, alignment films for liquid crystal display devices, color filters, low-pass filters, polarization prisms, and various optical filters.

The presently disclosed optical film preferably has α and β values that fall within predetermined ranges, which can be calculated as follows based on phase differences at 449.9 nm, 548.5 nm, and 650.2 nm in wavelength measured with an ellipsometer. Specifically, α value is preferably 0.70 to 0.99, and more preferably 0.75 to 0.90. β value is preferably 1.00 to 1.25, and more preferably 1.01 to 1.20.

$$\alpha = (\text{phase difference at 449.9 nm})/(\text{phase difference at 548.5 nm}).$$

β = (phase difference at 650.2 nm)/(phase difference at 548.5 nm).

(6) Optically anisotropic product

The presently disclosed optically anisotropic product has a layer having the presently disclosed polymer as the constituent material.

The presently disclosed optically anisotropic product can be obtained for example by forming an alignment film on a substrate and forming a layer made of the presently disclosed polymer (liquid crystal layer) on the alignment film. The presently disclosed optically anisotropic product may be obtained by directly forming a layer made of the presently disclosed polymer (liquid crystal layer) on a substrate or may consist only of a layer made of the presently disclosed polymer (liquid crystal layer).

The layer made of the polymer may be formed of a polymer film or may be an aggregate of a powdery polymer.

The alignment film is formed on the surface of the substrate to regulate the polymerizable liquid crystal compounds to align in one direction in the plane.

The alignment film can be obtained for example by applying a solution containing a polymer such as polyimide, polyvinyl alcohol, polyester, polyarylate, polyamideimide, or polyetherimide (alignment film composition) on the substrate to form a film, drying the film, and rubbing the film in one direction.

The thickness of the alignment film is preferably 0.001 μm to 5 μm, and further preferably 0.001 μm to 1 μm.

Any method can be used for the rubbing treatment. For example, the alignment film may be rubbed in a given direction using a roll around which a cloth or felt formed of synthetic fiber (e.g. nylon) or natural fiber (e.g. cotton) is wound. It is preferable to wash the alignment film with isopropyl alcohol or the like after completion of the rubbing treatment, in order to remove fine powder (foreign substance) formed during the rubbing treatment to clean the surface of the alignment film.

Alternative to the rubbing treatment, the alignment film can be provided with a function of in-plane one-direction alignment by irradiation with polarized UV light on the surface of the alignment film.

Examples of the substrates on which the alignment film is to be formed include glass substrates and substrates formed of synthetic resin films. Examples of synthetic resins include thermoplastic resins such as acrylic resins, polycarbonate resins, polyethersulfone resins, polyethylene terephthlate resins, polyimide resins, polymethyl methacrylate resins, polysulfone resins, polyarylate resins, polyethylene resins, polystyrene resins, polyvinyl chloride resins, cellulose diacetate, cellulose triacetate, and alicyclic olefin polymers.

Examples of the alicyclic olefin polymers include: cyclic olefin random multi-component copolymers described in JP H05-310845 A and US 5179171 A; hydrogenated polymers described in JP H05-97978 A and US 5202388 A; and thermoplastic dicyclopentadiene open-ring polymers and hydrogenated products thereof described in JP H11-124429 A (WO 99/20676 A1).

In the present disclosure, examples of methods of forming a liquid crystal layer made of the presently disclosed polymer on the alignment film are the same as those described in the above chapter for the presently disclosed polymer (the methods (α) and (β)).

The resulting liquid crystal layer may be of any thickness, and typically has a thickness of 1 μm to 10 μm.

The presently disclosed optically anisotropic product can be used as any product, e.g. as a retardation film, a viewing-angle enhancing film, or the like.

The presently disclosed optically anisotropic product preferably has α and β values that fall within predetermined ranges, which can be calculated as follows based on phase differences at 449.9 nm, 548.5 nm, and 650.2 nm in wavelength measured with an ellipsometer. Specifically, α value is preferably 0.70 to 0.99, and more preferably 0.75 to 0.90. β value is preferably 1.00 to 1.25, and more preferably 1.01 to 1.20.

α = (phase difference at 449.9 nm)/(phase difference at 548.5 nm).

β = (phase difference at 650.2 nm)/(phase difference at 548.5 nm).

(7) Polarizing plate, etc.

The presently disclosed polarizing plate includes the presently disclosed optically anisotropic product and a polarizing film.

A specific example of the presently disclosed polarizing plate is a polarizing plate obtained by laminating the presently disclosed optically anisotropic product on a polarizing film either directly or with other layer(s) (e.g. glass plate) disposed between the optically anisotropic product and the polarizing film.

Any method can be used for the manufacture of the polarizing film. Examples of methods of manufacturing a PVA polarizing film include: a method wherein iodine ions are adsorbed onto a PVA film followed by uniaxial stretching of the PVA film; a method wherein a PVA film is uniaxially stretched followed by adsorption of iodine ions; a method

wherein adsorption of iodine ions to a PVA film and uniaxial stretching are simultaneously performed; a method wherein a PVA film is dyed with dichroic dye followed by uniaxial stretching; a method wherein a PVA film is uniaxially stretched followed by dying with dichroic dye; and a method wherein dying of a PVA film with dichroic dye and uniaxial stretching are simultaneously performed. Examples of methods of manufacturing a polyene polarizing film include known methods in the art, e.g., a method wherein a PVA film is uniaxially stretched followed by heating and dehydration in the presence of a dehydration catalyst, and a method wherein a polyvinyl chloride film is uniaxially stretched followed by heating and dechlorination in the presence of a dechlorination catalyst.

In the presently disclosed polarizing plate, the polarizing film and the presently disclosed optically anisotropic product may be bonded with an adhesive layer made of an adhesive (including tackifier). The average thickness of the adhesive layer is typically 0.01 μm to 30 μm, and preferably 0.1 μm to 15 μm. The adhesive layer preferably has a tensile fracture strength of 40 MPa or less as measured in accordance with JIS K 7113.

Examples of adhesives for the adhesive layer include acrylic adhesives, urethane adhesives, polyester adhesives, polyvinyl alcohol adhesives, polyolefin adhesives, modified polyolefin adhesives, polyvinyl alkyl ether adhesives, rubber adhesives, vinyl chloride-vinyl acetate adhesives, styrene-butadiene-styrene copolymer (SBS copolymer) adhesives and their hydrogenated product (SEBS copolymer) adhesives, ethylene adhesives such as ethylene-vinyl acetate copolymers and ethylene-styrene copolymers, and acrylate adhesives such as ethylene-methyl methacrylate copolymer, ethylene-methyl acrylate copolymer, ethylene-ethyl methacrylate copolymer, and ethylene-ethyl acrylate copolymer.

The presently disclosed polarizing plate includes the presently disclosed optically anisotropic product, and therefore can be manufactured at low cost as well as having such superior performance as low reflected luminance and capability of uniform polarized light conversion over a wide wavelength range.

By using the presently disclosed polarizing plate, it is possible to suitably manufacture display devices that include a panel and a polarizing plate and antireflection films. Examples of such display devices include a flat panel display device using a liquid crystal panel as a panel, and an organic electroluminescent display device using an organic electroluminescent panel as a panel.

EXAMPLES

The presently disclosed techniques will be described in more detail below by way of examples, which however shall not be construed as limiting the scope of the present disclosure in any way.

(Synthesis Example 1) synthesis of compound 1

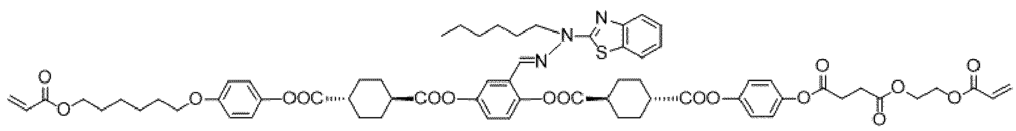

Compound 1

Step 1: synthesis of intermediate A

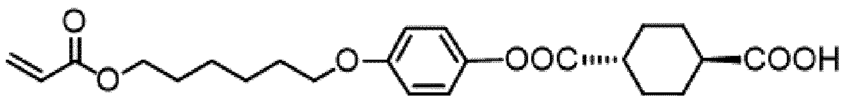

Intermediate A

A three-necked reactor equipped with a thermometer was charged with 17.98 g (104.42 mmol) of trans-1,4-cyclohexanedicarboxylic acid and 180 ml of tetrahydrofuran (THF) under a nitrogen stream. 6.58 g (57.43 mmol) of methanesulfonyl chloride was then added, and the reactor was immersed in a water bath to adjust the reaction solution temperature to 20 °C. Next, 6.34 g (62.65 mmol) of triethylamine was added dropwise over 10 min while retaining the

reaction solution temperature to 20 °C to 30 °C. After the dropwise addition, the entire mass was further stirred at 25 °C for 2 hr.

To the resulting reaction solution were added 0.64 g (5.22 mmol) of 4-(dimethylamino)pyridine and 13.80 g (52.21 mmol) of 4-(6-acryloyloxy-hex-1-yloxy)phenol (produced by DKSH Japan K.K.), and the reactor was again immersed in the water bath to adjust the reaction solution temperature to 15 °C. 6.34 g (62.65 mmol) of triethylamine was added dropwise over 10 min while retaining the reaction solution temperature to 20 °C to 30 °C. After the dropwise addition, the entire mass was further stirred at 25 °C for 2 hr. After the completion of the reaction, 1,000 ml of distilled water and 100 ml of saturated brine were added to the reaction solution, and extracted twice with 400 ml of ethyl acetate. The organic layer was collected and dried with sodium sulfate anhydrous, and sodium sulfate was filtered. The solvent was evaporated from the filtrate using a rotary evaporator, and the residue was purified by silica gel column chromatography (THF:toluene = 1:9 (volume ratio), hereafter the same). Analysis was performed by high-performance liquid chromatography, and purification by silica gel column chromatography was repeated until the purity reached 99.5 % or more. As a consequence, 14.11 g of intermediate A was obtained as a white solid (yield: 65 mol%). The structure of the target compound was identified by $^1$H-NMR. The results are as follows.

$^1$H-NMR (500 MHz, DMSO-$d_6$, TMS, $\delta$ ppm): 12.12 (s, 1H), 6.99 (d, 2H, J=9.0Hz), 6.92 (d, 2H, J=9.0Hz), 6.32 (dd, 1H, J=1.5Hz, 17.5Hz), 6.17 (dd, 1H, J=10.0Hz, 17.5Hz), 5.93 (dd, 1H, J=1.5Hz, 10.0Hz), 4.11 (t, 2H, J=6.5Hz), 3.94 (t, 2H, J=6.5Hz), 2.48-2.56 (m, 1H), 2.18-2.26 (m, 1H), 2.04-2.10 (m, 2H), 1.93-2.00 (m, 2H), 1.59-1.75 (m, 4H), 1.35-1.52 (m, 8H).

Step 2: synthesis of intermediate B

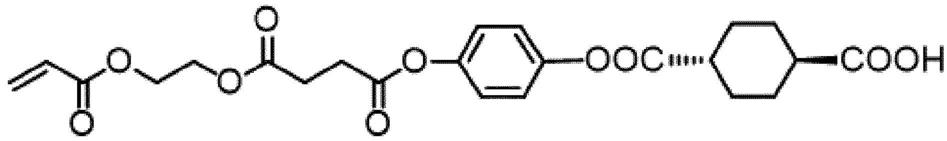

Intermediate B

The same operation as in Example 5 described in WO 2011/068138 A1 was performed to synthesize intermediate B.

Step 3: synthesis of intermediate C

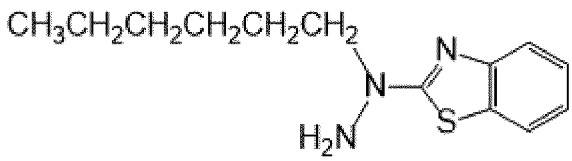

Intermediate C

In a four-necked reactor equipped with a thermometer, 2.00 g (12.1 mmol) of 2-hydrazinobenzothiazole was dissolved in 20 ml of N,N-dimethylformamide (DMF) under a nitrogen stream. 8.36 g (60.5 mmol) of potassium carbonate and 3.08 g (14.5 mmol) of 1-iodohexane were added to this solution, and stirred at 50 °C for 7 hr. After the completion of the reaction, the reaction solution was cooled to 20 °C, and the reaction solution was added to 200 ml of water and extracted with 300 ml of ethyl acetate. The ethyl acetate layer was then dried with anhydrous sodium sulfate. After filtering the sodium sulfate, the ethyl acetate was distilled under reduced pressure using a rotary evaporator, to obtain a yellow solid. The yellow solid was purified by silica gel column chromatography (hexane:ethyl acetate = 75:25), thus obtaining 2.10 g of intermediate C as a white solid (yield: 69.6 mol%).

The structure of the target compound was identified by $^1$H-NMR. The results are as follows.

$^1$H-NMR (500 MHz, $CDCl_3$, TMS, $\delta$ ppm): 7.60 (dd, 1H, J=1.0Hz, 8.0Hz), 7.53 (dd, 1H, J=1.0Hz, 8.0Hz), 7.27 (ddd, 1H,

J=1.0Hz, 8.0Hz, 8.0Hz), 7.06 (ddd, 1H, J=1.0Hz, 8.0Hz, 8.0Hz), 4.22 (s, 2H), 3.74 (t, 2H, J=7.5Hz), 1.69-1.76 (m, 2H), 1.29-1.42 (m,6H), 0.89 (t, 3H, J=7.0Hz)

Step 4: synthesis of intermediate D

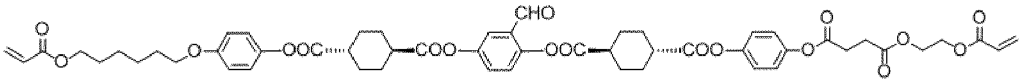

Intermediate D

5 g (36.2 mmol) of 2,5-dihydroxybenzaldehyde, 7.57 g (18.1 mmol) of intermediate A synthesized in step 1, and 22 mg (0.18 mmol) of 4-(dimethylamino)pyridine were added to 150 ml of chloroform. 2.28 g (18.1 mmol) of N, N'-diisopropylcarbodiimide was slowly added dropwise to this solution at room temperature. After performing reaction for 3 hr, 8.08 g (18.1 mmol) of intermediate B synthesized in step 2 was added. Subsequently, 2.28 g (18.1 mmol) of N, N'-diisopropylcarbodiimide was slowly added dropwise at room temperature, and reaction was further performed for 3 hr. After the completion of the reaction, the reaction solution was filtered using a filter medium precoated with a silica gel, and then concentrated under reduced pressure. The resulting residue was dissolved in 250 ml of THF. 1.5 liters of methanol was added to the solution to precipitate a solid, and the precipitated solid was filtered. The resulting solid was washed with methanol and then vacuum dried, thus obtaining 8 g of a white solid mainly composed of intermediate D. Without further purification, the white solid was used in the next step.

Step 5: synthesis of compound 1

80 ml of THF and 10 ml of ethanol were added to 5 g of the white solid mainly composed of intermediate D obtained in step 4 and 1.65 g (6.61 mmol) of intermediate C synthesized in step 3. 153 mg (0.66 mmol) of (±)-10-camphorsulfonic acid was added to this solution, and reacted at 40 °C for 3 hr. After the completion of the reaction, the reaction solution was added to 500 ml of 5 mass% sodium bicarbonate water and extracted with 300 ml of ethyl acetate. The ethyl acetate layer was then dried with anhydrous sodium sulfate. After filtering the sodium sulfate, the ethyl acetate was distilled under reduced pressure using a rotary evaporator, to obtain a yellow solid. The yellow solid was purified by silica gel column chromatography (toluene:ethyl acetate = 85:15), thus obtaining 2.10 g of compound 1 as a pale yellow solid. Mass spectrum measurement showed MS (m/z) = 1214 [M+].

Compound 1 corresponds to the polymerizable compound (I) in which $L^{1a}$ and $L^{2a}$ have different structures, $G^1$ and $G^2$ have different structures, and $P^{1a}$ and $P^{2a}$ have the same structure.

The phase transition temperature of the obtained compound 1 was measured by texture observation with a polarizing microscope. When increasing temperature, compound 1 changed to nematic phase at about 90 °C.

(Synthesis Example 2) synthesis of compound 2

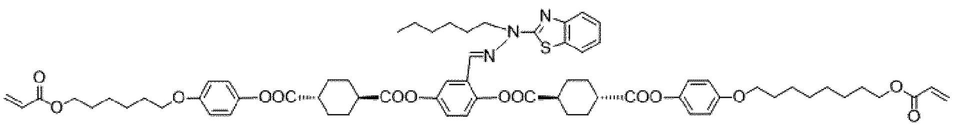

Compound 2

Step 1: synthesis of intermediate E

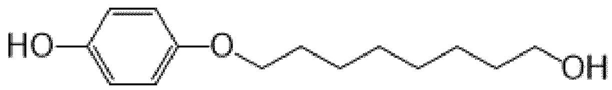

Intermediate E

A three-necked reactor equipped with a thermometer was charged with 7.28 g (66.1 mmol) of hydroquinone, 2.38 g (59.5 mmol) of sodium hydroxide, and 50 ml of distilled water under a nitrogen stream. The solution was heated under reflux, 9.90 g (60.1 mmol) of 8-chloro1-n-octanol was added dropwise over 30 min, and reaction was performed for 5 hr under reflux conditions. After the completion of the reaction, the solution was returned to room temperature, and the precipitated white solid was filtered. The obtained solid was recrystallized in 120 ml of toluene, thus obtaining 7.93 g of intermediate E as a white solid (yield: 56.1 mol%).

The structure of the target compound was identified by $^1$H-NMR. The results are as follows.
$^1$H-NMR (500 MHz, DMSO-$d_6$, TMS, $\delta$ ppm): 8.86 (s, 1H), 6.72 (dd, 2H, J=2.5Hz, 8.0Hz), 6.65 (dd, 2H, J=2.5Hz, 8.0Hz), 4.33 (t, 1H, J=5.0Hz), 3.82 (t, 2H, J=6.5Hz), 3.37 (dt, 2H, J=5.0Hz, 6.5Hz), 1.65 (tt, 2H, J=6.5Hz, 6.5Hz), 1.28-1.42 (m, 10H).

Step 2: synthesis of intermediate F

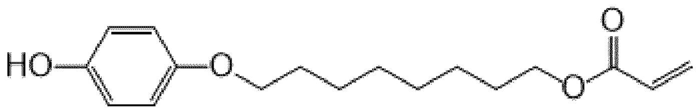

Intermediate F

A three-necked reactor equipped with a thermometer was charged with 7.84 g (32.9 mmol) of intermediate E synthesized in step 1, 2.61 g (36.2 mmol) of acrylic acid, 40.8 mg (0.329 mmol) of 4-methoxyphenol, 316 mg (3.29 mmol) of methanesulfonic acid, and 40 ml of toluene under a nitrogen stream. The solution was heated, reacted for 6 hr under reflux conditions, and then returned to room temperature. The reaction solution was added to 200 ml of water and extracted with 100 ml of ethyl acetate. The obtained ethyl acetate layer was then dried with anhydrous sodium sulfate. After filtering the sodium sulfate, the ethyl acetate was distilled under reduced pressure from the filtrate using a rotary evaporator, to obtain a brown solid. The brown solid was purified by silica gel column chromatography (toluene:tetrahydrofuran = 95:5), thus obtaining 6.95 g of intermediate F as a white solid (yield: 71.9 mol%)
The structure of the target compound was identified by $^1$H-NMR. The results are as follows.
$^1$H-NMR (500 MHz, DMSO-$d_6$, TMS, $\delta$ ppm): 8.86 (s, 1H), 6.72 (dd, 2H, J=2.5Hz, 9.0Hz), 6.65 (dd, 2H, J=2.5Hz, 8.0Hz), 6.31 (dd, 1H, J=1.5Hz, 17.5Hz), 6.17 (dd, 1H, J=10.5Hz, 17.5Hz), 5.93 (dd, 1H, J=1.5Hz, 10.5Hz), 4.10 (t, 2H, J=6.5Hz), 3.83 (t, 2H, J=6.5Hz), 1.58-1.68 (m, 4H), 1.30-1.39 (m, 8H)

Step 3: synthesis of intermediate G

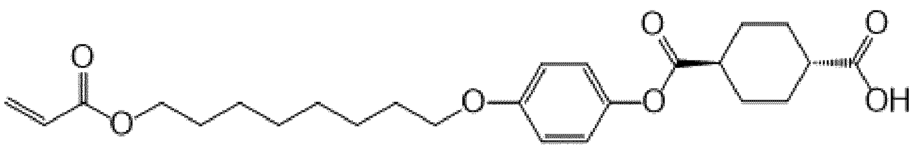

Intermediate G

A three-necked reactor equipped with a thermometer was charged with 6.86 g (39.8 mmol) of trans-1,4-cyclohexanedicarboxylic acid, 70 ml of tetrahydrofuran, and 14 ml of N,N-dimethylformamide under a nitrogen stream.

2.28 g (19.9 mmol) of methanesulfonyl chloride was then added, and the reactor was immersed in a water bath to adjust the reaction solution temperature to 20 °C. 2.20 g (21.7 mmol) of triethylamine was added dropwise over 5 min while retaining the reaction solution temperature to 20 °C to 30 °C. The water bath was removed, and reaction was performed at room temperature for 2 hr. Subsequently, 221 mg (1.81 mmol) of N,N-dimethylaminopyridine and 5.30 g (18.1 mmol) of intermediate F synthesized in step 2 were added to the reaction solution, and the reactor was again immersed in the water bath to adjust the reaction solution temperature to 15 °C. 2.20 g (21.7 mmol) of triethylamine was added dropwise over 5 min while retaining the reaction solution temperature to 20 °C to 30 °C. After performing reaction at room temperature for 2 hr, 300 ml of distilled water and 100 ml of saturated brine were added to the reaction solution, and extracted twice with 100 ml of ethyl acetate. The organic layer was dried with sodium sulfate anhydrous, and sodium sulfate was filtered. The filtrate was concentrated using a rotary evaporator, and purified by silica gel column chromatography (toluene:tetrahydrofuran = 85:15), thus obtaining 5.23 g of intermediate G as a white solid (yield: 64.6 mol%).

The structure of the target compound was identified by $^1$H-NMR. The results are as follows.

$^1$H-NMR (500 MHz, DMSO-$d_6$, TMS, $\delta$ ppm): 12.1 (s, 1H), 6.98 (dd, 2H, J=2.5Hz, 9.0Hz), 6.92 (dd, 2H, J=2.5Hz, 8.0Hz), 6.31 (dd, 1H, J=1.5Hz, 17.5Hz), 6.17 (dd, 1H, J=10.5Hz, 17.5Hz), 5.92 (dd, 1H, J=1.5Hz, 10.5Hz), 4.10 (t, 2H, J=6.5Hz), 3.93 (t, 2H, J=6.5Hz), 2.19-2.25 (m, 1H), 2.04-2.10 (m, 2H), 1.94-1.98 (m, 2H), 1.69 (tt, 2H, J=6.5Hz, 6.5Hz), 1.57-1.64 (m, 2H), 1.31-1.52 (m, 13H).

Step 4: synthesis of intermediate H

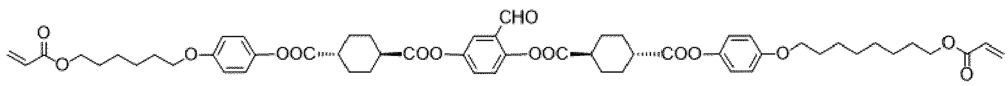

Intermediate H

5 g (36.2 mmol) of 2,5-dihydroxybenzaldehyde, 7.57 g (18.1 mmol) of intermediate A synthesized in step 1 of Synthesis Example 1, and 22 mg (0.18 mmol) of 4-(dimethylamino)pyridine were added to 120 ml of chloroform. 2.28 g (18.1 mmol) of N, N'-diisopropylcarbodiimide was slowly added dropwise to this solution at room temperature. After performing reaction for 3 hr, 8.08 g (18.1 mmol) of intermediate G synthesized in step 3 was added. Subsequently, 2.28 g (18.1 mmol) of N, N'-diisopropylcarbodiimide was slowly added dropwise at room temperature, and reaction was further performed for 3 hr. After the completion of the reaction, the reaction solution was filtered using a filter medium precoated with a silica gel, and then concentrated under reduced pressure. The resulting residue was dissolved in 230 ml of THF. 1.3 liters of methanol was added to the solution to precipitate a solid, and the precipitated solid was filtered. The resulting solid was washed with methanol and then vacuum dried, thus obtaining 8.3 g of a white solid mainly composed of intermediate H. Without further purification, the white solid was used in the next step.

Step 5: synthesis of compound 2

A three-necked reactor equipped with a thermometer was charged with 3.0 g of the white solid mainly composed of intermediate H synthesized in step 4, 1.01 mg (4.0 mmol) of intermediate C synthesized in step 3 of Synthesis Example 1, 72 mg (0.31 mmol) of (±)-10-camphorsulfonic acid, 100 ml of THF, and 10 ml of ethanol under a nitrogen stream, to yield a uniform solution. The solution was then reacted at 40 °C for 4 hr. After the completion of the reaction, the reaction solution was added to 500 ml of water and extracted with 220 ml of chloroform. The obtained chloroform layer was then dried with anhydrous sodium sulfate. After filtering the sodium sulfate, the chloroform was distilled under reduced pressure from the filtrate using a rotary evaporator, to obtain a yellow solid. The yellow solid was purified by silica gel column chromatography (toluene:ethyl acetate = 95:5), thus obtaining 1.38 g of compound 2 as a pale yellow solid.

The structure of the target compound was identified by $^1$H-NMR. The results are as follows.

Compound 2 corresponds to the polymerizable compound (I) in which $L^{1a}$ and $L^{2a}$ have the same structure, $G^1$ and $G^2$ have different structures, and $P^{1a}$ and $P^{2a}$ have the same structure.

$^1$H-NMR (500 MHz, $CDCl_3$, TMS, $\delta$ ppm): 7.75 (d, 1H, J=1.5Hz), 7.66-7.70 (m, 3H), 7.34 (dd, 1H, J=1.5Hz, 7.8Hz), 7.09-7.18 (m, 3H), 6.96-7.00 (m, 4H), 6.86-6.90 (m, 4H), 6.41 (dd, 2H, J=1.5Hz, 17.5Hz), 6.12 (dd, 2H, J=10.5Hz, 17.5Hz), 5.81 (dd, 2H, J=1.5Hz, 10.5Hz), 4.30 (t, 2H, J=7.5Hz), 4.16 (t, 4H, J=6.5Hz), 3.94 (t, 4H, J=6.5Hz), 2.56-2.72 (m, 4H), 2.27-2.38 (m, 8H), 1.65-1.81 (m, 18H), 1.32-1.49 (m, 18H), 0.90 (t, 3H, J=7.5Hz).

The phase transition temperature of the obtained compound 2 was measured by texture observation with a polarizing microscope. When increasing temperature, compound 2 changed to nematic phase at about 92 °C.

(Synthesis Example 3) synthesis of compound 3

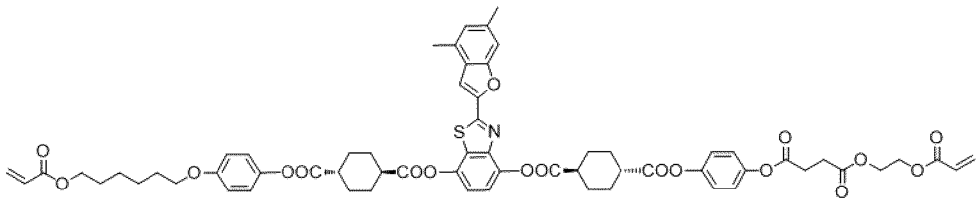

Compound 3

Step 1: synthesis of intermediate I

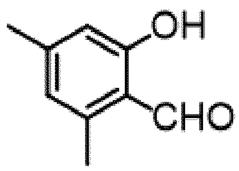

Intermediate I

In a four-necked reactor equipped with a thermometer, 20.0 g (164 mmol) of 3,5-dimethylphenol was dissolved in 500 ml of acetonitrile under a nitrogen stream. 23.4 g (246 mmol) of magnesium chloride and 58.1 g (574 mmol) of triethylamine were added to this solution, and stirred at 25 °C for 30 min. After this, 14.8 g (492 mmol) of paraformaldehyde was added, and stirred at 75 °C for 3 hr. After the completion of the reaction, the reaction solution was cooled to 30 °C, and then 600 ml of 1M hydrochloric acid was added and extracted with 800 ml of diethylether. The diethylether layer was washed with 300 ml of a saturated aqueous solution of sodium hydrogen carbonate and 300 ml of saturated brine, and then dried with anhydrous magnesium sulfate. After filtering the magnesium sulfate, the diethylether was distilled under reduced pressure using a rotary evaporator, to obtain a white solid. The white solid was purified by silica gel column chromatography (hexane:ethyl acetate = 90:10), thus obtaining 17.7 g of intermediate I as a white solid (yield: 71.9 mol%).

The structure of the target compound was identified by $^1H$-NMR. The results are as follows.

$^1H$-NMR (500 MHz, $CDCl_3$, TMS, $\delta$ ppm): 11.95 (s, 1H), 10.22 (s, 1H), 6.61 (s, 1H), 6.53 (s, 1H), 2.54 (s, 3H), 2.30 (s, 3H).

Step 2: synthesis of intermediate J

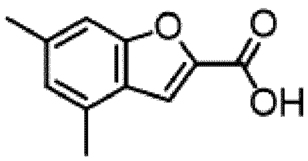

Intermediate J

In a four-necked reactor equipped with a thermometer, 12.0 g (79.9 mmol) of intermediate I synthesized in step 1 was dissolved in 105 ml of dimethylacetoamide under a nitrogen stream. 11.0 g (79.9 mmol) of potassium carbonate was added to this solution and heated to 80 °C, and then 13.3 g (79.9 mmol) of bromoethyl acetate was added over 30 min. The solution was stirred at 80 °C for 1 hr, and then heated to 130 °C and further stirred for 1 hr. After this, the

reaction solution was cooled to 30 °C, and then 300 ml of 1M hydrochloric acid was added and extracted with 120 ml of methylisobutylketone. The methylisobutylketone layer was dried with anhydrous sodium sulfate, the sodium sulfate was filtered, and then the methylisobutylketone was distilled under reduced pressure using a rotary evaporator to obtain a pale yellow solid. The pale yellow solid was dissolved in 500 ml of ethanol. 12.0 g (214 mmol) of potassium hydroxide was added to the solution, and stirred at 80 °C for 1 hr. After the completion of the reaction, ethanol was distilled under reduced pressure using a rotary evaporator, thus obtaining a pale yellow solid. The pale yellow solid was dissolved in 300 ml of water, and the resulting solution was washed with 300 ml of toluene and 300 ml of heptane. A 2M sulfuric acid aqueous solution was added to the solution to adjust pH to 3, and then the precipitated solid was filtered, and the filtered solid was vacuum dried to obtain 12.3 g of intermediate J as a white solid (yield: 80.9 mol%).

The structure of the target compound was identified by $^1$H-NMR. The results are as follows.
$^1$H-NMR (500 MHz, $CDCl_3$, TMS, δ ppm): 13.42 (brs, 1H), 7.69 (d, 1H, J=1.0Hz), 7.30 (s, 1H), 6.98 (s, 1H), 2.48 (s, 3H), 2.41 (s, 3H).

Step 3: synthesis of intermediate K

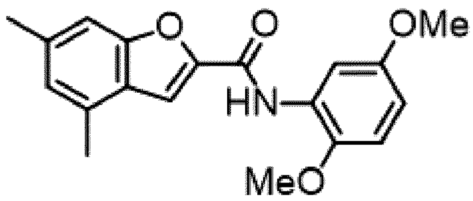

Intermediate K

In a four-necked reactor equipped with a thermometer, 12.0 g (63.1 mmol) of intermediate J synthesized in step 2 and 14.5 g (94.6 mmol) of 2,5-dimethoxyaniline were dissolved in 120 g of chloroform under a nitrogen stream. A mixed solution of 13.3 g (69.4 mmol) of 1-(3-dimethylaminopropyl)-3-ethylcarbodiimide hydrochloride and 120 g of chloroform was added to this solution, and stirred at 25 °C for 3 hr. After the completion of the reaction, chloroform was distilled under reduced pressure using a rotary evaporator, thus obtaining a pale yellow oil. A mixed solution of 200 ml of 1M hydrochloric acid, 200 ml of water, and 100 ml of methanol was added to the pale yellow oil, and stirred at 25 °C. The precipitated white solid was filtered, and the filtered solid was vacuum dried to obtain 16.7 g of intermediate K as a white solid (yield: 81.2 mol%).

The structure of the target compound was identified by $^1$H-NMR. The results are as follows.
$^1$H-NMR (500 MHz, $CDCl_3$, TMS, δ ppm): 8.28 (d, 1H, J=3.0Hz), 7.56 (d, 1H, J=1.0Hz), 7.26 (s, 1H), 7.22 (s, 1H), 6.94 (s, 1H), 6.86 (d, 1H, J=9.0Hz), 6.64 (dd, 1H, J=3.0Hz, 9.0Hz), 3.97 (s, 3H), 3.81 (s, 3H), 2.51 (s, 3H), 2.49 (s, 3H).

Step 4: synthesis of intermediate L

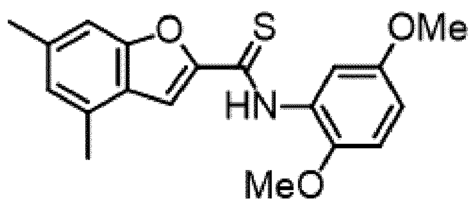

Intermediate L

In a four-necked reactor equipped with a thermometer, 16.0 g (49.2 mmol) of intermediate K synthesized in step 3 was dissolved in 200 ml of toluene under a nitrogen stream. 12.1 g (23.0 mmol) of 2,4-bis(4-methoxyphenyl)-1,3-dithia-2,4-diphosphetane-2,4-disulfide was added to this solution, and heated under reflux for 4 hr. After the completion of the reaction, the reaction solution was cooled to 30 °C, and then 400 ml of 1M sodium hydroxide aqueous solution was added and extracted with 500 ml of toluene. 500 ml of toluene was distilled under reduced pressure from the resulting

toluene layer using a rotary evaporator, and then 500 ml of heptane was added. The precipitated yellow solid was filtered, and the filtered solid was vacuum dried, thus obtaining 14.7 g of intermediate L as a yellow solid (yield: 87.5 mol%).

The structure was identified by $^1$H-NMR. The results are as follows.

$^1$H-NMR (500 MHz, $CDCl_3$, TMS, $\delta$ ppm): 10.45 (s, 1H), 9.13 (d, 1H, J=3.0Hz), 7.82 (d, 1H, J=1.0Hz), 7.18 (s, 1H), 6.93 (s, 1H), 6.91 (d, 1H, J=9.0Hz), 6.77 (dd, 1H, J=3.0Hz, 9.0Hz), 3.97 (s, 3H), 3.83 (s, 3H), 2.51 (s, 3H), 2.46 (s, 3H).

Step 5: synthesis of intermediate M

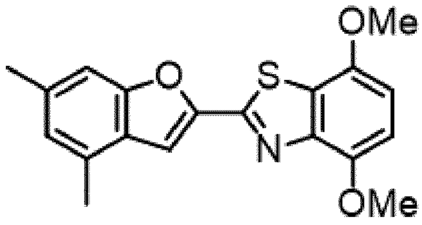

Intermediate M

A four-necked reactor equipped with a thermometer was charged with 13.2 g (38.6 mmol) of intermediate L synthesized in step 4, 220 g of water, and 11.9 g (212 mmol) of potassium hydroxide under a nitrogen stream, and stirred under ice cooling. 29.2 g (88.8 mmol) of potassium ferricyanide and 12 g of methanol were added to the resulting mixed solution, and then heated to 60 °C and stirred for 6 hr. After the completion of the reaction, the reaction solution was cooled to 30 °C, the precipitated yellow solid was filtered, and the filtered solid was vacuum dried, thus obtaining 10.2 g of intermediate M as a yellow solid (yield: 76.8 mol%).

The structure was identified by $^1$H-NMR. The results are as follows.

$^1$H-NMR (500 MHz, $CDCl_3$, TMS, $\delta$ ppm): 7.65 (d, 1H, J=1.0Hz), 7.21 (s, 1H), 6.91 (s, 1H), 6.84 (d, 1H, J=8.5Hz), 6.76 (d, 1H, J=8.5Hz), 4.04 (s, 3H), 3.97 (s, 3H), 2.51 (s, 3H), 2.46 (s, 3H).

Step 6: synthesis of intermediate N

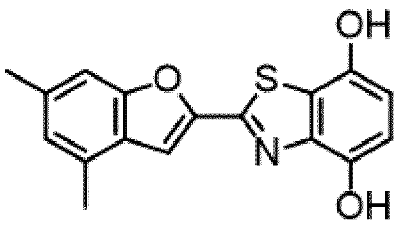

Intermediate N

A four-necked reactor equipped with a thermometer was charged with 7.2 g (21.2 mmol) of intermediate M synthesized in step 5 and 72 g of pyridine hydrochloride under a nitrogen stream, and stirred at 180 °C for 4 hr. After the completion of the reaction, the reaction solution was cooled to 30 °C, and 300 g of water was added. The precipitated solid was filtered, and washed with 30 g of water, 30 g of toluene, and 30 g of hexane. The resulting solid was vacuum dried, thus obtaining 6.38 g of intermediate N as a yellow solid (yield: 96.6 mol%).

The structure of the target compound was identified by $^1$H-NMR. The results are as follows.

$^1$H-NMR (500 MHz, DMSO-$d_6$, TMS, $\delta$ ppm): 9.91 (s, 1H), 9.59 (brs, 1H), 7.76 (d, 1H, J=1.0Hz), 7.36 (s, 1H), 6.99 (s, 1H), 6.79 (d, 1H, J=8.5Hz), 6.74 (d, 1H, J=8.5Hz), 2.53 (s, 3H), 2.43 (s, 3H).

Step 7: synthesis of intermediate O

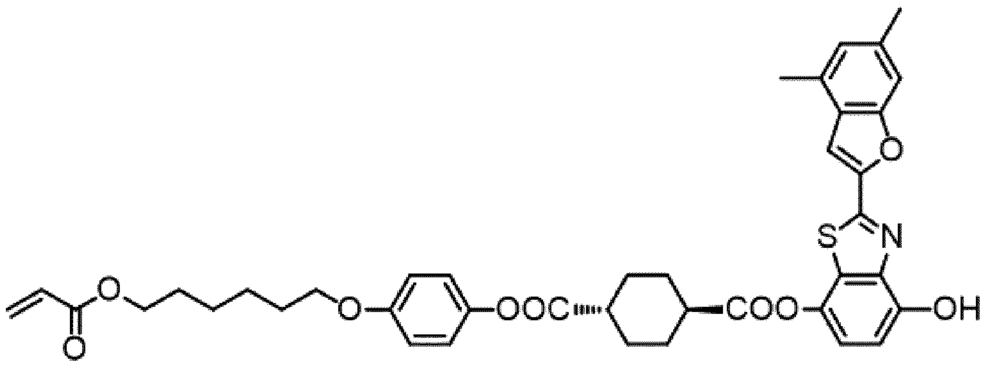

Intermediate O

A three-necked reactor equipped with a thermometer was charged with 4.0 g (12.8 mmol) of intermediate N synthesized in step 6 and 160 ml of THF under a nitrogen stream, and the solution was cooled to 0 °C. 6.44 g (15.4 mmol) of intermediate A synthesized in step 1 of Synthesis Example 1, 156 mg (1.28 mmol) of 4-dimethylaminopyridine, and 1.94 g (15.4 mmol) of N, N'-diisopropylcarbodiimide were added to this solution, and stirred at room temperature for 1 hr. After the completion of the reaction, the reaction solution was added to 200 ml of water and extracted with 400 ml of ethyl acetate. The obtained ethyl acetate layer was then dried with anhydrous sodium sulfate. After filtering the sodium sulfate, a rotary evaporator was used for concentration. The resulting residue was purified by silica gel column chromatography (toluene:ethyl acetate = 90:10), thus obtaining 1.29 g of intermediate O as a flesh-colored solid (yield: 14.1 mol%).

The structure of the target compound was identified by $^1$H-NMR. The results are as follows.

$^1$H-NMR (500 MHz, $CDCl_3$, TMS, $\delta$ ppm): 7.53 (d, 1H, J=1.0Hz), 7.21 (s, 1H), 7.10 (d, 1H, J=9.0Hz), 6.98-7.01 (m, 4H), 6.94 (s, 1H), 6.88 (d, 2H, J=9.0Hz), 6.41 (dd, 1H, J=1.5Hz, 17.5Hz), 6.13 (dd, 1H, J=10.5Hz, 17.5Hz), 6.13 (dd, 1H, J=1.5Hz, 10.5Hz), 4.18 (t, 2H, J=7.0Hz), 3.95 (t, 2H, J=6.5Hz), 2.53 (s, 3H), 2.47 (s, 3H), 2.32-2.43 (m, 4H), 1.67-1.82 (m, 10H), 1.45-1.56 (m, 4H).

Step 8: synthesis of compound 3

A three-necked reactor equipped with a thermometer was charged with 1.2 g (1.69 mmol) of intermediate O synthesized in step 7 and 80 ml of THF under a nitrogen stream, and the solution was cooled to 0 °C. 905 mg (2.03 mmol) of intermediate B synthesized in step 2 of Synthesis Example 1, 24 mg (0.20 mmol) of 4-dimethylaminopyridine, and 307 mg (2.44 mmol) of N, N'-diisopropylcarbodiimide were added to this solution, and stirred at room temperature for 3 hr. After the completion of the reaction, the reaction solution was added to 180 ml of water and extracted with 180 ml of ethyl acetate. The obtained ethyl acetate layer was then dried with anhydrous sodium sulfate. After filtering the sodium sulfate, a rotary evaporator was used for concentration. The resulting residue was purified by silica gel column chromatography (toluene:ethyl acetate = 92:8), thus obtaining 1.22 g of compound 3 as a pale yellow solid (yield: 62.4 mol%). Mass spectrum measurement showed MS (m/z) = 1156 [M+].

Compound 3 corresponds to the polymerizable compound (I) in which $L^{1a}$ and $L^{2a}$ have different structures, $G^1$ and $G^2$ have different structures, and $P^{1a}$ and $P^{2a}$ have the same structure.

The phase transition temperature of the obtained compound 3 was measured by texture observation with a polarizing microscope. When increasing temperature, compound 3 changed to nematic phase at about 165 °C.

(Synthesis Example 4) synthesis of compound 4

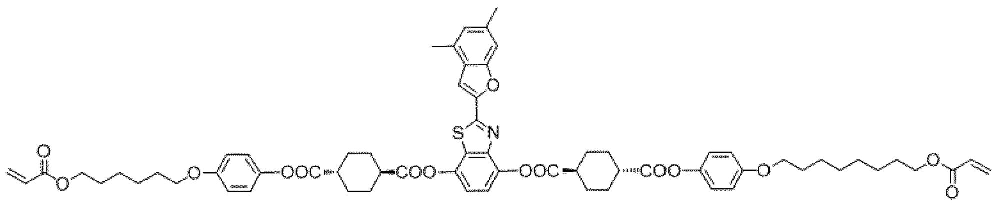

Compound 4

A three-necked reactor equipped with a thermometer was charged with 1.2 g (1.69 mmol) of intermediate O synthesized in step 7 of Synthesis Example 3 and 100 ml of THF under a nitrogen stream, and the solution was cooled to 0 °C. 906 mg (2.03 mmol) of intermediate G synthesized in step 3 of Synthesis Example 2, 24 mg (0.20 mmol) of 4-dimethylaminopyridine, and 307 mg (2.44 mmol) of N, N'-diisopropylcarbodiimide were added to this solution, and stirred at room temperature for 1 hr. After the completion of the reaction, the reaction solution was added to 200 ml of water and extracted with 200 ml of ethyl acetate. The obtained ethyl acetate layer was then dried with anhydrous sodium sulfate. After filtering the sodium sulfate, the filtrate was concentrated using a rotary evaporator. The resulting residue was purified by silica gel column chromatography (toluene:ethyl acetate = 90:10), thus obtaining 1.25 g of compound 4 as a pale yellow solid (yield: 64.9 mol%). Mass spectrum measurement showed MS (m/z) = 1140 [M+].

Compound 4 corresponds to the polymerizable compound (I) in which $L^{1a}$ and $L^{2a}$ have the same structure, $G^1$ and $G^2$ have different structures, and $P^{1a}$ and $P^{2a}$ have the same structure.

The phase transition temperature of the obtained compound 4 was measured by texture observation with a polarizing microscope. When increasing temperature, compound 4 changed to nematic phase at about 180 °C.

(Synthesis Example 5) synthesis of compound 5

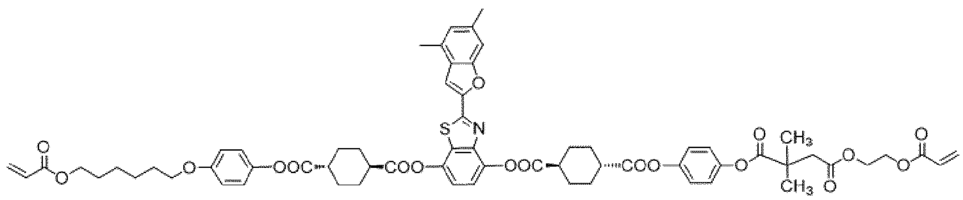

Compound 5

Step 1: synthesis of intermediate P

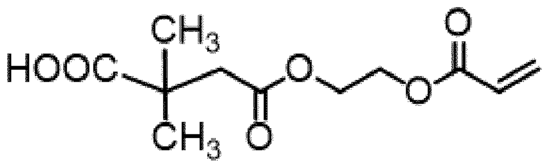

Intermediate P

A three-necked reactor equipped with a thermometer was charged with 10 g (78.05 mmol) of 2,2,-dimethylsuccinic anhydride, 9.06 g (78.05 mmol) of acrylic acid 2-hydroxyethyl, 1.0 g of 2,6-ditertiary butyl-p-cresol, and 200 ml of toluene under air, and the solution was heated under reflux for 3 hr. After the completion of the reaction, the reaction solution was added to 200 ml of water and extracted with 200 ml of ethyl acetate. The obtained ethyl acetate layer was then dried with anhydrous sodium sulfate. After filtering the sodium sulfate, the filtrate was concentrated using a rotary

evaporator. The resulting residue was purified by silica gel column chromatography (toluene:ethyl acetate = 85:15), thus obtaining 3.5 g of intermediate P as a colorless oil (yield: 18.36 mol%).

Step 2: synthesis of intermediate Q

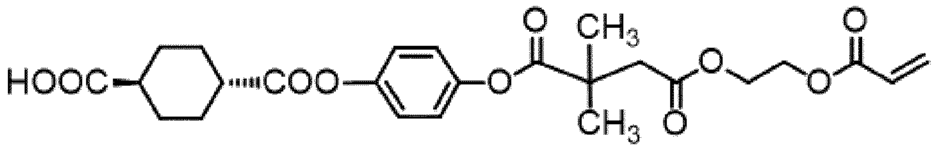

Intermediate Q

The same operation as in Example 5 described in WO 2011/068138 A1 was performed except that succinic mono(2-acryloyloxyethyl) was replaced with intermediate P synthesized in step 1, to synthesize intermediate Q.

Step 3: synthesis of compound 5

A three-necked reactor equipped with a thermometer was charged with 1.2 g (1.69 mmol) of intermediate O synthesized in step 7 of Synthesis Example 3 and 150 ml of THF under a nitrogen stream, and the solution was cooled to 0 °C. 996 mg (2.03 mmol) of intermediate Q synthesized in step 2, 24 mg (0.20 mmol) of 4-dimethylaminopyridine, and 307 mg (2.44 mmol) of N, N'-diisopropylcarbodiimide were added to this solution, and stirred at room temperature for 1 hr. After the completion of the reaction, the reaction solution was added to 250 ml of water and extracted with 200 ml of ethyl acetate. The obtained ethyl acetate layer was then dried with anhydrous sodium sulfate. After filtering the sodium sulfate, the filtrate was concentrated using a rotary evaporator. The resulting residue was purified by silica gel column chromatography (toluene:ethyl acetate = 90:10), thus obtaining 1.15 g of compound 5 as a pale yellow solid (yield: 57.5 mol%). Mass spectrum measurement showed MS (m/z) = 1184 [M+].

Compound 5 corresponds to the polymerizable compound (I) in which $L^{1a}$ and $L^{2a}$ have different structures, $G^1$ and $G^2$ have different structures, and $P^{1a}$ and $P^{2a}$ have the same structure.

The phase transition temperature of the obtained compound 5 was measured by texture observation with a polarizing microscope. When increasing temperature, compound 5 changed to nematic phase at about 177 °C.

(Synthesis Example 6) synthesis of compound 6

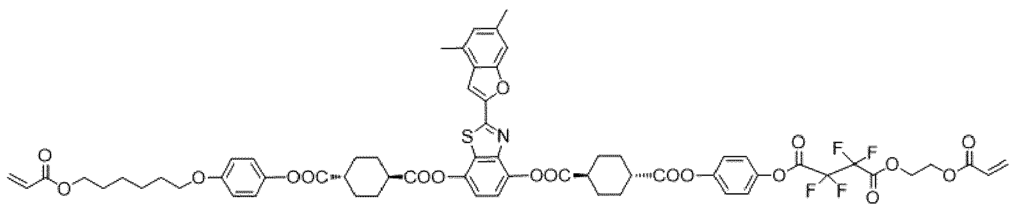

Compound 6

Step 1: synthesis of intermediate R

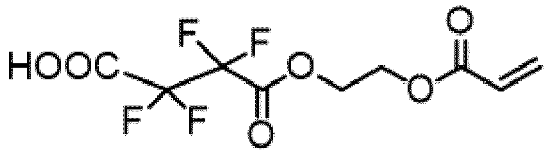

Intermediate R

A three-necked reactor equipped with a thermometer was charged with 10 g (58.13 mmol) of tetrafluorosuccinic anhydride, 6.75 g (58.13 mmol) of acrylic acid 2-hydroxyethyl, 1.0 g of 2,6-ditertiary butyl-p-cresol, and 220 ml of toluene under air, and the solution was heated under reflux for 4 hr. After the completion of the reaction, the reaction solution was added to 200 ml of water and extracted with 200 ml of ethyl acetate. The obtained ethyl acetate layer was then dried with anhydrous sodium sulfate. After filtering the sodium sulfate, the filtrate was concentrated using a rotary evaporator. The resulting residue was purified by silica gel column chromatography (toluene:ethyl acetate = 85:15), thus obtaining 2.2 g of intermediate R as a colorless oil (yield: 13.13 mol%).

Step 2: synthesis of intermediate S

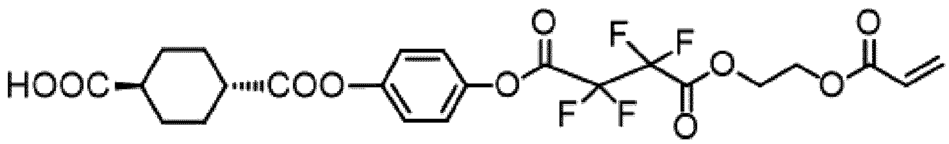

Intermediate S

The same operation as in Example 5 described in WO 2011/068138 A1 was performed except that succinic mono(2-acryloyloxyethyl) was replaced with intermediate R synthesized in step 1, to synthesize intermediate S.

Step 3: synthesis of compound 6

A three-necked reactor equipped with a thermometer was charged with 1.2 g (1.69 mmol) of intermediate O synthesized in step 7 of Synthesis Example 3 and 150 ml of THF under a nitrogen stream, and the solution was cooled to 0 °C. 1.09 g (2.03 mmol) of intermediate S synthesized in step 2, 24 mg (0.20 mmol) of 4-dimethylaminopyridine, and 307 mg (2.44 mmol) of N, N'-diisopropylcarbodiimide were added to this solution, and stirred at room temperature for 1 hr. After the completion of the reaction, the reaction solution was added to 250 ml of water and extracted with 200 ml of ethyl acetate. The obtained ethyl acetate layer was then dried with anhydrous sodium sulfate. After filtering the sodium sulfate, the filtrate was concentrated using a rotary evaporator. The resulting residue was purified by silica gel column chromatography (toluene:ethyl acetate = 90:10), thus obtaining 1.21 g of compound 6 as a pale yellow solid (yield: 58.3 mol%). Mass spectrum measurement showed MS (m/z) = 1128 [M+].

Compound 6 corresponds to the polymerizable compound (I) in which $L^{1a}$ and $L^{2a}$ have different structures, $G^1$ and $G^2$ have different structures, and $P^{1a}$ and $P^{2a}$ have the same structure.

The phase transition temperature of the obtained compound 6 was measured by texture observation with a polarizing microscope. When increasing temperature, compound 6 changed to nematic phase at about 172 °C.

(Comparative Synthesis Example 1) synthesis of compound X1

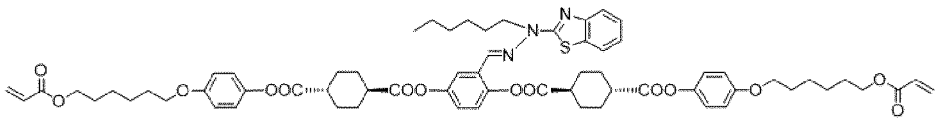

Compound X1

Step 1: synthesis of intermediate T

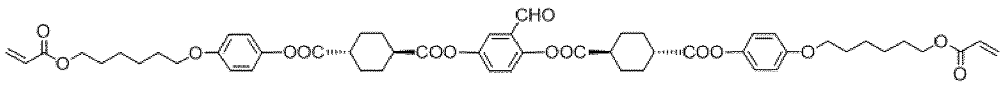

Intermediate T

A three-necked reactor equipped with a thermometer was charged with 4.00 g (9.56 mmol) of intermediate A synthesized in step 1 of Synthesis Example 1 and 60 ml of THF under a nitrogen stream, to yield a uniform solution. 1.12 g (9.78 mmol) of methanesulfonyl chloride was added, and the reactor was immersed in a water bath to adjust the reaction solution temperature to 20 °C. 1.01 g (9.99 mmol) of triethylamine was added dropwise over 5 min while retaining the reaction solution temperature to 20 °C to 30 °C. After the dropwise addition, the entire mass was further stirred at 25 °C for 2 hr. To the resulting reaction solution were added 0.11 g (0.87 mmol) of 4-(dimethylamino)pyridine and 0.60 g (4.35 mmol) of 2,5-dihydroxybenzaldehyde, and the reactor was again immersed in the water bath to adjust the reaction solution temperature to 15 °C. 1.10 g (10.87 mmol) of triethylamine was added dropwise over 5 min while retaining the reaction solution temperature to 20 °C to 30 °C. After the dropwise addition, the entire mass was further stirred at 25 °C for 2 hr. After the completion of the reaction, 400 ml of distilled water and 50 ml of saturated brine were added to the reaction solution, and extracted twice with 750 ml of ethyl acetate. The organic layer was collected and dried with sodium sulfate anhydrous, and sodium sulfate was filtered. The solvent was evaporated from the filtrate using a rotary evaporator. The resulting residue was dissolved in 100 ml of THF. 500 ml of methanol was added to the solution to precipitate crystals, and the precipitated crystals were filtered. The resulting crystals were washed with methanol and then vacuum dried, thus obtaining 2.51 g of intermediate T as a white solid (yield: 62 mol%).

The structure of the target compound was identified by $^1$H-NMR. The results are as follows.

$^1$H-NMR (500 MHz, DMSO-$d_6$, TMS, $\delta$ ppm): 10.02 (s, 1H), 7.67 (d, 1H, J=3.0Hz), 7.55 (dd, 1H, J=3.0Hz, 8.5Hz), 7.38 (d, 1H, J=8.5Hz), 6.99-7.04 (m, 4H), 6.91-6.96 (m, 4H), 6.32 (dd, 2H, J=1.5Hz, 17.5Hz), 6.17 (dd, 2H, J=10.0Hz, 17.5Hz), 5.93 (dd, 2H, J=1.5Hz, 10.0Hz), 4.11 (t, 4H, J=6.5Hz), 3.95 (t, 4H, J=6.5Hz), 2.56-2.81 (m, 4H), 2.10-2.26 (m, 8H), 1.50-1.76 (m, 16H), 1.33-1.49 (m, 8H).

Step 2: synthesis of compound X1

In a four-necked reactor equipped with a thermometer, 697 mg (2.37 mmol) of intermediate C synthesized in step 3 of Synthesis Example 1 and 2.00 g (2.13 mmol) of intermediate T synthesized in step 1 were dissolved in a mixed solvent of 3 ml of ethanol and 20 ml of THF under a nitrogen stream. 55.1 mg (0.237 mmol) of (±)-10-camphorsulfonic acid was added to this solution, and stirred at 40 °C for 5 hr. After the completion of the reaction, the reaction solution was added to 150 ml of water and extracted with 300 ml of ethyl acetate. The ethyl acetate layer was then dried with anhydrous sodium sulfate. After filtering the sodium sulfate, the ethyl acetate was distilled under reduced pressure using a rotary evaporator, to obtain a white solid. The white solid was purified by silica gel column chromatography (toluene:ethyl acetate = 90:10), thus obtaining 2.24 g of compound X1 as a white solid (yield: 86.4 mol%).

The structure of the target compound was identified by $^1$H-NMR. The results are as follows.

Compound X1 corresponds to the polymerizable compound (II) in which $L^{1a}$ and $L^{2a}$ have the same structure, $G^1$ and $G^2$ have the same structure, and $P^{1a}$ and $P^{2a}$ have the same structure.

$^1$H-NMR (400 MHz, $CDCl_3$, TMS, $\delta$ ppm): 7.75 (d, 1H, J=2.5Hz), 7.67-7.70 (m, 3H), 7.34 (ddd, 1H, J=1.0Hz, 7.0Hz, 7.5Hz), 7.17 (ddd, 1H, J=1.0Hz, 7.5Hz, 7.5Hz), 7.12 (d, 1H, J=9.0Hz), 7.10 (dd, 1H, J=2.5Hz, 9.0Hz), 6.99 (d, 2H, J=9.0Hz), 6.98 (d, 2H, J=9.0Hz), 6.88 (d, 4H, J=9.0Hz), 6.40 (dd, 2H, J=1.5Hz, 17.0Hz), 6.13 (dd, 2H, J=10.5Hz, 17.5Hz), 5.82 (dd, 2H, J=1.5Hz, 10.5Hz), 4.30 (t, 2H, J=8.0Hz), 4.18 (t, 4H, J=6.5Hz), 3.95 (t, 4H, J=6.5Hz), 2.58-2.70 (m, 4H), 2.31-2.35 (m, 8H), 1.66-1.82 (m, 18H), 1.31-1.54 (m, 14H), 0.90 (t, 3H, J=7.0Hz).

The phase transition temperature of the obtained compound X1 was measured by texture observation with a polarizing microscope. When increasing temperature, compound X1 changed to nematic phase at about 96 °C.

(Comparative Synthesis Example 2) synthesis of compound X2

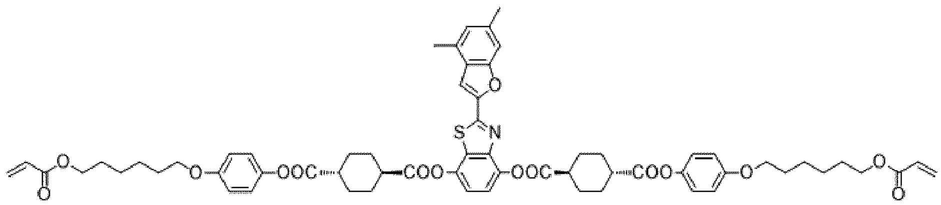

Compound X2

A four-necked reactor equipped with a thermometer was charged with 1.00 g (3.21 mmol) of intermediate N synthesized in step 6 of Synthesis Example 3 and 50 ml of chloroform under a nitrogen stream. 2.96 g (7.07 mmol) of intermediate A synthesized in step 1 of Synthesis Example 1 and 39.2 mg (0.321 mmol) of 4-dimethylaminopyridine were added to this solution, and cooled to 0 °C. After this, 972 mg (7.70 mmol) of N, N'-diisopropylcarbodiimide was added to the solution, and stirred at room temperature for 1.5 hr. After the completion of the reaction, the reaction solution was filtered using a filter medium precoated with a silica gel, and then concentrated under reduced pressure. The resulting residue was purified by silica gel column chromatography (chloroform:ethyl acetate = 90:10), thus obtaining 2.84 g of compound X2 as a white solid (yield: 79.5 %).

The structure of the target compound was identified by $^1$H-NMR. The results are as follows.

Compound X2 corresponds to the polymerizable compound (II) in which $L^{1a}$ and $L^{2a}$ have the same structure, $G^1$ and $G^2$ have the same structure, and $P^{1a}$ and $P^{2a}$ have the same structure.

$^1$H-NMR (500 MHz, $CDCl_3$, TMS, $\delta$ ppm): 7.53 (d, 1H, J=1.0Hz), 7.23 (s, 2H), 7.21 (s, 1H), 6.999 (d, 2H, J=9.0Hz), 6.995 (d, 2H, J=9.0Hz), 6.94 (s, 1H), 6.89 (d, 4H, J=9.0Hz), 6.40 (dd, 2H, J=1.5Hz, 17.5Hz), 6.12 (dd, 2H, J=10.5Hz, 17.5Hz), 5.82 (dd, 2H, J=1.5Hz, 10.5Hz), 4.18 (t, 4H, J=7.0Hz), 3.95 (t, 4H, J=6.5Hz), 2.84 (tt, 1H, J=3.5Hz, 12.0Hz), 2.59-2.75 (m, 3H), 2.54 (s, 3H), 2.47 (s, 3H), 2.42-2.46 (m, 2H), 2.31-2.41 (m, 6H), 1.69-1.87 (m, 16H), 1.41-1.57 (m, 8H).

The phase transition temperature of the obtained compound X2 was measured by texture observation with a polarizing microscope. When increasing temperature, compound X2 changed to nematic phase at about 186 °C.

<Preparation of polymerizable liquid crystal composition>

(Examples 1 to 6)

Compounds 1 to 6 obtained in Synthesis Examples 1 to 6 were each dissolved in 79.49 mass% of cyclopentanone together with 0.61 mass% of BYK316N as a polymerization initiator and 0.02 mass% of Irgacure #819 as a leveling agent in the mixing proportions shown in Table 1. The resulting solution was filtered through a disposable filter with a pore size of 0.45 $\mu$m, to obtain polymerizable liquid crystal compositions 1 to 6.

(Examples 7 to 12)

Combinations of compounds 1 to 6 obtained in Synthesis Examples 1 to 6 and compounds X1 to X2 obtained in Comparative Synthesis Examples 1 to 2 were each dissolved in 79.49 mass% of cyclopentanone together with 0.61 mass% of BYK316N as a polymerization initiator and 0.02 mass% of Irgacure #819 as a leveling agent in the mixing proportions shown in Table 1. The resulting solution was filtered through a disposable filter with a pore size of 0.45 $\mu$m, to obtain polymerizable liquid crystal compositions 7 to 12.

(Comparative Examples 1 to 2)

Compounds X1 to X2 obtained in Comparative Synthesis Examples 1 to 2 were each dissolved in 79.49 mass% of cyclopentanone together with 0.61 mass% of BYK316N as a polymerization initiator and 0.02 mass% of Irgacure #819 as a leveling agent in the mixing proportions shown in Table 1. The resulting solution was filtered through a disposable filter with a pore size of 0.45 $\mu$m, to obtain polymerizable liquid crystal compositions 1r to 2r.

[Table 1]

| | Polymerizable liquid crystal composition | Compound 1 | Compound 2 | Compound 3 | Compound 4 | Compound 5 | Compound 6 | Compound X1 | Compound X2 | Polymerization initiator Irgacure #819 (mass%) | Leveling agent BYK316N (mass%) | Solvent cyclopentanone (mass%) |
|---|---|---|---|---|---|---|---|---|---|---|---|---|
| Example 1 | 1 | 19.87 | | | | | | | | 0.61 | 0.02 | 79.49 |
| Example 2 | 2 | | 19.87 | | | | | | | | | |
| Example 3 | 3 | | | 19.87 | | | | | | | | |
| Example 4 | 4 | | | | 19.87 | | | | | | | |
| Example 5 | 5 | | | | | 19.87 | | | | | | |
| Example 6 | 6 | | | | | | 19.87 | | | | | |
| Example 7 | 7 | 9.935 | | | | | | 9.935 | | | | |
| Example 8 | 8 | | 9.935 | | | | | 9.935 | | | | |
| Example 9 | 9 | | | 9.935 | | | | | 9.935 | | | |
| Example 10 | 10 | | | | 9.935 | | | | 9.935 | | | |
| Example 11 | 11 | | | | | 9.935 | | | 9.935 | | | |
| Example 12 | 12 | | | | | | 9.935 | | 9.935 | | | |
| Comparative Example 1 | 1r | | | | | | | 19.87 | | | | |
| Comparative Example 2 | 2r | | | | | | | | 19.87 | | | |

<Measurement of phase difference and evaluation of wavelength dispersibility>

(i) Formation of liquid crystal layer using polymerizable liquid crystal composition

Using a #4 wire bar coater, each of polymerizable liquid crystal compositions 1 to 12 and 1r to 2r was applied to a transparent glass substrate with a rubbed polyimide alignment film (product name: alignment treated glass substrate (produced by E.H.C Co., Ltd.)). The resulting coating film was dried for 1 min at the temperature shown in Table 2 and subjected to alignment treatment for 1 min at the temperature shown in Table 2 to form a liquid crystal layer. The liquid crystal layer was then irradiated with UV light at 2500 mJ/cm$^2$ at the temperature shown in Table 2 from the coated surface side to effect polymerization, thus obtaining a measurement sample.

(ii) Measurement of phase difference

For each obtained sample, the phase differences between 400 nm and 800 nm were measured using Mueller Matrix Polarimeter Axoscan (produced by Axometrics, Inc.).

(iii) Evaluation of wavelength dispersibility

Wavelength dispersibility was evaluated based on the wavelength dispersion ratios calculated as described below using the measured phase differences.

$$\alpha = \text{(wavelength dispersion ratio at 450 nm)} = \text{(phase difference value at 450 nm)}/\text{(phase difference value at 550 nm)}$$

$$\beta = \text{(wavelength dispersion ratio at 650 nm)} = \text{(phase difference value at 650 nm)}/\text{(phase difference value at 550 nm)}.$$

In the case where the optically anisotropic product exhibits ideal wavelength dispersibility showing a broad band property, i.e. reverse wavelength dispersibility, $\alpha$ value is less than 1 and $\beta$ value is greater than 1. In the case where the optically anisotropic product exhibits flat wavelength dispersibility, $\alpha$ value and $\beta$ value are approximately equal. In the case where the optically anisotropic product exhibits typical (normal) wavelength dispersibility, $\alpha$ value is greater than 1 and $\beta$ value is less than 1. That is, flat wavelength dispersibility with approximately equal $\alpha$ value and $\beta$ value is preferable, and reverse wavelength dispersibility with $\alpha$ value of less than 1 and $\beta$ value of greater than 1 is particularly preferable.

The thickness of the optically anisotropic product was measured as follows: an optically anisotropic product equipped with a transparent glass substrate was scratched with a needle, and the difference in level was measured by surface profiler DEKTAK150 (produced by ULVAC, Inc.).

[Table 2]

| | Polymerizable liquid crystal composition | Compound 1 | Compound 2 | Compound 3 | Compound 4 | Compound 5 | Compound 6 | Compound X1 | Compound X2 | Drying temperature (°C) | Alignment treatment temperature (°C) | Temperature at exposure (°C) | Thickness (μm) | Re (550nm) | α | β |
|---|---|---|---|---|---|---|---|---|---|---|---|---|---|---|---|---|
| Example 1 | 1 | 19.87 | | | | | | | | 95 | 50 | 50 | 1.58 | 119.66 | 0.851 | 1.029 |
| Example 2 | 2 | | 19.87 | | | | | | | 100 | 50 | 50 | 1.59 | 118.12 | 0.838 | 1.031 |
| Example 3 | 3 | | | 19.87 | | | | | | 175 | 100 | 100 | 1.29 | 70.11 | 0.821 | 1.039 |
| Example 4 | 4 | | | | 19.87 | | | | | 185 | 110 | 110 | 1.33 | 69.66 | 0.802 | 1.044 |
| Example 5 | 5 | | | | | 19.87 | | | | 180 | 115 | 115 | 1.28 | 70.08 | 0.819 | 1.040 |
| Example 6 | 6 | | | | | | 19.87 | | | 175 | 115 | 115 | 1.28 | 68.77 | 0.799 | 1.039 |
| Example 7 | 7 | 9.935 | | | | | | 9.935 | | 95 | 50 | 50 | 1.59 | 118.95 | 0.842 | 1.032 |
| Examples 8 | 8 | | 9.935 | | | | | 9.935 | | 100 | 50 | 50 | 1.59 | 118.18 | 0.836 | 1.033 |
| Example 9 | 9 | | | 9.935 | | | | | 9.935 | 175 | 100 | 100 | 1.30 | 69.93 | 0.809 | 1.048 |
| Example 10 | 10 | | | | 9.935 | | | | 9.935 | 185 | 110 | 110 | 1.33 | 69.70 | 0.800 | 1.050 |
| Example 11 | 11 | | | | | 9.935 | | | 9.935 | 180 | 115 | 115 | 1.28 | 70.08 | 0.819 | 1.040 |
| Example 12 | 12 | | | | | | 9.935 | | 9.935 | 175 | 115 | 115 | 1.28 | 68.77 | 0.799 | 1.039 |
| Comparative Example 1 | 1r | | | | | | | 19.87 | | 115 | 50 | 50 | 1.60 | 118.23 | 0.833 | 1.034 |
| Comparative Example 2 | 2r | | | | | | | | 19.87 | 195 | 120 | 120 | 1.35 | 69.74 | 0.797 | 1.056 |

As can be seen from Table 2, by using each of compounds 1 to 2 having the predetermined structure and by using the mixture of each of compounds 1 to 2 having the predetermined structure and compound X1, the drying temperature, the alignment treatment temperature, and the temperature at exposure can be decreased as compared with the case of using only compound X1. Likewise, by using each of compounds 3 to 6 having the predetermined structure and by using the mixture of each of compounds 3 to 6 having the predetermined structure and compound X2, the drying temperature, the alignment treatment temperature, and the temperature at exposure can be decreased as compared with the case of using only compound X2. Thus, polymerizable liquid crystal compositions containing compounds 1 to 6 are easy to coat industrially, and contribute to excellent mass productivity of optically anisotropic products.

As can be seen from Table 2, the optically anisotropic products obtained from the polymerizable liquid crystal compositions containing compounds 1 to 6 all have $\alpha$ of less than 1 and $\beta$ of greater than 1. The optically anisotropic products thus maintain ideal wavelength dispersibility showing a broad band property, i.e. reverse wavelength dispersibility.

## Claims

1. A polymerizable compound represented by the following Formula (I):

$$P^{1a}-G^{1}-L^{1a}\left[B^{1}-L^{1}\right]_{a}A^{1}-Z^{1}-Ar^{1}-Z^{2}-A^{2}\left[L^{2}-B^{2}\right]_{b}L^{2a}-G^{2}-P^{2a}$$

(I)

where $Ar^1$ represents a divalent aromatic hydrocarbon ring group having at least $D^1$ as a substituent or a divalent aromatic heterocyclic group having at least $D^1$ as a substituent,
$D^1$ represents an organic group with a carbon number of 1 to 67 having at least one aromatic ring selected from the group consisting of an aromatic hydrocarbon ring and an aromatic heterocyclic ring,
$Z^1$ and $Z^2$ each independently represent a single bond, -O-, $-O-CH_2-$, $-CH_2-O-$, -C(=O)-O-, -O-C(=O)-, -C(=O)-S-, -S-C(=O)-, $-NR^{21}-C(=O)-$, $-C(=O)-NR^{21}-$, $-CF_2-O-$, $-O-CF_2-$, $-CH_2-CH_2-$, $-CF_2-CF_2-$, $-O-CH_2-CH_2-O-$, -CH=CH-C(=O)-O-, -O-C(=O)-CH=CH-, $-CH_2-CH_2-C(=O)-O-$, $-O-C(=O)-CH_2-CH_2-$, $-CH_2-CH_2-O-C(=O)-$, $-C(=O)-O-CH_2-CH_2-$, -CH=CH-, -N=CH-, -CH=N-, $-N=C(CH_3)-$, $-C(CH_3)=N-$, -N=N-, or -C≡C-, and $R^{21}$ each independently represent a hydrogen atom or an alkyl group with a carbon number of 1 to 6,
$A^1$ and $A^2$ and $B^1$ and $B^2$ each independently represent a cyclic aliphatic group that may have a substituent or an aromatic group that may have a substituent,
$L^1$ and $L^2$ and $L^{1a}$ and $L^{2a}$ each independently represent a single bond, -O-, -C(=O)-, -C(=O)-O-, -O-C(=O)-, $-NR^{22}-C(=O)-$, $-C(=O)-NR^{22}-$, -O-C(=O)-O-, $-NR^{22}-C(=O)-O-$, $-O-C(=O)-NR^{22}-$, or $-NR^{22}-C(=O)-NR^{23}-$, and $R^{22}$ and $R^{23}$ each independently represent a hydrogen atom or an alkyl group with a carbon number of 1 to 6,
$G^1$ and $G^2$ each independently represent an aliphatic hydrocarbon group with a carbon number of 3 to 20 that may have a substituent, the aliphatic hydrocarbon group with a carbon number of 3 to 20 may be interrupted by at least one intervening group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, -N=, =N-, and -N=N-, in the case where two or more intervening groups are present, the two or more intervening groups may be same or different and are not adjacent to each other, and R each independently represent a hydrogen atom or an alkyl group with a carbon number of 1 to 6,
$P^{1a}$ and $P^{2a}$ each independently represent a polymerizable group,
a and b each independently represent 0 or 1, and
a part represented by $-L^{1a}-G^{1}-P^{1a}$ and a part represented by $-L^{2a}-G^{2}-P^{2a}$ have different structures.

2. The polymerizable compound according to claim 1, wherein $L^{1a}$ and $L^{2a}$ have a same structure,
$G^1$ and $G^2$ have different structures, and
$P^{1a}$ and $P^{2a}$ have a same structure.

3. The polymerizable compound according to claim 1 or 2, wherein $G^1$ and $G^2$ have different structures,
one of $G^1$ and $G^2$ is an organic group composed of a plurality of methylene groups that may be substituted and at least one group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, $-O-(CH_2)_n-O-$, -CH=CH-C(=O)-O-, -O-C(=O)-CH=CH-, -CH=CH-, -N=CH-, -CH=N-, $-N=C(CH_3)-$, $-C(CH_3)=N-$, -N=N-, and -C≡C- located between methylene groups that may be substituted,

an other one of $G^1$ and $G^2$ is an alkylene group with a carbon number of 3 to 20 that may have a substituent, or an organic group composed of a plurality of methylene groups that may be substituted and at least one group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, $-O-(CH_2)_n-O-$, -CH=CH-C(=O)-O-, -O-C(=O)-CH=CH-, -CH=CH-, -N=CH-, -CH=N-, $-N=C(CH_3)-$, $-C(CH_3)=N-$, -N=N-, and -C≡C- located between methylene groups that may be substituted, and
R is as defined above, and n represents an integer of 1 to 18.

**4.** The polymerizable compound according to any of claims 1 to 3, wherein $G^1$ and $G^2$ have different structures,
the aliphatic hydrocarbon group with a carbon number of 3 to 20 is an alkylene group with a carbon number of 3 to 20, at least one of $G^1$ and $G^2$ has at least one hydrogen atom substituted by a substituent formed by a halogen atom, a cyano group, an alkyl group with a carbon number of 1 to 6, an alkenyl group with a carbon number of 2 to 6, an alkynyl group with a carbon number of 2 to 6, an alkyl halide group with a carbon number of 1 to 6, an N,N-dialkylamino group with a carbon number of 2 to 6, an alkoxy group with a carbon number of 1 to 6, a nitro group, $-C(=O)-R^{1a}$, $-C(=O)-O-R^{1a}$, or $-O-C(=O)-R^{1a}$, and $R^{1a}$ represents an alkyl group with a carbon number of 1 to 6, an aromatic hydrocarbon ring group with a carbon number of 6 to 20, or an aliphatic hydrocarbon ring group with a carbon number of 6 to 20, and
in the case where a plurality of substituents are present, the plurality of substituents may be same or different.

**5.** The polymerizable compound according to any of claims 1 to 4, wherein $Ar^1$ is a group represented by any of the following Formulas (III-1) to (III-3):

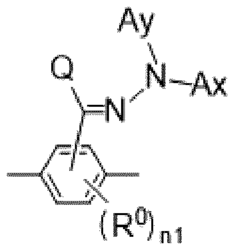
(III-1)

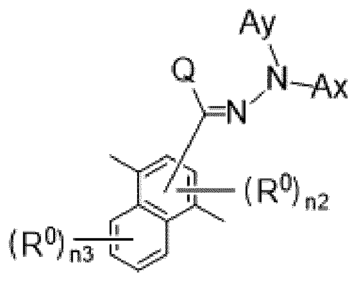
(III-2)

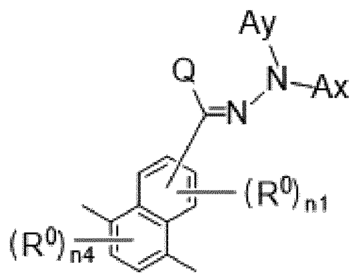
(III-3)

where Ax represents an organic group having at least one aromatic ring selected from the group consisting of an aromatic hydrocarbon ring with a carbon number of 6 to 30 and an aromatic heterocyclic ring with a carbon number of 2 to 30, and the aromatic ring of Ax may have a substituent,
Ay represents a hydrogen atom or an organic group with a carbon number of 1 to 30 that may have a substituent,
Q represents a hydrogen atom or an alkyl group with a carbon number of 1 to 6,
$R^0$ represents a halogen atom, a cyano group, an alkyl group with a carbon number of 1 to 6, an alkenyl group with a carbon number of 2 to 6, an alkyl halide group with a carbon number of 1 to 6, an N,N-dialkylamino group with a carbon number of 2 to 12, an alkoxy group with a carbon number of 1 to 6, a nitro group, $-C(=O)-R^a$, $-C(=O)-O-R^a$, or $-SO_2R^a$, and $R^a$ represents an alkyl group with a carbon number of 1 to 6, or an aromatic hydrocarbon ring group with a carbon number of 6 to 20 that may have an alkyl group with a carbon number of 1 to 6 or an alkoxy group with a carbon number of 1 to 6 as a substituent,
n1 represents an integer of 0 to 3, n2 represents 0 or 1, n3 represents an integer of 0 to 4, and n4 represents an integer of 0 to 2, and
in the case where a plurality of $R^0$ are present, the plurality of $R^0$ may be same or different.

**6.** The polymerizable compound according to claim 5, wherein $Ar^1$ is a group represented by any of the following Formulas (IV-1) to (IV-3):

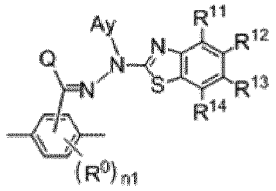

(IV-1)

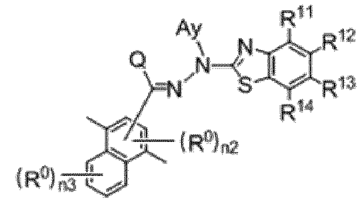

(IV-2)

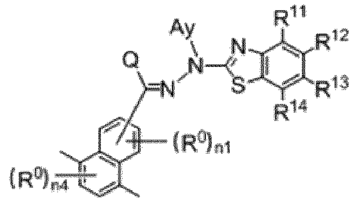

(IV-3)

where Ay, Q, $R^0$, n1, n2, n3, and n4 are as defined above, and
$R^{11}$ to $R^{14}$ each independently represent a hydrogen atom, a halogen atom, an alkyl group with a carbon number of 1 to 6, a cyano group, a nitro group, a fluoroalkyl group with a carbon number of 1 to 6, an alkoxy group with a carbon number of 1 to 6, or $-C(=O)-O-R^b$, $R^b$ represents an alkyl group with a carbon number of 1 to 20 that may have a substituent, an alkenyl group with a carbon number of 2 to 20 that may have a substituent, a cycloalkyl group with a carbon number of 3 to 12 that may have a substituent, or an aromatic hydrocarbon ring group with a carbon number of 5 to 12 that may have a substituent, and at least one of $C-R^{11}$ to $C-R^{14}$ forming a ring may be substituted by a nitrogen atom.

**7.** The polymerizable compound according to any of claims 1 to 4, wherein $Ar^1$ is a group represented by any of the following Formulas (V-1) to (V-4):

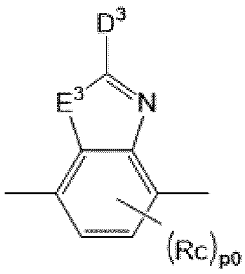

(V-1)

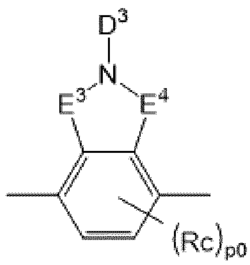

(V-2)

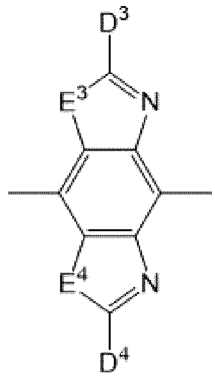

(V-3)

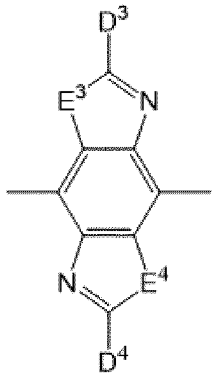

(V-4)

where $E^3$ and $E^4$ each independently represent $-CR^{24}R^{25}-$, -S-, $-NR^{24}-$, -C(=O)-, or -O-, and $R^{24}$ and $R^{25}$ each independently represent a hydrogen atom or an alkyl group with a carbon number of 1 to 4,
Rc represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p0 represents an integer of 0 to 2,
$D^3$ and $D^4$ each independently represent an aromatic hydrocarbon ring group that may have a substituent or an aromatic heterocyclic group that may have a substituent, and
in the case where a plurality of Rc are present, the plurality of Rc may be same or different.

**8.** The polymerizable compound according to claim 7, wherein $D^3$ and $D^4$ are each independently a group represented by any of the following Formulas (v-1) to (v-8):

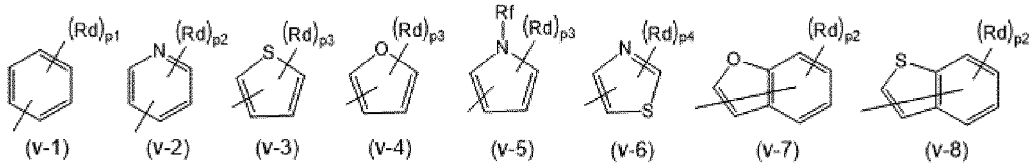

(v-1) (v-2) (v-3) (v-4) (v-5) (v-6) (v-7) (v-8)

where Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p1 represents an integer of 0 to 5, p2 represents an integer of 0 to 4, p3 represents an integer of 0 to 3, and p4 represents an integer of 0 to 2,
Rf represents a hydrogen atom or a methyl group, and
in the case where a plurality of Rd are present, the plurality of Rd may be same or different.

**9.** The polymerizable compound according to claim 7 or 8, wherein $Ar^1$ is a group represented by any of the following Formulas (VI-1) to (VI-5):

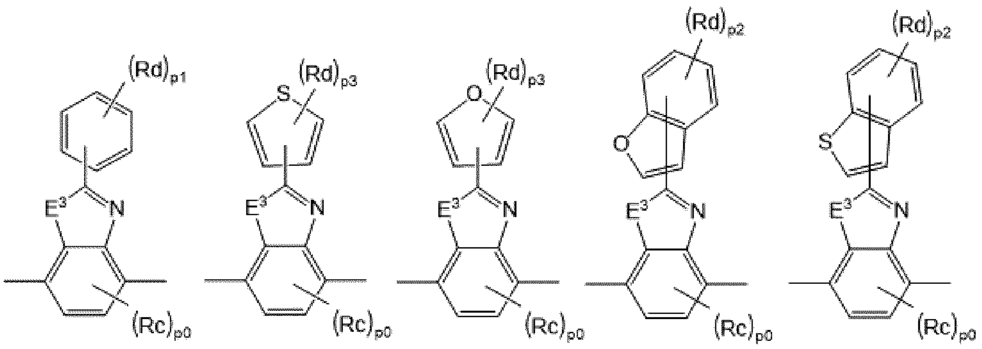

(VI-1) (VI-2) (VI-3) (VI-4) (VI-5)

where $E^3$, Rc, and p0 are as defined above,
Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p1 represents an integer of 0 to 5, p2 represents an integer of 0 to 4, and p3 represents an integer of 0 to 3, and in the case where a plurality of Rc and Rd are present, the plurality of Rc and Rd may be same or different.

**10.** A mixture comprising:

the polymerizable compound according to any of claims 1 to 9; and
a polymerizable compound represented by the following Formula (II):

$$P^{10a}-G^{10}-L^{10a}\left[B^{10}-L^{10}\right]_{a}A^{10}-Z^{10}-Ar^{10}-Z^{20}-A^{20}\left[L^{20}-B^{20}\right]_{b}L^{20a}-G^{20}-P^{20a}$$

(II)

where $Ar^{10}$ represents a divalent aromatic hydrocarbon ring group having at least $D^{10}$ as a substituent or a divalent aromatic heterocyclic group having at least $D^{10}$ as a substituent,
$D^{10}$ represents an organic group with a carbon number of 1 to 67 having at least one aromatic ring selected

from the group consisting of an aromatic hydrocarbon ring and an aromatic heterocyclic ring, $Z^{10}$ and $Z^{20}$ each independently represent a single bond, -O-, -O-$CH_2$-, -$CH_2$-O-, -C(=O)-O-, -O-C(=O)-, -C(=O)-S-, -S-C(=O)-, -$NR^{21}$-C(=O)-, -C(=O)-$NR^{21}$-, -$CF_2$-O-, -O-$CF_2$-, -$CH_2$-$CH_2$-, -$CF_2$-$CF_2$-, -O-$CH_2$-$CH_2$-O-, -CH=CH-C(=O)-O-, -O-C(=O)-CH=CH-, -$CH_2$-$CH_2$-C(=O)-O-, -O-C(=O)-$CH_2$-$CH_2$-, -$CH_2$-$CH_2$-O-C(=O)-, -C(=O)-O-$CH_2$-$CH_2$-, -CH=CH-, -N=CH-, -CH=N-, -N=C($CH_3$)-, -C($CH_3$)=N-, -N=N-, or -C≡C-, and $R^{21}$ each independently represent a hydrogen atom or an alkyl group with a carbon number of 1 to 6,
$A^{10}$ and $A^{20}$ and $B^{10}$ and $B^{20}$ each independently represent a cyclic aliphatic group that may have a substituent or an aromatic group that may have a substituent,
$L^{10}$ and $L^{20}$ and $L^{10a}$ and $L^{20a}$ each independently represent a single bond, -O-, -C(=O)-, -C(=O)-O-, -O-C(=O)-, -$NR^{22}$-C(=O)-, -C(=O)-$NR^{22}$-, -O-C(=O)-O-, -$NR^{22}$-C(=O)-O-, -O-C(=O)-$NR^{22}$-, or -$NR^{22}$-C(=O)-$NR^{23}$-, and $R^{22}$ and $R^{23}$ each independently represent a hydrogen atom or an alkyl group with a carbon number of 1 to 6,
$G^{10}$ and $G^{20}$ each independently represent an aliphatic hydrocarbon group with a carbon number of 3 to 20 that may have a substituent, the aliphatic hydrocarbon group with a carbon number of 3 to 20 may be interrupted by at least one intervening group selected from the group consisting of -O-, -C(=O)-, -C(=O)-O-, -O-C(=O)-, -S-, -C(=O)-S-, -S-C(=O)-, -NR-, -NR-C(=O)-, -C(=O)-NR-, -N=, =N-, and -N=N-, in the case where two or more intervening groups are present, the two or more intervening groups may be same or different and are not adjacent to each other, and R each independently represent a hydrogen atom or an alkyl group with a carbon number of 1 to 6,
$P^{10a}$ and $P^{20a}$ each independently represent a polymerizable group,
a and b each independently represent 0 or 1, and
a part represented by -$L^{10a}$-$G^{10}$-$P^{10a}$ and a part represented by -$L^{20a}$-$G^{20}$-$P^{20a}$ have a same structure.

**11.** The mixture according to claim 10, wherein $Ar^{10}$ is a group represented by any of the following Formulas (III-1) to (III-3):

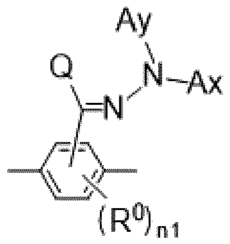

(III-1)

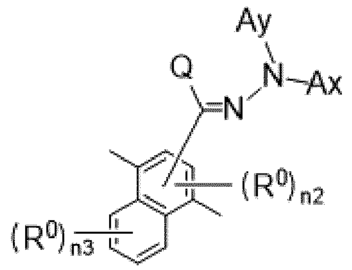

(III-2)

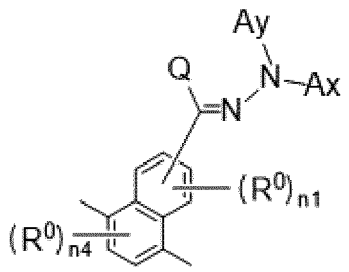

(III-3)

where Ax represents an organic group having at least one aromatic ring selected from the group consisting of an aromatic hydrocarbon ring with a carbon number of 6 to 30 and an aromatic heterocyclic ring with a carbon number of 2 to 30, and the aromatic ring of Ax may have a substituent,
Ay represents a hydrogen atom or an organic group with a carbon number of 1 to 30 that may have a substituent,
Q represents a hydrogen atom or an alkyl group with a carbon number of 1 to 6,
$R^0$ represents a halogen atom, a cyano group, an alkyl group with a carbon number of 1 to 6, an alkenyl group with a carbon number of 2 to 6, an alkyl halide group with a carbon number of 1 to 6, an N,N-dialkylamino group with a carbon number of 2 to 12, an alkoxy group with a carbon number of 1 to 6, a nitro group, -C(=O)-$R^a$, -C(=O)-O-$R^a$, or -$SO_2R^a$, and $R^a$ represents an alkyl group with a carbon number of 1 to 6, or an aromatic hydrocarbon ring group with a carbon number of 6 to 20 that may have an alkyl group with a carbon number of 1 to 6 or an alkoxy group with a carbon number of 1 to 6 as a substituent,
n1 represents an integer of 0 to 3, n2 represents 0 or 1, n3 represents an integer of 0 to 4, and n4 represents an integer of 0 to 2, and
in the case where a plurality of $R^0$ are present, the plurality of $R^0$ may be same or different.

**12.** The mixture according to claim 11, wherein $Ar^{10}$ is a group represented by any of the following Formulas (IV-1) to (IV-3):

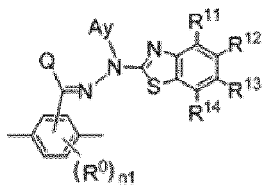

(IV-1)

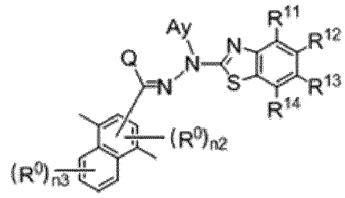

(IV-2)

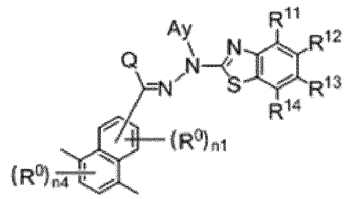

(IV-3)

where Ay, Q, $R^0$, n1, n2, n3, and n4 are as defined above, and
$R^{11}$ to $R^{14}$ each independently represent a hydrogen atom, a halogen atom, an alkyl group with a carbon number of 1 to 6, a cyano group, a nitro group, a fluoroalkyl group with a carbon number of 1 to 6, an alkoxy group with a carbon number of 1 to 6, or -C(=O)-O-$R^b$, $R^b$ represents an alkyl group with a carbon number of 1 to 20 that may have a substituent, an alkenyl group with a carbon number of 2 to 20 that may have a substituent, a cycloalkyl group with a carbon number of 3 to 12 that may have a substituent, or an aromatic hydrocarbon ring group with a carbon number of 5 to 12 that may have a substituent, and at least one of C-$R^{11}$ to C-$R^{14}$ forming a ring may be substituted by a nitrogen atom.

**13.** The mixture according to claim 10, wherein $Ar^{10}$ is a group represented by any of the following Formulas (V-1) to (V-4):

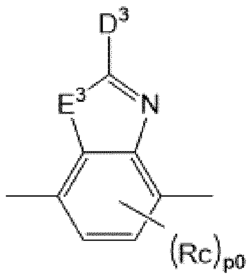

(V-1)

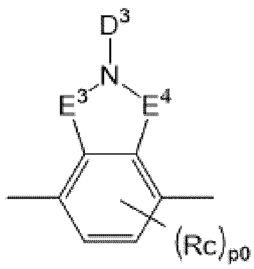

(V-2)

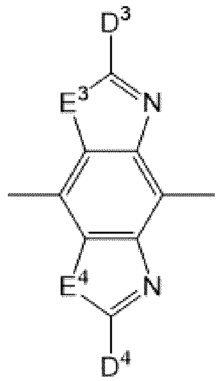

(V-3)

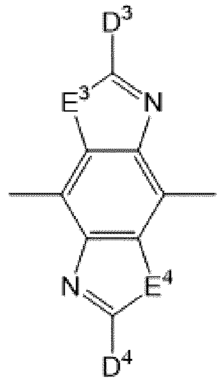

(V-4)

where $E^3$ and $E^4$ each independently represent -$CR^{24}R^{25}$-, -S-, -$NR^{24}$-, -C(=O)-, or -O-, and $R^{24}$ and $R^{25}$ each independently represent a hydrogen atom or an alkyl group with a carbon number of 1 to 4,
Rc represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p0 represents an integer of 0 to 2,
$D^3$ and $D^4$ each independently represent an aromatic hydrocarbon ring group that may have a substituent or an aromatic heterocyclic group that may have a substituent, and
in the case where a plurality of Rc are present, the plurality of Rc may be same or different.

**14.** The mixture according to claim 13, wherein $D^3$ and $D^4$ are each independently a group represented by any of the following Formulas (v-1) to (v-8):

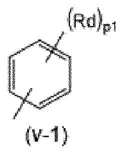
(v-1)
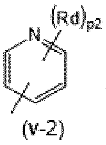
(v-2)
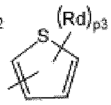
(v-3)
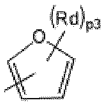
(v-4)
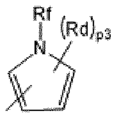
(v-5)
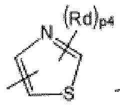
(v-6)
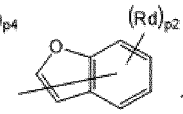
(v-7)
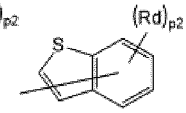
(v-8)

where Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p1 represents an integer of 0 to 5, p2 represents an integer of 0 to 4, p3 represents an integer of 0 to 3, and p4 represents an integer of 0 to 2,
Rf represents a hydrogen atom or a methyl group, and
in the case where a plurality of Rd are present, the plurality of Rd may be same or different.

**15.** The mixture according to claim 13 or 14, wherein $Ar^{10}$ is a group represented by any of the following Formulas (VI-1) to (VI-5):

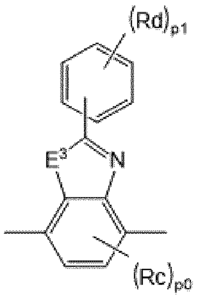
(VI-1)
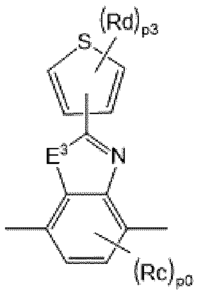
(VI-2)
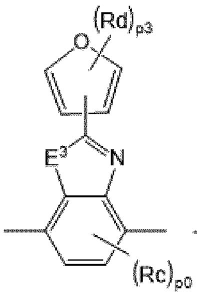
(VI-3)
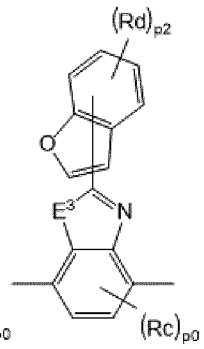
(VI-4)
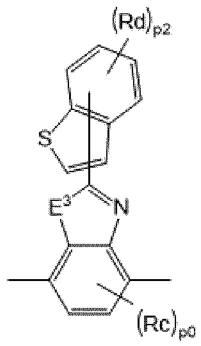
(VI-5)

where $E^3$, Rc, and p0 are as defined above,
Rd represents a halogen atom, an alkyl group with a carbon number of 1 to 6, a cyano group, a nitro group, an alkylsulfinyl group with a carbon number of 1 to 6, an alkylsulfonyl group with a carbon number of 1 to 6, a carboxyl group, a fluoroalkyl group with a carbon number of 1 to 6, an alkoxy group with a carbon number of 1 to 6, a thioalkyl group with a carbon number of 1 to 6, an N-alkylamino group with a carbon number of 1 to 6, an N,N-dialkylamino group with a carbon number of 2 to 12, an N-alkylsulfamoyl group with a carbon number of 1 to 6, or an N,N-dialkylsulfamoyl group with a carbon number of 2 to 12,
p1 represents an integer of 0 to 5, p2 represents an integer of 0 to 4, and p3 represents an integer of 0 to 3, and
in the case where a plurality of Rc and Rd are present, the plurality of Rc and Rd may be same or different.

**16.** A polymer obtainable by polymerization of the polymerizable compound according to any of claims 1 to 9 or the mixture according to any of claims 10 to 15.

**17.** An optical film comprising
the polymer according to claim 16 as a constituent material.

**18.** An optically anisotropic product comprising
a layer having the polymer according to claim 16 as a constituent material.

**19.** A polarizing plate comprising:

the optically anisotropic product according to claim 18; and
a polarizing film.

**20.** A display device comprising the polarizing plate according to claim 19.

**21.** An antireflection film comprising the polarizing plate according to claim 19.

## INTERNATIONAL SEARCH REPORT

International application No.
PCT/JP2017/044977

A. CLASSIFICATION OF SUBJECT MATTER
Int.Cl. C07D277/82(2006.01)i, C07D417/04(2006.01)i, C08F20/38(2006.01)i, G02B1/111(2015.01)i, G02B5/30(2006.01)i

According to International Patent Classification (IPC) or to both national classification and IPC

B. FIELDS SEARCHED
Minimum documentation searched (classification system followed by classification symbols)
Int.Cl. C07D277/82, C07D417/04, C08F20/38, G02B1/111, G02B5/30

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2015-200877 A (FUJIFILM CORP.) 12 November 2015, general expression (1), paragraph [0072] 37-E-37-J, 38-E-38-J, paragraph [0075] 49-I-49-J, claims 1-16, paragraphs [0134]-[0135], [0043], [0048] & US 2015/0277007 A1, formula (1), paragraph [0118] 37-E-37-J, 38-E-38-J, 49-I-49-J, claims 1-16, paragraphs [0119]-[0120], [0086], [0095] & KR 10-2015-0113855 A & CN 104950373 A | 1-6, 10-21<br>4, 7-9 |
| X<br>Y | WO 2016/114347 A1 (DIC CORP.) 21 July 2016, general expression (1), 1-56, claims 1-22, paragraph [0027], 5-a-6, paragraphs [0119], [0120] & TW 201700586 A & KR 10-2017-0105012 A & CN 107209307 A | 1-21<br>4, 7-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 February 2018 (22.02.2018) | 06 March 2018 (06.03.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2017/044977

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2016/104317 A1 (DIC CORP.) 30 June 2016 general expression (1), A11-5, A11-9, A11-13, A12-10, A12-11, A13-9, A14-8, A141-8, A15-5, A2-7, A2-10, C11-7, C11-13, C11-18, C2-10, C2-11, D11-5, claims 1-39, paragraphs [0447]-[0454], A2-6, etc., paragraph [0348] & CN 107108458 A & KR 10-2017-0101194 A | 1-21<br>4, 7-9 |
| X<br>Y | WO 2016/114255 A1 (DIC CORP.) 21 July 2016, general expression (1), A11-5, A11-7, A11-9, claims 1-39, A2-1, etc., paragraphs [0024], [0010] & TW 201708282 A & KR 10-2017-0105000 A & CN 107207676 A | 1-21<br>4, 7-9 |
| Y | XIE, H. et al., "Nonlinear optical crosslinked polymers and interpenetrating polymer networks containing azo-benzothiazole chromophore groups", Polymer, 1998, vol. 39, no. 12, p. 2393-8, DOI: 10.1016/S0032-3861 (97) 00537-5, abstract, BT-2 | 4 |
| Y | WO 2016/114348 A1 (DIC CORP.) 21 July 2016, paragraphs [0119]-[0122] (Family: none) | 4 |
| P, X | JP 6191754 B1 (NIPPON ZEON CO., LTD.) 18 August 2017, formula (I), in particular, compounds 1, 5 (Family: none) | 1-3, 5-21 |
| P, A | US 2017/0184766 A1 (SUMITOMO CHEMICAL CO., LTD.) 29 June 2017, the formula (A-1), example 1 & JP 2017-120430 A & KR 10-2017-0077817 A & CN 106990472 A | 1-2, 7-21 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014010325 A1
- JP 2015200877 A
- JP 2010031223 A
- JP 2007002208 A
- JP 2009173893 A
- JP 2009274984 A
- JP 2010030979 A
- JP 2011006360 A
- JP 2010024438 A
- JP H05310845 A
- US 5179171 A
- JP H0597978 A
- US 5202388 A
- JP H11124429 A
- WO 9920676 A1
- WO 2011068138 A1


### Non-patent literature cited in the description


- March's Advanced Organic Chemistry. Wiley
- **S.R. SANDLER ; W. KARO.** Organic Functional Group Preparations. Hirokawa Shoten